(19) Europäisches Patentamt
European Patent Office
Office européen des brevets

(11) **EP 2 391 636 B1**

(12) **EUROPÄISCHE PATENTSCHRIFT**

(45) Veröffentlichungstag und Bekanntmachung des Hinweises auf die Patenterteilung:
**27.09.2017 Patentblatt 2017/39**

(21) Anmeldenummer: **10705318.3**

(22) Anmeldetag: **29.01.2010**

(51) Int Cl.:
**C07K 7/08** (2006.01)   **A61K 47/50** (2017.01)

(86) Internationale Anmeldenummer:
**PCT/EP2010/051072**

(87) Internationale Veröffentlichungsnummer:
**WO 2010/086401 (05.08.2010 Gazette 2010/31)**

(54) **ANTIBIOTISCHE PEPTIDE**

ANTIBIOTIC PEPTIDES

PEPTIDES ANTIBIOTIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **29.01.2009 DE 102009007381**

(43) Veröffentlichungstag der Anmeldung:
**07.12.2011 Patentblatt 2011/49**

(73) Patentinhaber: **AMP-Therapeutics GmbH 04103 Leipzig (DE)**

(72) Erfinder:
- **HOFFMANN, Ralf**
  **04463 Großpösna (DE)**
- **KNAPPE, Daniel**
  **04105 Leipzig (DE)**
- **HANSEN, Anna Klara Brigitte**
  **30989 Gehrden (DE)**

(74) Vertreter: **CH Kilger Anwaltspartnerschaft mbB Fasanenstrasse 29 10719 Berlin (DE)**

(56) Entgegenhaltungen:
**WO-A1-2009/013262    WO-A2-02/079467**

- **ROSENGREN K J ET AL: "Cyclization of pyrrhocoricin retains structural elements crucial for the antimicrobial activity of the native peptide" BIOPOLYMERS - PEPTIDE SCIENCE SECTION 2004 JOHN WILEY AND SONS INC. US LNKD-DOI:10.1002/BIP.20159, Bd. 76, Nr. 5, 2004, Seiten 446-458, XP002582149**

- **KRAGOL G ET AL: "Identification of crucial residues for the antibacterial activity of the proline-rich peptide, pyrrhocoricin" EUROPEAN JOURNAL OF BIOCHEMISTRY, BLACKWELL PUBLISHING, BERLIN, DE LNKD-DOI:10.1046/J.1432-1033.2002.03119.X, Bd. 269, Nr. 17, 1. September 2002 (2002-09-01), Seiten 4226-4237, XP008106140 ISSN: 0014-2956 [gefunden am 2002-08-07]**
- **BORYSOWSKI JAN ET AL: "Fusion to cell-penetrating peptides will enable lytic enzymes to kill intracellular bacteria." MEDICAL HYPOTHESES JAN 2010 LNKD-PUBMED:19656633, Bd. 74, Nr. 1, Januar 2010 (2010-01), Seiten 164-166, XP002587106 ISSN: 1532-2777**
- **MAI J C ET AL: "A Proapoptotic Peptide for the Treatment of Solid Tumors", CANCER RESEARCH, AMERICAN ASSOCIATION FOR CANCER RESEARCH, US, vol. 61, 1 November 2001 (2001-11-01), pages 7709-7712, XP002991656, ISSN: 0008-5472**
- **DANIEL KNAPPE ET AL: "Oncocin (VDKPPYLPRPRPPRRIYNR-NH2): A Novel Antibacterial Peptide Optimized against Gram-Negative Human Pathogens", JOURNAL OF MEDICINAL CHEMISTRY, AMERICAN CHEMICAL SOCIETY, US, vol. 53, no. 14, 22 July 2010 (2010-07-22) , pages 5240-5247, XP002658974, ISSN: 0022-2623, DOI: 10.1021/JM100378B [retrieved on 2010-06-21]**

- **M Schneider ET AL: "Differential Infectivity of Two Pseudomonas Species and the Immune Response in the Milkweed Bug, Oncopeltus fasciatus (Insecta: Hemiptera)", Journal of Invertebrate Pathology, vol. 78, no. 3, 1 October 2001 (2001-10-01), pages 135-140, XP055109497, ISSN: 0022-2011, DOI: 10.1006/jipa.2001.5054**
- **BENCIVENGO A-M ET AL: "The efficacy of the antibacterial peptide, pyrrhocoricin, is finely regulated by its amino acid residues and active domains", LETTERS IN PEPTIDE SCIENCE, ESCOM SCIENCE PUBLISHERS, NL, vol. 8, 1 January 2002 (2002-01-01), pages 201-209, XP002329693, ISSN: 0929-5666, DOI: 10.1007/BF02446518**

Bemerkungen:
    Die Akte enthält technische Angaben, die nach dem Eingang der Anmeldung eingereicht wurden und die nicht in dieser Patentschrift enthalten sind.

SEQUENCE LISTING

<110> Universität Leipzig

<120> Antibiotische Peptide

<130> R2835 PCT BLN

<150> DE 10 2009 007 381.7
<151> 2009-01-29

<160> 126

<170> PatentIn version 3.5

<210> 1
<211> 18
<212> PRT
<213> Artificial Sequence

<220>
<223> Artificial Peptide

<220>
<221> misc_feature
<222> (18)..(18)
<223> Xaa is Asparagine amide

<400> 1

```
Val Asp Lys Pro Pro Tyr Leu Pro Arg Pro Pro Pro Pro Arg Arg Ile
1               5                   10                  15

Tyr Xaa
```

<210> 2
<211> 20
<212> PRT
<213> Artificial Sequence

<220>
<223> Artificial Peptide

<400> 2

```
Val Asp Lys Pro Pro Tyr Leu Pro Arg Pro Pro Pro Pro Arg Arg Ile
1               5                   10                  15

Tyr Asn Asn Arg
                20
```

<210> 3
<211> 19
<212> PRT
<213> Artificial Sequence

<220>
<223> Artificial Peptide

<220>
<221> misc_feature
<222> (11)..(11)
<223> Xaa is 4Hyp

<400> 3

```
Val Asp Lys Pro Pro Tyr Leu Pro Arg Pro Xaa Pro Pro Arg Arg Ile
1               5                   10                  15

Tyr Asn Arg
```

<210> 4
<211> 20
<212> PRT
<213> Artificial Sequence

<220>
<223> Artificial Peptide

<400> 4

Val Asp Lys Pro Pro Tyr Leu Pro Arg Pro Thr Pro Pro Arg Arg Ile
1               5               10              15

Tyr Asn Asn Arg
                20

<210> 5
<211> 20
<212> PRT
<213> Artificial Sequence

<220>
<223> Artificial Peptide

<400> 5

Val Asp Lys Pro Pro Tyr Leu Pro Arg Pro Lys Pro Pro Arg Arg Ile
1               5               10              15

Tyr Asn Asn Arg
                20

<210> 6
<211> 20
<212> PRT
<213> Artificial Sequence

<220>
<223> Artificial Peptide

<400> 6

Val Asp Lys Pro Pro Tyr Leu Pro Arg Pro Lys Pro Pro Arg Arg Ile
1               5               10              15

Tyr Asn Arg Asn
                20

<210> 7
<211> 19
<212> PRT
<213> Artificial Sequence

<220>
<223> Artificial Peptide

<220>
<221> misc_feature
<222> (19)..(19)
<223> Xaa is Arginine amide

<400> 7

Val Asp Lys Pro Pro Tyr Leu Pro Arg Pro Lys Pro Pro Arg Arg Ile
1               5               10              15

Tyr Asn Xaa

<210> 8
<211> 20
<212> PRT
<213> Artificial Sequence

<220>
<223> Artificial Peptide

<400> 8

Val Asp Lys Pro Pro Tyr Leu Pro Arg Pro His Pro Pro Arg Arg Ile
1               5               10              15

Tyr Asn Asn Arg
              20

<210> 9
<211> 20
<212> PRT
<213> Artificial Sequence

<220>
<223> Artificial Peptide

<400> 9

Val Asp Lys Pro Pro Tyr Leu Pro Arg Pro His Pro Pro Arg Arg Ile
1               5               10              15

Tyr Asn Arg Asn
              20

<210> 10
<211> 19
<212> PRT
<213> Artificial Sequence

<220>
<223> Artificial peptide

<400> 10

Val Asp Lys Pro Pro Tyr Leu Pro Arg Pro Tyr Pro Pro Arg Arg Ile
1               5               10              15

Tyr Asn Arg

<210> 11
<211> 19
<212> PRT
<213> Artificial Sequence

<220>
<223> Artificial Peptide

<400> 11

Val Asp Lys Pro Pro Tyr Leu Pro Arg Pro Asn Pro Pro Arg Arg Ile
1               5               10              15

Tyr Asn Arg

<210> 12
<211> 19
<212> PRT
<213> Artificial Sequence

<220>
<223> Artificial Peptide

<400> 12

Val Asp Lys Pro Pro Tyr Leu Pro Arg Pro Gln Pro Pro Arg Arg Ile
1               5               10              15

Tyr Asn Arg

<210> 13
<211> 19
<212> PRT
<213> Artificial Sequence

<220>
<223> Artificial Peptide

<400> 13

Val Asp Lys Pro Pro Tyr Leu Pro Arg Pro Phe Pro Pro Arg Arg Ile
1               5               10              15

Tyr Asn Arg

<210> 14
<211> 20
<212> PRT
<213> Artificial Sequence

<220>
<223> Artificial Peptide

<400> 14

```
Val Asp Lys Pro Pro Tyr Leu Pro Arg Pro Arg Pro Pro Arg Arg Ile
1               5                   10                  15
```

```
Tyr Asn Asn Arg
            20
```

<210> 15
<211> 20
<212> PRT
<213> Artificial Sequence

<220>
<223> Artificial Peptide

<220>
<221> misc_feature
<222> (20)..(20)
<223> Xaa is Arginine amide

<400> 15

```
Val Asp Lys Pro Pro Tyr Leu Pro Arg Pro Arg Pro Pro Arg Arg Ile
1               5                   10                  15
```

```
Tyr Asn Asn Xaa
            20
```

<210> 16
<211> 20
<212> PRT
<213> Artificial Sequence

<220>
<223> Artificial Peptide

<400> 16

```
Val Asp Lys Pro Pro Tyr Leu Pro Arg Pro Arg Pro Pro Arg Arg Ile
1               5                   10                  15
```

```
Tyr Asn Arg Asn
            20
```

<210> 17
<211> 20
<212> PRT
<213> Artificial Sequence

<220>
<223> Artificial Peptide

<220>
<221> misc_feature
<222> (20)..(20)
<223> Xaa is Asparagine amide

<400> 17

```
Val Asp Lys Pro Pro Tyr Leu Pro Arg Pro Arg Pro Arg Arg Ile
1               5               10              15
```

```
Tyr Asn Arg Xaa
            20
```

<210> 18
<211> 19
<212> PRT
<213> Artificial Sequence

<220>
<223> Artificial Peptide

<220>
<221> misc_feature
<222> (19)..(19)
<223> Xaa is Arginine amide

<400> 18

```
Val Asp Lys Pro Pro Tyr Leu Pro Arg Pro Arg Pro Pro Arg Arg Ile
1               5               10              15
```

```
Tyr Asn Xaa
```

<210> 19
<211> 19
<212> PRT
<213> Artificial Sequence

<220>
<223> Artificial Peptide

<400> 19

```
Val Asp Lys Pro Pro Tyr Leu Pro Arg Pro Arg Pro Pro Arg Arg Ile
1               5               10                  15

Tyr Asn Arg
```

<210> 20
<211> 19
<212> PRT
<213> Artificial Sequence

<220>
<223> Artificial Peptide

<400> 20

```
Val Asp Lys Pro Pro Tyr Leu Pro Arg Pro Arg Pro Pro Arg Pro Ile
1               5               10                  15

Tyr Asn Arg
```

<210> 21
<211> 19
<212> PRT
<213> Artificial Sequence

<220>
<223> Artificial Peptide

<220>
<221> misc_feature
<222> (8)..(8)
<223> Xaa is 4Hyp

<400> 21

```
Val Asp Lys Pro Pro Tyr Leu Xaa Arg Pro Arg Pro Pro Arg Arg Ile
1               5               10                  15

Tyr Asn Arg
```

<210> 22
<211> 19
<212> PRT
<213> Artificial Sequence

<220>
<223> Artificial Peptide

<220>
<221> misc_feature
<222> (8)..(8)
<223> Xaa is 4Hyp

<220>
<221> misc_feature
<222> (19)..(19)
<223> Xaa is Arginine amide

<400> 22

```
Val Asp Lys Pro Pro Tyr Leu Xaa Arg Pro Arg Pro Pro Arg Arg Ile
1               5               10                  15

Tyr Asn Xaa
```

<210> 23
<211> 19
<212> PRT
<213> Artificial Sequence

<220>
<223> Artificial Peptide

<220>
<221> misc_feature
<222> (4)..(4)
<223> Xaa is 4Hyp

<400> 23

```
Val Asp Lys Xaa Pro Tyr Leu Pro Arg Pro Arg Pro Pro Arg Arg Ile
1               5               10                  15

Tyr Asn Arg
```

<210> 24
<211> 19
<212> PRT
<213> Artificial Sequence

<220>
<223> Artificial Peptide

<220>
<221> misc_feature
<222> (13)..(13)
<223> Xaa is 4Hyp

<220>
<221> misc_feature
<222> (19) .. (19)
<223> Xaa is Arginine amide

<400> 24

```
Val Asp Lys Pro Pro Tyr Leu Pro Arg Pro Arg Pro Xaa Arg Arg Ile
1               5               10              15

Tyr Asn Xaa
```

<210> 25
<211> 19
<212> PRT
<213> Artificial Sequence

<220>
<223> Artificial Peptide

<220>
<221> misc_feature
<222> (15)..(15)
<223> Xaa is 4Hyp

<220>
<221> misc_feature
<222> (19) .. (19)
<223> Xaa is Arginine amide

<400> 25

```
Val Asp Lys Pro Pro Tyr Leu Pro Arg Pro Arg Pro Pro Arg Xaa Ile
1               5               10              15

              Tyr Asn Xaa
```

<210> 26
<211> 20
<212> PRT
<213> Artificial Sequence

<220>
<223> Artificial Peptide

<220>
<221> misc_feature
<222> (19)..(19)
<223> Xaa is Orn

<220>
<221> misc_feature
<222> (20)..(20)
<223> Xaa is Asparagine amide

<400> 26

```
Val Asp Lys Pro Pro Tyr Leu Pro Arg Pro Arg Pro Pro Arg Arg Ile
1               5               10              15

Tyr Asn Xaa Xaa
            20
```

<210> 27
<211> 18
<212> PRT
<213> Artificial Sequence

<220>
<223> Artificial Peptide

<220>
<221> misc_feature
<222> (18)..(18)
<223> Xaa is Asparagine amide

<400> 27

```
Val Asp Lys Pro Pro Tyr Leu Pro Arg Pro Arg Pro Pro Arg Arg Ile
1               5               10              15

Tyr Xaa
```

<210> 28
<211> 18
<212> PRT
<213> Artificial Sequence

<220>
<223> Artificial Peptide

<400> 28

```
Val Asp Lys Pro Pro Tyr Leu Pro Arg Pro Arg Pro Pro Arg Arg Ile
1               5               10              15

Tyr Asn
```

<210> 29
<211> 19
<212> PRT
<213> Artificial Sequence

<220>
<223> Artificial Peptide

<220>
<221> misc_feature
<222> (19)..(19)

<223> Xaa is Arginine amide

<400> 29

Ala Asp Lys Pro Pro Tyr Leu Pro Arg Pro Arg Pro Pro Arg Arg Ile
1               5                   10                  15

Tyr Asn Xaa

<210> 30
<211> 19
<212> PRT
<213> Artificial Sequence

<220>
<223> Artificial Peptide

<220>
<221> misc_feature
<222> (19)..(19)
<223> Xaa is Arginine amide

<400> 30

Val Ala Lys Pro Pro Tyr Leu Pro Arg Pro Arg Pro Pro Arg Arg Ile
1               5                   10                  15

                        Tyr Asn Xaa

<210> 31
<211> 19
<212> PRT
<213> Artificial Sequence

<220>
<223> Artificial Peptide

<220>
<221> misc_feature
<222> (19)..(19)
<223> Xaa is Arginine amide

<400> 31

Val Asp Ala Pro Pro Tyr Leu Pro Arg Pro Arg Pro Pro Arg Arg Ile
1               5                   10                  15

Tyr Asn Xaa

<210> 32
<211> 19
<212> PRT

<213> Artificial Sequence

<220>
<223> Artificial Peptide

<220>
<221> misc_feature
<222> (19)..(19)
<223> Xaa is Arginine amide

<400> 32

```
Val Asp Lys Ala Pro Tyr Leu Pro Arg Pro Arg Pro Pro Arg Arg Ile
1                   5                   10                  15

Tyr Asn Xaa
```

<210> 33
<211> 19
<212> PRT
<213> Artificial Sequence

<220>
<223> Artificial Peptide

<220>
<221> misc_feature
<222> (19)..(19)
<223> Xaa is Arginine amide

<400> 33

```
Val Asp Lys Pro Ala Tyr Leu Pro Arg Pro Arg Pro Pro Arg Arg Ile
1                   5                   10                  15

Tyr Asn Xaa
```

<210> 34
<211> 19
<212> PRT
<213> Artificial Sequence

<220>
<223> Artificial Peptide

<220>
<221> misc_feature
<222> (19)..(19)
<223> Xaa is Arginine amide

<400> 34

Val Asp Lys Pro Pro Ala Leu Pro Arg Pro Arg Pro Pro Arg Arg Ile
1               5               10                  15

Tyr Asn Xaa

<210> 35
<211> 19
<212> PRT
<213> Artificial Sequence

<220>
<223> Artificial Peptide

<220>
<221> misc_feature
<222> (19)..(19)
<223> Xaa is Arginine amide

<400> 35

Val Asp Lys Pro Pro Tyr Ala Pro Arg Pro Arg Pro Pro Arg Arg Ile
1               5               10                  15

Tyr Asn Xaa

<210> 36
<211> 19
<212> PRT
<213> Artificial Sequence

<220>
<223> Artificial Peptide

<220>
<221> misc_feature
<222> (19)..(19)
<223> Xaa is Arginine amide

<400> 36

Val Asp Lys Pro Pro Tyr Leu Ala Arg Pro Arg Pro Pro Arg Arg Ile
1               5               10                  15

Tyr Asn Xaa

<210> 37
<211> 19
<212> PRT
<213> Artificial Sequence

<220>
<223> Artificial Peptide

<220>
<221> misc_feature
<222> (19)..(19)
<223> Xaa is Arginine amide

<400> 37

Val Asp Lys Pro Pro Tyr Leu Pro Ala Pro Arg Pro Pro Arg Arg Ile
1                   5                   10                  15

Tyr Asn Xaa

<210> 38
<211> 19
<212> PRT
<213> Artificial Sequence

<220>
<223> Artificial Peptide

<220>
<221> misc_feature
<222> (19)..(19)
<223> Xaa is Arginine amide

<400> 38

Val Asp Lys Pro Pro Tyr Leu Pro Arg Ala Arg Pro Pro Arg Arg Ile
1                   5                   10                  15

Tyr Asn Xaa

<210> 39
<211> 19
<212> PRT
<213> Artificial Sequence

<220>
<223> Artificial Peptide

<220>
<221> misc_feature
<222> (19)..(19)
<223> Xaa is Arginine amide

<400> 39

Val Asp Lys Pro Pro Tyr Leu Pro Arg Pro Ala Pro Pro Arg Arg Ile
1                   5                   10                  15

Tyr Asn Xaa

<210> 40
<211> 19
<212> PRT
<213> Artificial Sequence

<220>
<223> Artificial Peptide

<220>
<221> misc_feature
<222> (19)..(19)
<223> Xaa is Arginine amide

<400> 40

Val Asp Lys Pro Pro Tyr Leu Pro Arg Pro Arg Ala Pro Arg Arg Ile
1               5               10              15

Tyr Asn Xaa

<210> 41
<211> 19
<212> PRT
<213> Artificial Sequence

<220>
<223> Artificial Peptide

<220>
<221> misc_feature
<222> (19)..(19)
<223> Xaa is Arginine amide

<400> 41

Val Asp Lys Pro Pro Tyr Leu Pro Arg Pro Arg Pro Ala Arg Arg Ile
1               5               10              15

Tyr Asn Xaa

<210> 42
<211> 19
<212> PRT
<213> Artificial Sequence

<220>
<223> Artificial Peptide

<220>
<221> misc_feature
<222> (19)..(19)
<223> Xaa is Arginine amide

<400> 42

Val Asp Lys Pro Pro Tyr Leu Pro Arg Pro Arg Pro Pro Ala Arg Ile
1                   5                   10                  15

Tyr Asn Xaa

<210> 43
<211> 19
<212> PRT
<213> Artificial Sequence

<220>
<223> Artificial Peptide

<220>
<221> misc_feature
<222> (19)..(19)
<223> Xaa is Arginine amide

<400> 43

Val Asp Lys Pro Pro Tyr Leu Pro Arg Pro Arg Pro Pro Arg Ala Ile
1                   5                   10                  15

Tyr Asn Xaa

<210> 44
<211> 19
<212> PRT
<213> Artificial Sequence

<220>
<223> Artificial Peptide

<220>
<221> misc_feature
<222> (19)..(19)
<223> Xaa is Arginine amide

<400> 44

Val Asp Lys Pro Pro Tyr Leu Pro Arg Pro Arg Pro Pro Arg Arg Ala
1                   5                   10                  15

Tyr Asn Xaa

<210> 45
<211> 19
<212> PRT
<213> Artificial Sequence

<220>
<223> Artificial Peptide

<220>
<221> misc_feature
<222> (19)..(19)
<223> Xaa is Arginine amide

<400> 45

```
Val Asp Lys Pro Pro Tyr Leu Pro Arg Pro Arg Pro Pro Arg Arg Ile
1               5               10              15

                    Ala Asn Xaa
```

<210> 46
<211> 19
<212> PRT
<213> Artificial Sequence

<220>
<223> Artificial Peptide

<220>
<221> misc_feature
<222> (19)..(19)
<223> Xaa is Arginine amide

<400> 46

```
Val Asp Lys Pro Pro Tyr Leu Pro Arg Pro Arg Pro Pro Arg Arg Ile
1               5               10              15


Tyr Ala Xaa
```

<210> 47
<211> 19
<212> PRT
<213> Artificial Sequence

<220>
<223> Artificial Peptide

<220>
<221> misc_feature
<222> (19)..(19)
<223> Xaa is Alanine amide

<400> 47

```
Val Asp Lys Pro Pro Tyr Leu Pro Arg Pro Arg Pro Pro Arg Arg Ile
1               5               10              15


Tyr Asn Xaa
```

<210> 48
<211> 18
<212> PRT
<213> Artificial Sequence

<220>
<223> Artificial Peptide

<400> 48

```
Val Asp Lys Pro Pro Tyr Leu Pro Arg Pro Arg Pro Pro Arg Pro Ile
1               5                   10                  15

Arg Val
```

<210> 49
<211> 27
<212> PRT
<213> Artificial Sequence

<220>
<223> Artificial Peptide

<220>
<221> misc_feature
<222> (27)..(27)
<223> Xaa is Leucine amide

<400> 49

```
Val Asp Lys Pro Pro Tyr Leu Pro Arg Pro Arg Pro Pro Arg Arg Ile
1               5                   10                  15

Tyr Pro Gln Pro Arg Pro Pro His Pro Arg Xaa
            20          25
```

<210> 50
<211> 19
<212> PRT
<213> Artificial Sequence

<220>
<223> Artificial Peptide

<220>
<221> misc_feature
<222> (19)..(19)
<223> Xaa is Ornithine amide

<400> 50

Val Asp Lys Pro Pro Tyr Leu Pro Arg Pro Arg Pro Pro Arg Arg Ile
1               5                   10                  15

Tyr Asn Xaa

<210> 51
<211> 19
<212> PRT
<213> Artificial Sequence

<220>
<223> Artificial Peptide

<220>
<221> misc_feature
<222> (19)..(19)
<223> Xaa is Asparagine amide

<400> 51

Val Asp Lys Pro Pro Tyr Leu Pro Arg Pro Arg Pro Pro Arg Arg Ile
1               5                   10                  15

Tyr Asn Xaa

<210> 52
<211> 19
<212> PRT
<213> Artificial Sequence

<220>
<223> Artificial Peptide

<220>
<221> misc_feature
<222> (19)..(19)
<223> Xaa is 2,3-Diamino-3-N-acetylpropionic acid amide

<400> 52

Val Asp Lys Pro Pro Tyr Leu Pro Arg Pro Arg Pro Pro Arg Arg Ile
1               5                   10                  15

Tyr Asn Xaa

<210> 53
<211> 19
<212> PRT
<213> Artificial Sequence

<220>
<223> Artificial Peptide

<220>
<221> misc_feature
<222> (19)..(19)
<223> Xaa is Histidine amide

<400> 53

```
Val Asp Lys Pro Pro Tyr Leu Pro Arg Pro Arg Pro Pro Arg Arg Ile
1               5               10              15

Tyr Asn Xaa
```

<210> 54
<211> 19
<212> PRT
<213> Artificial Sequence

<220>
<223> Artificial Peptide

<220>
<221> misc_feature
<222> (19)..(19)
<223> Xaa is 2-Amino-3-guanidinopropionic acid amide

<400> 54

```
Val Asp Lys Pro Pro Tyr Leu Pro Arg Pro Arg Pro Pro Arg Arg Ile
1               5               10              15

Tyr Asn Xaa
```

<210> 55
<211> 19
<212> PRT
<213> Artificial Sequence

<220>
<223> Artificial Peptide

<220>
<221> misc_feature
<222> (19)..(19)
<223> Xaa is Nitroarginine amide

<400> 55

```
Val Asp Lys Pro Pro Tyr Leu Pro Arg Pro Arg Pro Pro Arg Arg Ile
1               5               10              15

Tyr Asn Xaa
```

<210> 56
<211> 19
<212> PRT
<213> Artificial Sequence

<220>
<223> Artificial Peptide

<220>
<221> misc_feature
<222> (19)..(19)
<223> Xaa is N-Methyl-arginine amide

<400> 56

```
Val Asp Lys Pro Pro Tyr Leu Pro Arg Pro Arg Pro Pro Arg Arg Ile
1               5               10              15
```

```
Tyr Asn Xaa
```

<210> 57
<211> 19
<212> PRT
<213> Artificial Sequence

<220>
<223> Artificial Peptide

<220>
<221> misc_feature
<222> (19)..(19)
<223> Xaa is Homoarginine amide

<400> 57

```
Val Asp Lys Pro Pro Tyr Leu Pro Arg Pro Arg Pro Pro Arg Arg Ile
1               5               10              15
```

```
Tyr Asn Xaa
```

<210> 58
<211> 19
<212> PRT
<213> Artificial Sequence

<220>
<223> Artificial Peptide

<220>
<221> misc_feature
<222> (19)..(19)
<223> Xaa is Arginine propyl amide

<400> 58

```
Val Asp Lys Pro Pro Tyr Leu Pro Arg Pro Arg Pro Pro Arg Arg Ile
1               5               10              15

Tyr Asn Xaa
```

<210> 59
<211> 19
<212> PRT
<213> Artificial Sequence

<220>
<223> Artificial Peptide

<220>
<221> misc_feature
<222> (19)..(19)
<223> Xaa is Arginine amide

<400> 59

```
Val Asp Lys Pro Pro Tyr Leu Pro Arg Pro Arg Pro Arg Pro Arg Ile
1               5               10              15

Tyr Asn Xaa
```

<210> 60
<211> 19
<212> PRT
<213> Artificial Sequence

<220>
<223> Artificial Peptide

<220>
<221> misc_feature
<222> (19)..(19)
<223> Xaa is Arginine amide

<400> 60

```
Val Asp Lys Pro Pro Tyr Leu Pro Arg Pro Arg Pro Pro Arg Pro Ile
1               5               10              15

Tyr Asn Xaa
```

<210> 61
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is Arginine amide

<400> 61

Val Asp Lys Pro Pro Tyr Leu Pro Arg Pro Arg Pro Pro Arg Xaa Ile
1               5                   10                  15

Tyr Asn Xaa

<210> 62
<211> 19
<212> PRT
<213> Artificial Sequence

<220>
<223> Artificial Peptide

<220>
<221> misc_feature
<222> (15)..(15)
<223> Xaa is beta-Homoarginine

<220>
<221> misc_feature
<222> (19)..(19)
<223> Xaa is Arginine amide

<400> 62

Val Asp Lys Pro Pro Tyr Leu Pro Arg Pro Arg Pro Pro Arg Xaa Ile
1               5                   10                  15

Tyr Asn Xaa

<210> 63
<211> 19
<212> PRT
<213> Artificial Sequence

<220>
<223> Artificial Peptide

<220>
<221> misc_feature
<222> (15)..(15)
<223> Xaa is 4Hyp

<220>
<221> misc_feature
<222> (19)..(19)
<223> Xaa is Ornithine amide

<400> 63

Val Asp Lys Pro Pro Tyr Leu Pro Arg Pro Arg Pro Pro Arg Xaa Ile
1               5                   10                  15

Tyr Asn Xaa

<210> 64
<211> 19
<212> PRT
<213> Artificial Sequence

<220>
<223> Artificial Peptide

<220>
<221> misc_feature
<222> (19)..(19)
<223> Xaa is Histidine amide

<400> 64

Val Asp Lys Pro Pro Tyr Leu Pro Arg Pro Arg Pro Pro Arg His Ile
1               5                   10                  15

Tyr Asn Xaa

<210> 65
<211> 19
<212> PRT
<213> Artificial Sequence

<220>
<223> Artificial Peptide

<220>
<221> misc_feature
<222> (15)..(15)
<223> Xaa is 2-Amino-3-guanidinopropionic acid

<220>
<221> misc_feature
<222> (19)..(19)
<223> Xaa is Homoarginine amide

<400> 65

Val Asp Lys Pro Pro Tyr Leu Pro Arg Pro Arg Pro Pro Arg Xaa Ile
1               5                   10                  15

Tyr Asn Xaa

<210> 66
<211> 19
<212> PRT
<213> Artificial Sequence

<220>
<223> Artificial Peptide

<220>
<221> misc_feature
<222> (15)..(15)
<223> Xaa is 2-Amino-3-guanidinopropionic acid

<220>
<221> misc_feature
<222> (19)..(19)
<223> Xaa is 2-Amino-3-guanidinopropionic acid amide

<400> 66

Val Asp Lys Pro Pro Tyr Leu Pro Arg Pro Arg Pro Pro Arg Xaa Ile
1               5                   10                  15

Tyr Asn Xaa

<210> 67
<211> 19
<212> PRT
<213> Artificial Sequence

<220>
<223> Artificial Peptide

<220>
<221> misc_feature
<222> (15)..(15)
<223> Xaa is Homoarginine

<220>
<221> misc_feature
<222> (19)..(19)
<223> Xaa is is Homoarginine amide

<400> 67

Val Asp Lys Pro Pro Tyr Leu Pro Arg Pro Arg Pro Pro Arg Xaa Ile
1                   5                   10                  15

Tyr Asn Xaa

<210> 68
<211> 19
<212> PRT
<213> Artificial Sequence

<220>
<223> Artificial Peptide

<220>
<221> misc_feature
<222> (15)..(15)
<223> Xaa is Homoarginine

<220>
<221> misc_feature
<222> (19)..(19)
<223> Xaa is 2-Amino-3-guanidinopropionic acid amide

<400> 68

Val Asp Lys Pro Pro Tyr Leu Pro Arg Pro Arg Pro Pro Arg Xaa Ile
1                   5                   10                  15

Tyr Asn Xaa

<210> 69
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is 2-Amino-3-guanidinopropionic acid amide

<400> 69

Val Asp Lys Pro Pro Tyr Leu Pro Arg Pro Arg Pro Pro Arg Xaa Ile
1                   5                   10                  15

Tyr Asn Xaa

<210> 70
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is Homoarginine amide

<400> 70

Val Asp Lys Pro Pro Tyr Leu Pro Arg Pro Arg Pro Pro Arg Xaa Ile
1                   5                   10                  15

Tyr Asn Xaa

<210> 71
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is Arginine propyl amide

<400> 71

```
Val Asp Lys Pro Pro Tyr Leu Pro Arg Pro Arg Pro Pro Arg Xaa Ile
1               5                   10                  15
```

```
Tyr Asn Xaa
```

<210> 72
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is Ornithine amide

<400> 72

```
Val Asp Lys Pro Pro Tyr Leu Pro Arg Pro Arg Pro Pro Arg Xaa Ile
1               5                   10                  15
```

```
Tyr Asn Xaa
```

<210> 73
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is Ornithine amide

<400> 73

Val Asp Lys Pro Pro Tyr Leu Pro Arg Pro Arg Pro Pro Arg Xaa Leu
1               5               10              15

Tyr Asn Xaa

<210> 74
<211> 19
<212> PRT
<213> Artificial Sequence

<220>
<223> Artificial Peptide

<220>
<221> misc_feature
<222> (15)..(15)
<223> Xaa is Orn

<220>
<221> misc_feature
<222> (19)..(19)
<223> Xaa is is Ornithine amide

<400> 74

Val Asp Lys Pro Pro Tyr Leu Pro Arg Pro Arg Pro Pro Arg Xaa Ile
1               5               10              15

Tyr Gln Xaa

<210> 75
<211> 19
<212> PRT
<213> Artificial Sequence

<220>
<223> Artificial Peptide

<220>
<221> misc_feature
<222> (19)..(19)
<223> Xaa is Arginine amide

<400> 75

Val Glu Lys Pro Pro Tyr Leu Pro Arg Pro Arg Pro Pro Arg Arg Ile
1               5               10              15

                              Tyr Asn Xaa

<210> 76
<211> 19

<212> PRT
<213> Artificial Sequence

<220>
<223> Artificial Peptide

<220>
<221> misc_feature
<222> (19)..(19)
<223> Xaa is Arginine amide

<400> 76

```
Val Asp Arg Pro Pro Tyr Leu Pro Arg Pro Arg Pro Pro Arg Arg Ile
1               5               10              15

Tyr Asn Xaa
```

<210> 77
<211> 19
<212> PRT
<213> Artificial Sequence

<220>
<223> Artificial Peptide

<220>
<221> misc_feature
<222> (19)..(19)
<223> Xaa is Arginine amide

<400> 77

```
Val Asp Lys Pro Pro Tyr Ile Pro Arg Pro Arg Pro Pro Arg Arg Ile
1               5               10              15

Tyr Asn Xaa
```

<210> 78
<211> 19
<212> PRT
<213> Artificial Sequence

<220>
<223> Artificial Peptide

<220>
<221> misc_feature
<222> (19)..(19)
<223> Xaa is Arginine amide

<400> 78

Val Asp Lys Pro Pro Tyr Leu Pro Arg Pro Arg Pro Pro Arg Arg Leu
1               5               10              15

Tyr Asn Xaa

<210> 79
<211> 19
<212> PRT
<213> Artificial Sequence

<220>
<223> Artificial Peptide

<220>
<221> misc_feature
<222> (19)..(19)
<223> Xaa is Arginine amide

<400> 79

Val Asp Lys Pro Pro Tyr Leu Pro Arg Pro Arg Pro Pro Arg Arg Ile
1               5               10              15

Tyr Gln Xaa

<210> 80
<211> 19
<212> PRT
<213> Artificial Sequence

<220>
<223> Artificial Peptide

<220>
<221> misc_feature
<222> (1)..(1)
<223> Xaa is N-Acetyl-Valine

<400> 80

Xaa Asp Lys Pro Pro Tyr Leu Pro Arg Pro Arg Pro Pro Arg Arg Ile
1               5               10              15

Tyr Asn Arg

<210> 81
<211> 19
<212> PRT
<213> Artificial Sequence

<220>
<223> Artificial Peptide

<220>
<221> misc_feature
<222> (1)..(1)
<223> Xaa is N-Formyl-Valine

<400> 81

Xaa Asp Lys Pro Pro Tyr Leu Pro Arg Pro Arg Pro Pro Arg Arg Ile
1               5                   10                  15

Tyr Asn Arg

<210> 82
<211> 19
<212> PRT
<213> Artificial Sequence

<220>
<223> Artificial Peptide

<220>
<221> misc_feature
<222> (1)..(1)
<223> Xaa is Ornithine

<220>
<221> misc_feature
<222> (19)..(19)
<223> Xaa is Arginine amide

<400> 82

Xaa Asp Lys Pro Pro Tyr Leu Pro Arg Pro Arg Pro Pro Arg Arg Ile
1               5                   10                  15

Tyr Asn Xaa

<210> 83
<211> 19
<212> PRT
<213> Artificial Sequence

<220>
<223> Artificial Peptide

<220>
<221> misc_feature
<222> (1)..(1)
<223> Xaa is N-alpha-Acetyl-Ornithine

<220>
<221> misc_feature
<222> (19)..(19)
<223> Xaa is Arginine amide

<400> 83

```
Xaa Asp Lys Pro Pro Tyr Leu Pro Arg Pro Arg Pro Pro Arg Arg Ile
1               5                   10                  15

Tyr Asn Xaa
```

<210> 84
<211> 19
<212> PRT
<213> Artificial Sequence

<220>
<223> Artificial Peptide

<220>
<221> misc_feature
<222> (1)..(1)
<223> Xaa is N-Guanidino-Valine

<220>
<221> misc_feature
<222> (19)..(19)
<223> Xaa is Arginine amide

<400> 84

```
Xaa Asp Lys Pro Pro Tyr Leu Pro Arg Pro Arg Pro Pro Arg Arg Ile
1               5                   10                  15

Tyr Asn Xaa
```

<210> 85
<211> 19
<212> PRT
<213> Artificial Sequence

<220>
<223> Artificial Peptide

<220>
<221> misc_feature
<222> (1)..(1)
<223> Xaa is N-Guanidino-Valine

<220>
<221> misc_feature
<222> (15)..(15)
<223> Xaa is Orn

<220>
<221> misc_feature
<222> (19)..(19)
<223> Xaa is Ornithine amide

<400> 85

Xaa Asp Lys Pro Pro Tyr Leu Pro Arg Pro Arg Pro Pro Arg Xaa Ile
1               5                   10                  15

Tyr Asn Xaa

<210> 86
<211> 18
<212> PRT
<213> Artificial Sequence

<220>
<223> Artificial Peptide

<220>
<221> misc_feature
<222> (18)..(18)
<223> Xaa is Arginine amide

<400> 86

Asp Lys Pro Pro Tyr Leu Pro Arg Pro Arg Pro Pro Arg Arg Ile Tyr
1               5                   10                  15

Asn Xaa

<210> 87
<211> 18
<212> PRT
<213> Apis mellifera

<400> 87

Gly Asn Asn Arg Pro Val Tyr Ile Pro Gln Pro Arg Pro Pro His Pro
1               5                   10                  15

Arg Leu

<210> 88
<211> 18
<212> PRT
<213> Artificial Sequence

<220>
<223> Artificial Peptide

<220>
<221> misc_feature
<222> (1)..(1)
<223> Xaa is 5(6)-Carboxyfluorescein-Glycine

<400> 88

```
Xaa Asn Asn Arg Pro Val Tyr Ile Pro Gln Pro Arg Pro Pro His Pro
1               5                   10                  15

Arg Leu
```

<210> 89
<211> 19
<212> PRT
<213> Drosophila melanogaster

<400> 89

```
Gly Lys Pro Arg Pro Tyr Ser Pro Arg Pro Thr Ser His Pro Arg Pro
1               5                   10                  15

Ile Arg Val
```

<210> 90
<211> 19
<212> PRT
<213> Artificial Sequence

<220>
<223> Artificial Peptide

<220>
<221> misc_feature
<222> (1)..(1)
<223> Xaa is 5(6)-Carboxyfluorescein-Glycine

<400> 90

```
Xaa Lys Pro Arg Pro Tyr Ser Pro Arg Pro Thr Ser His Pro Arg Pro
1               5                   10                  15

Ile Arg Val
```

<210> 91
<211> 20
<212> PRT
<213> Pyrrhocoris apterus

<220>
<221> misc_feature
<222> (20)..(20)
<223> Xaa is Asparagine amide

<400> 91

```
Val Asp Lys Gly Ser Tyr Leu Pro Arg Pro Thr Pro Pro Arg Pro Ile
1               5                   10                  15

Tyr Asn Arg Xaa
            20
```

<210> 92
<211> 20
<212> PRT
<213> Artificial Sequence

<220>
<223> Artificial Peptide

<220>
<221> misc_feature
<222> (1)..(1)
<223> Xaa is 5(6)-Carboxyfluorescein-Valine

<220>
<221> misc_feature
<222> (20)..(20)
<223> Xaa is Asparagine amide

<400> 92

```
Xaa Asp Lys Gly Ser Tyr Leu Pro Arg Pro Thr Pro Pro Arg Pro Ile
1               5                   10                  15

Tyr Asn Arg Xaa
            20
```

<210> 93
<211> 19
<212> PRT
<213> Artificial Sequence

<220>
<223> Artificial Peptide

<220>
<221> misc_feature
<222> (19)..(19)
<223> Xaa is Arginine amide

<400> 93

```
Val Asp Lys Pro Pro Tyr Leu Pro Arg Pro Arg Pro Pro Arg Arg Ile
1               5                   10                  15

Tyr Asn Xaa
```

<210> 94
<211> 19
<212> PRT
<213> Artificial Sequence

<220>
<223> Artificial Peptide

<220>
<221> misc_feature
<222> (1)..(1)
<223> Xaa is 5(6)-Carboxyfluorescein-Valine

<220>
<221> misc_feature
<222> (19)..(19)
<223> Xaa is Arginine amide

<400> 94

```
Xaa Asp Lys Pro Pro Tyr Leu Pro Arg Pro Arg Pro Pro Arg Arg Ile
1               5               10              15
```

```
Tyr Asn Xaa
```

<210> 95
<211> 18
<212> PRT
<213> Artificial Sequence

<220>
<223> Artificial Peptide

<220>
<221> misc_feature
<222> (1)..(1)
<223> Xaa is N-Acetyl-Glycine with Arg-Gln-Ile-Lys-Ile-Trp-Phe-Gln-Asn-Arg-Arg-Met-Lys-Trp-Lys-Lys-C ys coupled to the alpha-C of the acetyl residue via an thioether bond to the S of Cysteine

<400> 95

```
Xaa Asn Asn Arg Pro Val Tyr Ile Pro Gln Pro Arg Pro Pro His Pro
1               5               10              15
```

```
Arg Leu
```

<210> 96
<211> 18
<212> PRT
<213> Artificial Sequence

<220>
<223> Artificial Peptide

```
<220>
<221> misc_feature
<222> (1)..(1)
<223> Xaa is N-Acetyl-Glycine with  N-alpha-5(6)-Carboxyfluorescein-Arg-Gln-Ile-Lys-Ile-Trp-Phe-Gln-A sn-Arg-
Arg-Met-Lys-Trp-Lys-Lys-Cys coupled to the alpha-C of the acetyl residue via an thioether bond to the S of Cysteine

<400> 96
```

**Xaa Asn Asn Arg Pro Val Tyr Ile Pro Gln Pro Arg Pro Pro His Pro**
**1               5               10                  15**


**Arg Leu**

```
<210> 97
<211> 19
<212> PRT
<213> Artificial Sequence

<220>
<223> Artificial Peptide

<220>
<221> misc_feature
<222> (1)..(1)
<223> Xaa is N-Acetyl-Glycine with Arg-Gln-Ile-Lys-Ile-Trp-Phe-Gln-Asn-Arg-Arg-Met-Lys-Trp-Lys-Lys-C ys coupled to the alpha-C of the acetyl residue via an thioether bond to the S of Cysteine

<400> 97
```

**Xaa Lys Pro Arg Pro Tyr Ser Pro Arg Pro Thr Ser His Pro Arg Pro**
**1               5               10                  15**


**Ile Arg Val**

```
<210> 98
<211> 19
<212> PRT
<213> Artificial Sequence

<220>
<223> Artificial Peptide

<220>
<221> misc_feature
<222> (1)..(1)
<223> Xaa is N-Acetyl-Glycine with  N-alpha-5(6)-Carboxyfluorescein-Arg-Gln-Ile-Lys-Ile-Trp-Phe-Gln-A sn-Arg-
Arg-Met-Lys-Trp-Lys-Lys-Cys coupled to the alpha-C of the acetyl residue via an thioether bond to the S of Cysteine

<400> 98
```

Xaa Lys Pro Arg Pro Tyr Ser Pro Arg Pro Thr Ser His Pro Arg Pro
1                   5                   10                  15


Ile Arg Val

<210> 99
<211> 20
<212> PRT
<213> Artificial Sequence

<220>
<223> Artificial Peptide

<220>
<221> misc_feature
<222> (1)..(1)
<223> Xaa is N-Acetyl-Valine with Arg-Gln-Ile-Lys-Ile-Trp-Phe-Gln-Asn-Arg-Arg-Met-Lys-Trp-Lys-Lys-C ys coupled
to the alpha-C of the acetyl residue via an thioether bond to the S of Cysteine

<220>
<221> misc_feature
<222> (20)..(20)
<223> Xaa is Asparagine amide

<400> 99


Xaa Asp Lys Gly Ser Tyr Leu Pro Arg Pro Thr Pro Pro Arg Pro Ile
1                   5                   10                  15


Tyr Asn Arg Xaa
                20

<210> 100
<211> 20
<212> PRT
<213> Artificial Sequence

<220>
<223> Artificial Peptide

<220>
<221> misc_feature
<222> (1)..(1)
<223> Xaa is N-Acetyl-Valine with N-alpha-5(6)-Carboxyfluorescein-Arg-Gln-Ile-Lys-Ile-Trp-Phe-Gln-A sn-Arg-Arg-
Met-Lys-Trp-Lys-Lys-Cys coupled to the alpha-C of the acetyl residue via an thioether bond to the S of Cysteine

<220>
<221> misc_feature
<222> (20)..(20)
<223> Xaa is Asparagine amide

<400> 100

Xaa Asp Lys Gly Ser Tyr Leu Pro Arg Pro Thr Pro Pro Arg Pro Ile
1               5                   10                  15

Tyr Asn Arg Xaa
                20

<210> 101
<211> 19
<212> PRT
<213> Artificial Sequence

<220>
<223> Artificial Peptide

<220>
<221> misc_feature
<222> (1)..(1)

<220>
<221> misc_feature
<222> (19)..(19)
<223> Xaa is Arginine amide

<400> 101

Xaa Asp Lys Pro Pro Tyr Leu Pro Arg Pro Arg Pro Pro Arg Arg Ile
1               5                   10                  15

Tyr Asn Xaa

<210> 102
<211> 19
<212> PRT
<213> Artificial Sequence

<220>
<223> Artificial Peptide

<220>
<221> misc_feature
<222> (1)..(1)
<223> Xaa is N-Acetyl-Valine with N-alpha-5(6)-Carboxyfluorescein-Arg-Gln-Ile-Lys-Ile-Trp-Phe-Gln-A sn-Arg-Arg-
Met-Lys-Trp-Lys-Lys-Cys coupled to the alpha-C of the acetyl residue via an thioether bond to the S of Cysteine

<220>
<221> misc_feature
<222> (19)..(19)
<223> Xaa is Arginine amide

<400> 102

Xaa Asp Lys Pro Pro Tyr Leu Pro Arg Pro Arg Pro Pro Arg Arg Ile
1                   5                   10                  15


Tyr Asn Xaa

<210> 103
<211> 17
<212> PRT
<213> Artificial Sequence

<220>
<223> Artificial Peptide

<220>
<221> misc_feature
<222> (17)..(17)
<223> Xaa is Cysteine with Arg-Gln-Ile-Lys-Ile-Trp-Phe-Gln-Asn-Arg-Arg-Met-Lys-Trp-Lys-Lys-C ys coupled to the S via a disulfide bond to the S of cysteine

<400> 103


Arg Gln Ile Lys Ile Trp Phe Gln Asn Arg Arg Met Lys Trp Lys Lys
1                   5                   10                  15


Xaa

<210> 104
<211> 17
<212> PRT
<213> Artificial Sequence

<220>
<223> Artificial Peptide

<220>
<221> misc_feature
<222> (1)..(1)
<223> Xaa is N-alpha-5(6)-Carboxyfluorescein-Arginine

<220>
<221> misc_feature
<222> (17)..(17)
<223> Xaa is Cysteine with N-alpha-5(6)-Carboxyfluorescein-Arg-Gln-Ile-Lys-Ile-Trp-Phe-Gln-A sn-Arg-Arg-Met-Lys-Trp-Lys-Lys-Cys coupled to the S via a disulfide bond to the S of cysteine

<400> 104


Xaa Gln Ile Lys Ile Trp Phe Gln Asn Arg Arg Met Lys Trp Lys Lys
1                   5                   10                  15


Xaa

<210> 105
<211> 16
<212> PRT
<213> Artificial Sequence

<220>
<223> Artificial Peptide

<400> 105

```
Arg Gln Ile Lys Ile Trp Phe Gln Asn Arg Arg Met Lys Trp Lys Lys
1               5                   10                  15
```

<210> 106
<211> 14
<212> PRT
<213> Artificial Sequence

<220>
<223> Artificial Peptide

<220>
<221> misc_feature
<222> (1)..(1)
<223> Xaa is N-Acetyl-alpha-Amino hexanoic acid with Arg-Gln-Ile-Lys-Ile-Trp-Phe-Gln-Asn-Arg-Arg-Met-Lys-Trp-Lys-Lys-C ys coupled to the alpha-C of the acetyl residue via an thioether bond to the S of Cysteine

<220>
<221> misc_feature
<222> (2)..(2)
<223> Xaa is alpha-Amino hexanoic acid

<400> 106

```
Xaa Xaa Ser Gly Asp Arg Ser Gly Tyr Ser Ser Arg Gly Ser
1               5                   10
```

<210> 107
<211> 19
<212> PRT
<213> Artificial Sequence

<220>
<223> Artificial Peptide

<220>
<221> misc_feature
<222> (13)..(13)
<223> Xaa is 4Hyp

<220>
<221> misc_feature
<222> (15)..(15)
<223> Xaa is Orn

<220>

<221> misc_feature
<222> (19)..(19)
<223> Xaa is Ornithine amide

<400> 107

```
Val Asp Lys Pro Pro Tyr Leu Pro Arg Pro Arg Pro Xaa Arg Xaa Ile
1               5                   10                  15

Tyr Asn Xaa
```

<210> 108
<211> 19
<212> PRT
<213> Artificial Sequence

<220>
<223> Artificial Peptide

<220>
<221> misc_feature
<222> (13)..(13)
<223> Xaa is 4Hyp

<220>
<221> misc_feature
<222> (15)..(15)
<223> Xaa is 4Hyp

<220>
<221> misc_feature
<222> (19)..(19)
<223> Xaa is Ornithine amide

<400> 108

```
Val Asp Lys Pro Pro Tyr Leu Pro Arg Pro Arg Pro Xaa Arg Xaa Ile
1               5                   10                  15

Tyr Asn Xaa
```

<210> 109
<211> 19
<212> PRT
<213> Artificial Sequence

<220>
<223> Artificial Peptide

<220>
<221> misc_feature
<222> (15)..(15)
<223> Xaa is Orn

```
<220>
<221> misc_feature
<222> (16)..(16)
<223> Xaa is tert.-Butylglycin

<220>
<221> misc_feature
<222> (19)..(19)
<223> Xaa is Ornithine amide

<400> 109
```

Val Asp Lys Pro Pro Tyr Leu Pro Arg Pro Arg Pro Pro Arg Xaa Xaa
1                   5                   10                  15

Tyr Asn Xaa

```
<210> 110
<211> 19
<212> PRT
<213> Artificial Sequence

<220>
<223> Artificial Peptide

<220>
<221> misc_feature
<222> (13)..(13)
<223> Xaa is 4Hyp

<220>
<221> misc_feature
<222> (15)..(15)
<223> Xaa is 4Hyp

<220>
<221> misc_feature
<222> (16)..(16)
<223> Xaa is tert.-Butylglycin

<220>
<221> misc_feature
<222> (19)..(19)
<223> Xaa is Ornithine amide

<400> 110
```

Val Asp Lys Pro Pro Tyr Leu Pro Arg Pro Arg Pro Xaa Arg Xaa Xaa
1                   5                   10                  15

Tyr Asn Xaa

```
<210> 111
<211> 19
```

<212> PRT
<213> Artificial Sequence

<220>
<223> Artificial Peptide

<220>
<221> misc_feature
<222> (15)..(15)
<223> Xaa is D-Arginine

<220>
<221> misc_feature
<222> (19)..(19)
<223> Xaa is Arginine amide

<400> 111

```
Val Asp Lys Pro Pro Tyr Leu Pro Arg Pro Arg Pro Pro Arg Xaa Ile
1               5                   10                  15

Tyr Asn Xaa
```

<210> 112
<211> 19
<212> PRT
<213> Artificial Sequence

<220>
<223> Artificial Peptide

<220>
<221> misc_feature
<222> (15)..(15)
<223> Xaa is D-Arginine

<220>
<221> misc_feature
<222> (19)..(19)
<223> Xaa is D-Arginine amide

<400> 112

```
Val Asp Lys Pro Pro Tyr Leu Pro Arg Pro Arg Pro Pro Arg Xaa Ile
1               5                   10                  15

Tyr Asn Xaa
```

<210> 113
<211> 19
<212> PRT
<213> Artificial Sequence

<220>

<223> Artificial Peptide

<220>
<221> misc_feature
<222> (1)..(1)
<223> Xaa is Orn

<220>
<221> misc_feature
<222> (5)..(5)
<223> Xaa is Orn

<220>
<221> misc_feature
<222> (19)..(19)
<223> Xaa is Valine amide

<400> 113

```
Xaa Asn Tyr Ile Xaa Arg Pro Pro Arg Pro Arg Pro Leu Tyr Pro Pro
1               5                   10              15
```

```
Lys Asp Xaa
```

<210> 114
<211> 19
<212> PRT
<213> Artificial Sequence

<220>
<223> Artificial Peptide

<220>
<221> misc_feature
<222> (1)..(1)
<223> Xaa is Orn

<220>
<221> misc_feature
<222> (5)..(5)
<223> Xaa is 4Hyp

<220>
<221> misc_feature
<222> (19)..(19)
<223> Xaa is Valine amide

<400> 114

```
Xaa Asn Tyr Ile Xaa Arg Pro Pro Arg Pro Arg Pro Leu Tyr Pro Pro
1               5                   10              15
```

```
Lys Asp Xaa
```

<210> 115
<211> 19
<212> PRT
<213> Artificial Sequence

<220>
<223> Artificial Peptide

<220>
<221> misc_feature
<222> (1)..(19)
<223> all amino acids are D-amino acids

<220>
<221> misc_feature
<222> (19)..(19)
<223> Xaa is D-Arginine amide

<400> 115

```
Val Asp Lys Pro Pro Tyr Leu Pro Arg Pro Arg Pro Pro Arg Arg Ile
1               5               10              15

Tyr Asn Xaa
```

<210> 116
<211> 19
<212> PRT
<213> Artificial Sequence

<220>
<223> Artificial Peptide

<220>
<221> misc_feature
<222> (1)..(19)
<223> all amino acids are D-amino acids

<220>
<221> misc_feature
<222> (15)..(15)
<223> Xaa is D-Orn

<220>
<221> misc_feature
<222> (19)..(19)
<223> Xaa is D-Ornithine amide

<400> 116

```
Val Asp Lys Pro Pro Tyr Leu Pro Arg Pro Arg Pro Pro Arg Xaa Ile
1               5               10              15

Tyr Asn Xaa
```

<210> 117
<211> 19
<212> PRT
<213> Artificial Sequence

<220>
<223> Artificial Peptide

<220>
<221> misc_feature
<222> (1)..(19)
<223> All amino acids ar D-amino acids

<220>
<221> misc_feature
<222> (19)..(19)
<223> Xaa is D-Valine amide

<400> 117

```
Arg Asn Tyr Ile Arg Arg Pro Pro Arg Pro Arg Pro Leu Tyr Pro Pro
1               5                   10                  15

Lys Asp Xaa
```

<210> 118
<211> 19
<212> PRT
<213> Artificial Sequence

<220>
<223> Artificial Peptide

<220>
<221> misc_feature
<222> (1)..(19)
<223> All amino acids are D-amino acids

<220>
<221> misc_feature
<222> (1)..(1)
<223> Xaa is D-Orn

<220>
<221> misc_feature
<222> (5)..(5)
<223> Xaa is D-Orn

<220>
<221> misc_feature
<222> (19)..(19)
<223> Xaa is D-Valine amide

<400> 118

Xaa Asn Tyr Ile Xaa Arg Pro Pro Arg Pro Arg Pro Leu Tyr Pro Pro
1                   5                   10                  15

                            Lys Asp Xaa

<210> 119
<211> 18
<212> PRT
<213> Apis mellifera

<400> 119

Gly Asn Asn Arg Pro Val Tyr Ile Pro Gln Pro Arg Pro Pro His Pro
1                   5                   10                  15

Arg Ile

<210> 120
<211> 16
<212> PRT
<213> Myrmecia gulosa

<400> 120

Gly Arg Pro Asn Pro Val Asn Asn Lys Pro Thr Pro Tyr Pro His Leu
1                   5                   10                  15

<210> 121
<211> 15
<212> PRT
<213> Palomena prasina

<400> 121

    Val Asp Lys Pro Asp Tyr Arg Pro Arg Pro Arg Pro Pro Asn Met
    1               5                   10                  15

<210> 122
<211> 34
<212> PRT
<213> Oncopeltus fasciatus

<400> 122

Glu Val Ser Leu Lys Gly Glu Gly Gly Ser Asn Lys Gly Phe Ile Gln
1                   5                   10                  15

Gly Ser Gly Thr Lys Thr Leu Phe Gln Asp Asp Lys Thr Lys Leu Asp
                20                  25                  30

Gly Thr

<210> 123
<211> 20
<212> PRT
<213> Oncopeltus fasciatus

<220>
<221> misc_feature
<222> (11)..(11)
<223> Xaa is Pro or any other naturally occurring amino acid

<220>
<221> misc_feature
<222> (19)..(20)
<223> Optional residues

<400> 123

Val Asp Lys Pro Pro Tyr Leu Pro Arg Pro Xaa Pro Pro Arg Arg Ile
1                   5                   10                  15

Tyr Asn Asn Arg
                20

<210> 124
<211> 18
<212> PRT
<213> Artificial Sequence

<220>
<223> Artificial Peptide

<220>
<221> misc_feature
<222> (18)..(18)
<223> Xaa is Alanine amide

<400> 124

Lys Leu Ala Leu Lys Leu Ala Leu Lys Ala Leu Lys Ala Ala Leu Lys
1                   5                   10                  15

Leu Xaa

<210> 125
<211> 9
<212> PRT
<213> Artificial Sequence

<220>
<223> Artificial Peptide

<400> 125

```
Arg Lys Lys Arg Arg Gln Arg Arg Arg
1               5
```

<210> 126
<211> 14
<212> PRT
<213> Artificial Sequence

<220>
<223> Artificial Peptide

<400> 126

```
Val Asp Lys Pro Pro Tyr Leu Pro Arg Pro Arg Pro Pro Arg
1           5                   10
```

**Beschreibung**

**Gebiet der Erfindung**

Diese Erfindung betrifft antibiotische Peptide und Peptiderivate insbesondere für die Verwendung in der Medizin.
Weiterhin bezieht sich die Erfindung auf Zusammensetzungen und Methoden zum Abtöten von Mikroorganismen, wie Bakterien oder Pilze, und Methoden zur Behandlung mikrobieller Infektionen. Die Erfindung beinhaltet weiterhin eine Methode für das Wirkstoff-Screening.

**Hintergrund der Erfindung**

Das Auftreten ernsthafter Bakterien- und Pilzinfektionen ist ein steigendes Problem trotz bemerkenswerter Fortschritte in der Antibiotikatherapie. Jedes Jahr gibt es mehr als 40 Millionen Krankenhausaufenthalte in den Vereinigten Staaten von Amerika und mehr als 2 Millionen dieser Patienten infizieren sich im Krankenhaus. In 50-60% dieser Fälle sind Antibiotika-resistente Bakterien involviert (Tomasz A. Multiple-Antibiotic-Resistant Pathogenic Bacteria - A Report on the Rockefeller-University Workshop. New England Journal of Medicine 330: 1247-51, 1994). Diese im Krankenhaus erworbenen Krankheiten führen zu schätzungsweise 60.000-70.000 Todesfällen in den USA und bis zu 10.000 Todesfällen in Deutschland (Wenzel R P. The Mortality of Hospital-Acquired Blood-Stream-Infections - Need for A New Vital Statistic. International Journal of Epidemiology 17: 225-7, 1988). Während resistente Gram-negative Bakterien in den 70iger Jahren das Hauptproblem darstellten, konnte im letzten Jahrzehnt ein Anstieg der Fälle beobachtet werden, in denen Gram-positive Bakterien, die gegenüber mehreren Antibiotika resistent sind, eine Rolle spielen (Moellering R C. Emerging resistance with gram-positive aerobic infections: Where do we go from here? Introduction: Problems with antimicrobial resistance in gram-positive cocci. Clinical Infectious Diseases 26: 1177-8, 1998). Das derzeitige rasante Auftreten resistenter Stämme betrifft sowohl Gram-positive als auch Gram-negative Pathogene (Hand W L. Current challenges in antibiotic resistance. Adolescent Medicine 11: 427-38, 2000). Hierbei entwickelten sich Resistenzen zuerst in Arten, in denen Einfachmutationen genügten um klinisch wichtige Level zu erreichen z.B. *Staphylococcus aureus* und *Pseudomonas aeruginosa*; es folgten Bakterien, in denen multiple Mutationen notwendig waren, wie z.B. *E. coli* und *Neisseria gonorrhoeae*. Dies wird vornehmlich durch den häufigen Einsatz von Fluoroquinologen-Antibiotika verursacht (Hooper D C. Emerging mechanisms of fluoroquinolone resistance. Emerging Infectious Diseases 7: 337-41, 2001). Ein weiterer wichtiger Grund der Resistenzentwicklung in Gram-negativen Bakterien ist das große Spektrum an Laktamasen in *Escherichia coli* und *Klebsiella pneumonie* (Jones R N. Resistance patterns among nosocomial pathogens - Trends over the past few years. Chest 119: 397S-404S, 2001). Nahezu die Hälfte der klinisch relevanten Stämme von *Haemophilus ducreyi,* der Verursacher von Ulcus molle, trägt Gene, die dieses Bakterium gegen Amoxicillin, Ampicillin und eine Reihe anderer β-Laktame resistent machen (Prachayasittikul V, Lawung R, & Bulow L. Episome profiles and mobilizable beta-lactamase plasmid in Haemophilus ducreyi. Southeast Asian J Trop Med Public Health 31: 80-4, 2000). Gleichermaßen stieg die Resistenz von *Salmonella enterica* serovar *typhimurium* gegenüber Tetrazyklinen von Null Prozent im Jahr 1948 auf 98% im Jahr 1998 (Teuber M. Spread of antibiotic resistance with food-borne pathogens. Cellular and Molecular Life Sciences 56: 755-63, 1999).
Dies verdeutlicht die Notwendigkeit der weiteren Suche nach neuen Antibiotika. Induzierbare antibakterielle Peptide repräsentieren ein Forschungsfeld, in dem die heutige Biochemie, Immunologie und Wirkstoffforschung zusammentreffen. Peptidantibiotika, mit einer Größe von 13 bis zu mehr als hundert Aminosäuren, wurden aus Pflanzen, Tieren und Mikroben isoliert (Boman H G. Peptide Antibiotics and Their Role in Innate Immunity. Annual Review of Immunology 13: 61-92, 1995). Ein einzelnes Tier besitzt ca. 6-10 Peptidantibiotika, wobei jedes Peptid oft ein komplett anderes Aktivitätsspektrum zeigt (Barra D, Simmaco M, & Boman H G. Gene-encoded peptide antibiotics and innate immunity. Do 'animalcules' have defence budgets? Febs Letters 430: 130-4, 1998). Es ist bekannt, dass die überwältigende Anzahl an antibakteriellen Peptiden einschließlich den gut untersuchten Defensinen, Cecropinen und Magaininen, durch einen "lytischen/ionischen" Mechanismus wirken. Als gemeinsamen Wirkmechanismus dieser "lytischen" Peptide wird ein permeabilisierender Effekt auf die bakterielle Zytoplasmamembran diskutiert (Ludtke S, He K, & Huang H. Membrane thinning caused by magainin 2. Biochemistry 34: 16764-9, 1995; Wimley W C, Selsted M E, & White S H. Interactions Between Human Defensins and Lipid Bilayers - Evidence for Formation of Multimeric Pores. Protein Science 3: 1362-73, 1994; Shai Y. Molecular Recognition Between Membrane-Spanning Polypeptides. Trends in Biochemical Sciences 20: 460-4, 1995). Eine kationische, amphipathische Struktur, die hydrophile Ionen-(Protonen-) Kanäle in einer Lipiddoppelschicht ausbildet, ist Grundlage dieser Aktivität. Durch das Austreten von Protonen wird das für viele grundlegende Lebensprozesse notwendige Membranpotential zerstört und so die Zelle abgetötet. Da die Störung der Membran durch diese Peptide von der Erkennung chiraler Moleküle abhängig ist, wird ein Aminosäureaustausch, der die generelle amphipathische Struktur oder basische Nettoladung nicht aufhebt, funktionell toleriert (Wade D et al. All-D Amino Acid-Containing Channel-Forming Antibiotic Peptides. Proceedings of the National Academy of Sciences of the United States of America 87: 4761-5, 1990; Steiner H, Andreu D, & Merrifield R B. Binding and Action of Cecropin and Cecropin Analogs - Antibacterial Peptides from Insects. Biochimica et Biophysica Acta 939: 260-6, 1988). Diese lytischen Peptide wirken oft in höheren Konzentrationen toxisch auf Säugetiermembranen, was ihre Eignung als mögliche Arzneimittel einschränkt. Wird Prolin in die Sequenz der $\alpha$-helikalen antimikrobiellen Peptide eingefügt so sinkt, in Abhängigkeit von der Anzahl der Prolin-Reste, die Fähigkeit der Peptide die Cytoplasmamembran von *E. coli* zu permeabilisieren. Bei dieser Betrachtung ist es verblüffend, dass einige der aktivsten, nativen antibakteriellen Peptide, zumindest in Bezug auf einige Gram-negative Pathogene, zu der Familie der Prolin-reichen Peptide gehören (Otvos L et al. Insect peptides with improved protease-resistance protect mice against bacterial infection. Protein Science 9: 742-9, 2000).

Die oben beschriebenen Nebeneffekte werden durch antimikrobielle Peptide (AMP) überwunden, die ein bakterielles Protein oder andere intra- oder extrazelluläre Komponenten spezifisch erkennen, ohne eine Kreuzreaktivität mit Säugetieranaloga zu zeigen. Dies scheint auf Prolin-reiche antimikrobielle Peptide, einschließlich Apidaecine, Drosocin und Pyrrhocoricin die ursprünglich aus Insekten isoliert wurden, zuzutreffen. Mit der enormen Variation in der Größe und in den biochemischen Eigenschaften, ist es nicht überraschend, dass die Struktur-Wirkungs- und Konformations-Wirkungs-Beziehungen der Fokus der antibakteriellen Peptidforschung ist. Eine komplette Untersuchung des natürlichen, antibakteriellen Peptidrepertoires auf die biologische Stärke ist nicht nur für generelle biochemische Fragestellungen wichtig, sondern auch für die pharmazeutische Industrie von anhaltendem Interesse. Trotz der Probleme von *in vitro* Tests mit Peptid-basierenden Antibiotika, haben einige natürliche, kationische antibakterielle Peptide schon die klinische Testphase erreicht (Boman H G. Peptide Antibiotics and Their Role in Innate Immunity. Annual Review of Immunology 13: 61-92, 1995). Während einige dieser Peptide als topische (örtlich) Mittel in der frühen klinischen Testphase Wirkung zeigten, waren andere in der systemischen Therapie aktiv. Zum Beispiel hat das kationische Protein rBPI 21, das zur parentalen Behandlung von Meningococcaemia eingesetzt wird, die dritte Phase der klinischen Prüfung abgeschlossen (Boman H G. Peptide Antibiotics and Their Role in Innate Immunity. Annual Review of Immunology 13: 61-92, 1995).

Die Familie der Prolin-reichen Peptide (z.B. Apidaecin, Drosocin und Pyrrhocoricin) töten Bakterien nicht nur durch Permeabilisierung ihrer Membran, sondern binden stereospezifisch an ein oder mehrere Zielproteine. Diese möglichen Interaktionspartner, bisher wurde das Hitzeschock Protein DnaK gut untersucht (Kragol G et al. Identification of crucial residues for the antibacterial activity of the proline-rich peptide, pyrrhocoricin. European Journal of Biochemistry 269: 4226-37, 2002; Kragol G et al. The antibacterial peptide pyrrhocoricin inhibits the ATPase actions of DnaK and prevents chaperone-assisted protein folding. Biochemistry 40: 3016-26, 2001), werden durch die Prolin-reichen Peptide inhibiert und vermutlich die korrekte Proteinfaltung verhindert, was letztendlich zum Zelltod führt. Zudem scheinen Prolin-reiche Peptide, im starken Gegensatz zu AMPs mit definierter Sekundärstruktur wie Melittin oder Gramicidin, in vitro weder hämolytisch noch toxisch auf eukaryotische Zellen zu wirken. Entscheidenden Einfluss auf die Entwicklung neuer peptidbasierter Antibiotika hat neben der antimikrobiellen Aktivität vor allem die Stabilität in Säugetierserum (25%). So wird beispielsweise Drosocin innerhalb einer Stunde abgebaut, während Pyrrhocoricin mit Halbwertszeiten von 120 Minuten erheblich stabiler gegenüber Proteasen ist.

Die Sequenz von Pyrrhocoricin ist in den folgenden Dokumenten offenbart: Rosengren K J et al. (Cyclization of pyrrhocoricin retains structural elements crucial for the antimicrobial activity of the native peptide. Biopolymers 76(5): 446-58, 2004) und Kragol G et al. (Identification of crucial residues for the antibacterial activity of the proline-rich peptide, pyrrhocoricin. European Journal of Biochemistry 269: 4226-37, 2002).

In biologischen Experimenten von Schneider und Dorn (2001) (Schneider M & Dorn A. Differential infectivity of two Pseudomonas species and the immune response in the milkweed bug, oncopeltus fasciatus (Insecta : Hemiptera). Journal of Invertebrate Pathology 78: 135-40, 2001) wurden Nymphen und Puppen der Milchkrautwanze Oncopeltus fasciatus aus der Familie der Lygaeidae mit zwei verschiedenen gram-negativen Pseudomonas Spezies infiziert und deren Immunantwort analysiert. Während eine Infektion der Nymphen von O. fasciatus mit dem humanen Pathogen Pseudomonas aeruginosa, nach 48 h den Tod aller Individuen zur Folge hatte, überlebten 71 % der mit weniger pathogenen Pseudomonas putida infizierten Individuen mindestens 96 h. Wurden die Nymphen der Milchkrautwanze nun zuerst mit P. putida und nach 24 h mit P. aeruginosa infiziert, stieg hier die überlebensrate der doppelt infizierten Individuen innerhalb der ersten 24 h signifikant auf 73%. Die wahrscheinliche Induzierung der Synthese von antibakteriellen Peptiden, durch die sich Insekten im Rahmen ihres angeborenen Immunsystems gegen eindringende Mikroorganismen wehren, wurde anschließend untersucht. Vier Peptide (Oncopeltus antibakterielles Peptid 1-4) wurden mit Molekulargewichten von 15, 8, 5 bzw. 2 kDa identifiziert und für die antibakterielle Wirkung verantwortlich gemacht. Die Sequenzanalyse nach Edman ergab neben einer 34 Aminosäure langen Teilsequenz für Peptid 1 (15 kDa) auch die unvollständige Sequenz des Prolin-reichen 2 kDa Peptids 4. Es war nicht möglich die Aminosäuren an Positionen 11 und die C-terminale Sequenz ab Position 19 eindeutig zu identifizieren. Das genaue Molekulargewicht ist unbekannt.

Eine Auswahl bisher bekannter Sequenzen von antibiotischen Peptiden ist in Tabelle 1 aufgelistet:

**Tabelle 1:**

| Peptid | Species | Sequenz | SEQ ID NO. | Lit.-Stelle |
|---|---|---|---|---|
| Apidaecin 1a | *Apis mellifera* | GNNRPVYIPQPRPPHPRI | 119 | [1] |
| Apidaecin 1b | *Apis mellifera* | GNNRPVYIPQPRPPHPRL | 87 | [1] |
| Drosocin | *Drosphila melanogaster* | GKPRPYSPRPTSHPRPIRV | 89 | [2] |
| Formaecin 1 | *Myrmecia gulosa* | GRPNPVNNKPTPYPHL | 120 | [3] |
| Pyrrhocoricin | *Pyrrhocoris apterus* | VDKGSYLPRPTPPRPIYNRN-NH$_2$ | 91 | [4] |
| Metalnikowin 1 | *Palomena prasina* | VDKPDYRPRPRPPNM | 121 | [5] |
| Oncopeltus antibakterielles Peptid 1 | *Oncopel tus fasciatus* | EVSLKGEGGSiVKGFTQGSGTKTLFQD DKTKLDGT | 122 | [6] |
| Oncopeltus antibakterielles Peptid 4 | *Oncopel tus fasciatus* | VDKPPYLPRP(X/P)PPRRIYN(NR) | 123 | [6] |

[1] Casteels P, Ampe C, Jacobs F, Vaeck M, & Tempst P. Apidaecins - Antibacterial Peptides from Honeybees. Embo Journal 8: 2387-91, 1989 [2] Bulet P et al. A Novel Inducible Antibacterial Peptide of Drosophila Carries An O-Glycosylated Substitution. Journal of Biological Chemistry 268: 14893-7, 1993 [3] Mackintosh J A et al. Isolation from an ant Myrmecia gulosa of two inducible O-glycosylated proline-rich antibacterial peptides. Journal of Biological Chemistry 273: 6139-41,1998 [4] Cociancich S et al. Novel Inducible Antibacterial Peptides from A Hemipteran Insect, the Sap-Sucking Bug Pyrrhocoris-Apterus. Biochernical Jourrial 300: 567-75, 1994 [5] Chernysh S, Cociancich S, Briand J P, Hetru C, & Bulet P. The inducible antibacterial peptides of the hemipteran insect Palomena prasina: Identification of a unique family of proline-rich peptides and of a novel insect defensin. Journal of Insect Physiology 42: 81-9, 1996 [6] Schneider M & Dorn A. Differential infectivity of two Pseudomonas species and the immune response in the milkweed bug, oncopeltus fasciatus (Insecta : Hemiptera). Journal of Invertebrate Pathology 78: 135-40, 2001

Nach wie vor besteht ein Bedarf an neuen antibakteriellen und antimykotischen Verbindungen, neuen antibakteriellen und antimykotischen pharmazeutischen Zusammensetzungen, sowie Methoden, welche diese verwenden, sowie Verbindungen, welche für das Wirkstoff-Screnning genutzt werden können, um neue pharmazeutische Antibiotika zu detektieren.

Die Aufgabe der vorliegenden Erfindung ist es neue antibiotische Peptide mit gesteigerter Stabilität zur Verfügung zu stellen-, das Wirkungsspektrum der AMP-'s auf Gram-positive Bakterien auszuweiten und so moderne Breitband-Antibiotika zur Verfügung zu stellen, sowie die Peptide in eukaryotische Zellen einzuschleusen und so verborgene Bakterien zu bekämpfen.

**Beschreibung der Erfindung:**

Die Aufgabe wird gelöst durch die erfindungsgemäßen Peptide mit Sequenzen SEQ ID NOs. 61-74, 111 und 112.

Eine Auswahl von Peptide mit der folgenden Formel wurden getestet:

$$\text{Sub}_1\text{-}X_1\text{-}D_2\text{-}K_3\text{-}P_4\text{-}P_5\text{-}Y_6\text{-}L_7\text{-}P_8\text{-}R_9\text{-}P_{10}\text{-}X_2\text{-}P_{12}\text{-}P_{13}\text{-}R_{14}\text{-}X_3\text{-}I_{16}\text{-}P_{17}/Y_{17}\text{-}N_{18}\text{-}N_{19}\text{-}X_4\text{-}\text{Sub}_2 \qquad (\text{Formel 1})$$

$X_1$ ist ein Rest mit einer unpolaren, hydrophoben Seitenkette oder mit einer positiven Nettoladung oder einer unter physiologischen Bedingungen positiv geladenen Seitenkette;

$D_2$ ist ein Asparaginsäure- oder Glutaminsäurerest,

$K_3$ ist ein Rest mit einer positiven Nettoladung oder einer unter physiologischen Bedingungen positiv geladenen Seitenkette, bevorzugt Lysin oder Arginin,

$X_2$ und $X_4$ sind unabhängig von einander ausgewählt aus Resten mit einer positiven Nettoladung oder einer unter physiologischen Bedingungen positiv geladenen Seitenkette;

$X_3$ ist ein Rest mit einer positiven Nettoladung oder einer unter physiologischen Bedingungen positiv geladenen Seitenkette oder Prolin oder ein Prolinderivat;

$L_7$ und $I_{16}$ sind unabhängig voneinander ausgewählt aus Resten mit einer unpolaren, hydrophoben Seitenkette, bevorzugt Leucin, Isoleucin und Valin,

$Y_6$ und $Y_{17}$ sind jeweils Tyrosin, $R_9$ und $R_{14}$ sind jeweils Arginin, $N_{18}$ ist Asparagin oder Glutamin, $N_{19}$ ist Asparagin oder Glutamin oder ist abwesend, $P_4$, $P_5$, $P_8$, $P_{10}$, $P_{12}$, $P_{13}$ und $P_{17}$ sind unabhängig von einander ausgewählt aus Prolin und Prolinderivaten oder Hydroxyprolin und Hydroxyprolinderivaten,

wobei gegebenenfalls $P_{13}$ und $R_{14}$ vertauscht sind, und/oder

gegebenenfalls ein oder zwei der Reste ausgewählt aus $D_2$, $P_4$, $P_5$, $P_8$, $P_{10}$, $P_{12}$ $P_{13}$, $P_{17}$ und $Y_{17}$ durch einen beliebigen Rest ersetzt sind,

$Sub_1$ ist der freie N-Terminus der Aminosäure $X_1$ oder eine modifzierte N-terminale Aminogruppe;

$Sub_2$ ist die freie C-terminale Carboxylgruppe der C-terminalen Aminosäure (-COOH) oder eine modifizierte C-terminale Carboxylgruppe.

In einer besonders bevorzugten Ausführungsform ist $I_{16}$ ausgewählt aus der Gruppe bestehend aus Leucin, Isoleucin, *tert*.-Butylglycin und Valin.

In einer Ausführungsform ist $N_{19}$ abwesend wenn $P_{17}$ vorliegt.

Bevorzugt sind Peptide und Peptidderivate mit einer der generellen Formeln 1 bis 3:

$$Sub_1\text{-}X_1\text{-}D_2\text{-}K_3\text{-}P_4\text{-}P_5\text{-}Y_6\text{-}L_7\text{-}P_8\text{-}R_9\text{-}P_{10}\text{-}X_2\text{-}P_{12}\text{-}P_{13}\text{-}R_{14}\text{-}X_3\text{-}I_{16}\text{-}Y_{17}\text{-}N_{18}\text{-}X_4\text{-}Sub_2 \qquad \text{(Formel 2)}$$

$$Sub_1\text{-}X_1\text{-}D_2\text{-}K_3\text{-}P_4\text{-}P_5\text{-}Y_6\text{-}L_7\text{-}P_8\text{-}R_9\text{-}P_{10}\text{-}X_2\text{-}P_{12}\text{-}P_{13}\text{-}R_{14}\text{-}X_3\text{-}I_{16}\text{-}Y_{17}\text{-}N_{18}\text{-}X_4\text{-}Sub2 \qquad \text{(Formel 3)}$$

$$Sub_1\text{-}X_1\text{-}D_2\text{-}K_3\text{-}P_4\text{-}P_5\text{-}Y_6\text{-}L_7\text{-}P_8\text{-}R_9\text{-}P_{10}\text{-}X_2\text{-}P_{12}\text{-}P_{13}\text{-}R_{14}\text{-}X_3\text{-}I_{16}\text{-}P_{17}\text{-}N_{18}\text{-}X_4\text{-}Sub_2 \qquad \text{(Formel 4)}$$

$X_1$ ist ein Rest mit einer unpolaren, hydrophoben Seitenkette oder mit einer positiven Nettoladung oder einer unter physiologischen Bedingungen positiv geladenen Seitenkette;

$D_2$ ist ein Asparaginsäure- oder Glutaminsäurerest,

$K_3$ ist ein Rest mit einer positiven Nettoladung oder einer unter physiologischen Bedingungen positiv geladenen Seitenkette, bevorzugt Lysin oder Arginin,

$X_2$ und $X_4$ sind unabhängig von einander ausgewählt aus Resten mit einer positiven Nettoladung oder einer unter physiologischen Bedingungen positiv geladenen Seitenkette;

$X_3$ ist ein Rest mit einer positiven Nettoladung oder einer unter physiologischen Bedingungen positiv geladenen Seitenkette oder Prolin oder ein Prolinderivat;

$L_7$ und $I_{16}$ sind unabhängig voneinander ausgewählt aus Resten mit einer unpolaren, hydrophoben Seitenkette, bevorzugt Leucin, Isoleucin und Valin.

In einer besonders bevorzugten Ausführungsform ist $I_{16}$ ausgewählt aus der Gruppe bestehend aus Leucin, Isoleucin, *tert.*-Butylglycin und Valin.

$Y_6$ und $Y_{17}$ sind jeweils Tyrosin, $R_9$ und $R_{14}$ sind jeweils Arginin, $N_{18}$ und $N_{19}$ sind jeweils Asparagin oder Glutamin, $P_4$, $P_5$, $P_8$, $P_{10}$, $P_{12}$, $P_{13}$ und $P_{17}$ sind unabhängig von einander ausgewählt aus Prolin und Prolinderivaten oder Hydroxyprolin und Hydroxyprolinderivaten.

Gegebenenfalls sind einer oder zwei der Reste ausgewählt aus $D_2$, $P_4$, $P_5$, $P_8$, $P_{10}$, $P_{12}$ $P_{13}$, $P_{17}$ und $Y_{17}$ durch einen beliebigen Aminosäurerest bevorzugt einen neutralen Rest, besonders bevorzugt einen neutralen polaren Rest ersetzt.

Weiter sind gegebenenfalls $P_{13}$ und $R_{14}$ vertauscht.

Reste mit einer positiven Nettoladung oder einer unter physiologischen Bedingungen positiv geladenen Seitenkette sind bevorzugt ausgewählt aus der Gruppe bestehend aus Arginin, Lysin, $\delta$-Hydroxylysin, Homoarginin, 2,4-Diaminobuttersäure, $\beta$-Homoarginin, D-Arginin, Arginal (-COOH in Arginin ist ersetzt durch -CHO), 2-Amino-3-guanidinopropionsäure, Nitroarginin (bevorzugt N(G)-Nitroargenin), Nitrosoarginine (bevorzugt N(G)-Nitrosoarginin), Methylarginin (bevorzugt N-Methyl-Arginin), $\varepsilon$-N-Methyllysin, allo-Hydroxylysin, 2,3-Diaminopropionsäure, 2,2'-Diaininopimelinsäure, Ornithin, sym-Dimethylarginin, asym-Dimethylarginin, 2,6-Diaminohexinsäure, *p*-Aminobenzoesäure und 3-Aminotyrosin und weniger bevorzugt Histidin, 1-Methylhistidin und 3-Methylhistidin. $X_1$, $X_2$ und $X_3$ sind bevorzugt unabhängig voneinander aus dieser Liste ausgewählt.

Der Begriff Prolinderivat steht für einen von Prolin abgeleiteten Aminosäurenrest, der aus Prolin bevorzugt durch strukturelle Veränderung einer funktionellen Gruppe erhalten wird. Bevorzugte Prolinderivate sind ausgewählt aus der Gruppe bestehend aus $\beta$-Cyclohexylalanin, 3,4-cis-Methanoprolin, 3,4-Dehydroprolin, Homoprolin oder Pseudoprolin. Der Begriff Hydroxyprolin schließt unter anderem *cis*-4-Hydroxyprolin, *trans*-4-Hydroxyprolin, *cis*-3-Hydroxyprolin und *trans*-3-Hydroxyprolin mit ein. Der Begriff Hydroxyprolinderivat steht entsprechend für einen von Hydroxyprolin abgeleiteten Aminosäurenrest, der aus Hydroxyprolin bevorzugt durch strukturelle Veränderung einer funktionellen Gruppe erhalten wird. Bevorzugte Hydroxyprolinderivate sind ausgewählt aus Hydroxy-$\beta$-Cyclohexylalanin und den oben genannten Prolinderivaten, die mit einer Hydroxylgruppe substituiert sind.

Ein neutraler Rest ist ein Rest mit einer unter physiologischen Bedingungen ungeladenen Seitenkette.

Ein polarer Rest weist bevorzugt mindestens eine polare Gruppe in der Seitenkette auf. Diese sind bevorzugt ausgewählt aus der Gruppe bestehend aus Hydroxyl-, Sulfhydryl-, Amin-, Amid- oder Estergruppen oder anderen Gruppen, welche die Ausbildung von Wasserstoffbrücken erlauben.

Bevorzugte neutrale polare Reste sind ausgewählt aus der Gruppe bestehend aus Asparagin, Cystein, Glutamin, Serin, Threonin, Tyrosin, Citrullin, N-Methylserin, Homoserin, allo-Threonin und 3,5-Dinitrotyrosin und $\beta$-Homoserin.

In einer bevorzugten Ausführungsform ist $P_5$ ausgewählt aus der Gruppe bestehend aus $\beta$-Cyclohexylalanin, 3,4-cis-Methanoprolin, 3,4-Dehydroprolin, Homoprolin oder Pseudoprolin. *cis*-4-Hydroxyprolin, *cis*-3-Hydroxyprolin, *trans*-3-Hydroxyprolin Asparagin, Cystein, Glutamin, Serin, Threonin, Tyrosin, Citrullin, N-Methylserin, Homoserin, allo-Threonin und 3,5-Dinitrotyrosin und $\beta$-Homoserin.

Die Reste mit einer unpolaren, hydrophoben Seitenkette sind unter physiologischen Bedingungen ungeladene Reste, bevorzugt mit einem Hydropathie-Index über 0, besonders bevorzugt über 3. Bevorzugte unpolare, hydrophoben Seitenketten sind ausgewählt aus der Gruppe bestehend aus Alkyl-, Alkylen- Alkoxy-, Alkenoxy-, Alkylsulfanyl- und Alkenylsulfanylresten mit 1 bis 10, bevorzugt 2 bis 6 C-Atomen, oder Arylresten mit 5 bis 12 C-Atomen. Bevorzugte Reste mit einer unpolaren, hydrophoben Seitenkette sind ausgewählt aus Leucin, Isoleucin, Valin, Methionin, Alanin, Phenylalanin, N-Methylleucin, *tert.*-Butylglycin, Cyclohexylalanin, $\beta$-Alanin, 1-Aminocylcohexylcarbonsäure, N-Methylisoleucin, Norleucin, Norvalin und N-Methylvalin.

Unter physiologische Bedingungen sind ein pH-Wert von pH 6 bis 8 und eine Temperatur von 30°C bis 40°C zu verstehen, bevorzugt eine Temperatur von 37°C, ein pH-Wert von 7,4 und ein osmotischer Druck von 300 mosmol/kg.

Die erfindungsgemäßen Peptide oder Peptidderivate enthalten bevorzugt mindestens 19 Aminosäurereste, bevorzugt bis zu 50 Aminosäurereste.

$Sub_1$ ist der freie N-Terminus der Aminosäure $X_1$ oder eine modifizierte N-terminale Aminogruppe. $Sub_2$ ist die freie C-terminale Carboxylgruppe der C-terminalen Aminosäure (-COOH) oder eine modifizierte C-terminale Carboxylgruppe. "Modifizierte N-terminale Aminogruppe" und "modifizierte C-terminale Carboxylgruppe" bedeutet, dass die Aminogruppe bzw. Carboxylgruppe verändert ist (z. B. reduziert oder substituiert).

$Sub_1$ stellt somit den freien N-Terminus der Aminosäure $X_1$ oder eine Modifikation der N-terminalen Aminogruppe (welche die N-terminale Aminogruppe der Aminosäure $X_1$ durch $Sub_1$ ersetzt) mit der generellen Formel $NR_1R_2$, dar. $Sub_1 = NR_1R_2$, wobei $R_1$ und $R_2$ unabhängig voneinander sind und bevorzugt aus Wasserstoff oder aus folgenden Gruppen ausgewählt werden:

(i) einer geradkettigen, verzweigten, zyklischen oder heterozyklischen Alkylgruppe, wie z. B. Methyl, Ethyl, n-Propyl, Isopropyl, n-Butyl, Isobutyl oder Cyclohexyl;

(ii) einer geradkettigen, verzweigten, zyklischen oder heterozyklischen Alkanoylgruppe, wie z. B. Acetyl oder Methanoyl (Formyl), Propionyl, n-Butyryl, Isobutyryl, Pentanoyl, Hexanoyl oder Cyclohexanoyl;

(iii) eine Reportergruppe, bevorzugt ein Fluoreszenzfarbstoff (wie z. B. Fluorescein, Alexa488) oder Biotin;

(iv) zusammen mit $COR_3$ (siehe unten) einen Linker zwischen N- und C-Terminus um ein zyklisches Peptid zu erhalten, z. B. basierend auf Guanidin, Ethyleneglycololigomere, 2,4-Diaminobuttersäure, 2,3-Diaminopropionsäure, 2,2'-Diaminopimelinsäure, Desmosin oder Isodesmosine.

(v) einen Linker zur Kopplung eines weiteren Peptids oder Peptidderivats ($Y_1$) über eine spezifische chemische oder enzymatische Reaktion, z.B. basierend auf Iod-, Brom- oder Chloralkansäuren (z.B. Iodessigsäure) oder Maleimid zur Kopplung an ein Thiol-haltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein.

(vi) einen wie in (v) genannten Linker, an den ein weiteres Peptid oder Peptidderivat $Y_1$ gekoppelt ist.

Beispiele für N-terminale Modifikationen sind acetylierte, formylierte oder guanylierte N-Termini.

Bevorzugt ist über $Sub_1$ ein weiteres Peptid oder Peptidderivat $Y_1$ gekoppelt. $Y_1$ ist vorzugsweise ein Biopolymer (z.B. Peptid), welches das antimikrobielle Peptid nach irgendeinem der Formeln 1 bis 4 in Bakterien einschleust und damit die Aktivität des antimikrobiellen Peptids gegenüber diesem Bakterium erhöht und/oder in Säugerzellen einschleust und damit die Behandlung von Bakterien ermöglicht, die sich in Säugerzellen verstecken. $Y_1$ ist über $Sub_1$ entweder permanent (z.B. Peptid- oder Amidinbindung für $Sub_1=NH_2$ oder Thioether für $Sub_1=SH$, Iodacetat oder Maleimid) oder durch eine Verbindung, die unter bestimmten Bedingungen spaltbar ist (wie z. B. Disulfidbrücken oder säurelabile Linker), mit $X_1$ des Peptids verknüpft. Bevorzugte Sequenzen für $Y_1$ sind Zell-penetrierende Peptide (CPP, cell penetrating peptides), beispielsweise Penetratin, Tat-Peptide, amphipathischen Modell-Peptiden (model amphipathic peptides) und Transportans (Langel, U.in Handbook of Cell-Penetrating Peptides 5-28 (CRC - Taylor & Francis Group, 2006).

Ein Linker ist eine Bezeichnung für Moleküle oder Molekülgruppen, die zum Verknüpfen von zwei Substanzen herangezogen werden, bevorzugte Linker enthalten zwei reaktive Gruppen (wie z. B. Iodacetat, Maleimid, Imido- oder NHs-Ester oder Hydrazid), die durch eine Molekülbrücke (z. B. Polyethylenglykol) mit bevorzugt 10 bis 20 C-Atomen verbunden sind.

$Sub_2$ ist die freie C-terminale Carboxylgruppe der C-terminalen Aminosäure (-COOH) oder eine modifizierte C-terminalen Carboxylgruppe, vorzugsweise mit der allgemeinen Formel $COR_3$ ($R_3$ ersetzt die Hydroxylgruppe der letzten Aminosäure), $X_5$-$COR_3$ oder $X_6$-$COR_3$ oder $X_5X_6$-$COR_3$.

$COR_3$ ist vorzugsweise aus der folgenden Gruppe ausgewählt:

(i) Carboxyl ($R_3$ ist eine freie Hydroxylgruppe), ein Ester ($R_3$ ist eine Alkoxygruppe), ein Amid ($R_3$ ist ein Amin) oder ein Imid;

(ii) ein Linker, der zusammen mit $Sub_1$, die N- und C-Termini zu einem zyklischen Peptid verbrückt;

(iii) $COR_3$, worin $R_3$ entweder ein zusätzlicher Aminosäurerest ist, der aus der Gruppe die Pro, Ile, Leu, Arg und Gln enthält, ausgewählt ist, oder worin $R_3$ ein Peptid, mit bevorzugt zwei bis sechs Aminosäuren ist, wovon mindestens eine Aminosäure aus der Gruppe, die Pro, Ile, Leu, Arg oder Gln enthält, ausgewählt ist, wobei diese mit einem Mitglied aus der Gruppe mit Carboxyl ($R_3$ ist eine freie Hydroxylgruppe), einem Ester ($R_3$ ist ein Alkohol, wie Methanol, Ethanol, Propanol, iso-Propanol oder Butanol), einem Amid ($R_3$ ist ein Amid) oder ein Imid ($R_3$ ist ein Alkylamin oder Dialkylamin, wie Methylamin, Ethylamin, Dimethylamin oder Zyklohexylamin) substituiert ist.

(iv) $COR_3$ worin $R_3$ eine zusätzliche, verzweigte Aminosäure ist, um eine Dimer- oder Oligomerstruktur auszubilden, wie beispielsweise Lysin, Hydroxylysin, Ornithin, 2,4-Diaminobuttersäure, 2,3-Diaminopropionsäure, 2,2'-Diaminopimelinsäure, Desmosin, Isodesmosin oder eine Kombination aus diesen verzweigten Aminosäuren.

(v) ein Linker zur Kopplung eines weiteren Peptids oder Peptidderivats ($Y_1$) über eine spezifische chemische oder enzymatische Reaktion, z.B. basierend auf Iod-, Brom- oder Chloralkansäuren (z.B. Iodessigsäure) oder Maleimid zur Kopplung an ein Thiol-haltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein.

(vi) einen wie in (v) genannten Linker, an den ein weiteres Peptid oder Peptidderivat $Y_1$ gekoppelt ist

Auf diesem Weg können C-terminale Peptidderivate als Ester ($R_3$ = Alkoxy), Amid ($R_3$ = Amin, z. B. -$NH_2$) oder Imid ($R_2$=Alkylamin, z. B. -$NHC_3H_7$) oder ein Peptid, das um weitere Aminosäuren verlängert wurde, die aus der Gruppe, die Pro, Ile, Arg und Val enthält, ausgewählt wurde und ebenfalls wieder am C-Terminus als Ester, Amid oder Imid modifiziert sind, erhalten werden. Weitere Peptidderivate können durch Modifikationen der N-terminalen oder C-terminalen Enden der Peptide gebildet werden. Diese Änderungen können beispielsweise eine zusätzliche Alkyl- oder Alkanoylgruppe (entweder mit einer geraden Kette oder verzweigt, zyklisch oder heterozyklisch) oder eine zusätzliche Guanidinogruppe oder ein zusätzliches Makromolekül oder ein Reporterrest sein, der entweder permanent oder durch eine Verbindung, die unter bestimmten Bedingungen spaltbar ist (wie Disulfidbrücken oder säurelabile Linker), verknüpft ist. Bevorzugt erfolgt eine Modifikation des C-Terminus mittels Thioestersynthese und anschließender Substitition mit primären Aminen.

Alle natürlichen Aminosäuren, unnatürlichen Aminosäuren oder Aminosäurederivate (wie z.B. Iminosäuren), welche die erfindungsgemäßen Peptide oder Peptidderivate bilden, können entweder in der L- oder D-Konformation vorliegen. Wenn nicht anders spezifiziert sind die Bausteine in den Sequenzen jedoch bevorzugt in der L-Konfonnation.

$X_5$ und $X_6$ sind optional zusätzliche Reste. In dem Fall, dass $X_5$ und $X_6$ abwesend sind, hat das letzte Arginin (Arg) in der oben erwähnten Sequenz eine freie C-terminale Carboxylgruppe oder ist mit $Sub_2$ verbunden.

In dem Fall, in dem mindestens ein Rest $X_5$ und $X_6$ vorhanden ist, hat das Peptid beispielsweise eine der folgenden allgemeinen Formeln:

$$Y_1\text{-Sub}_1\text{-}X_1\text{-D-K-P-P-Y-L-P-R-P-}X_2\text{-P-P-R-}X_3\text{-I-Y-N-}X_4\text{-}X_5\text{-}X_6\text{-COR}_3 \qquad \text{(Formel 5)}$$

$$Y_1\text{-Sub}_1\text{-}X_1\text{-D-K-P-P-Y-L-P-R-P-}X_2\text{-P-P-R-}X_3\text{-I-Y-N-}X_4\text{-}X_5\text{-COR}_3 \qquad \text{(Formel 6)}$$

$$Y_1\text{-Sub}_1\text{-}X_1\text{-D-K-P-P-Y-L-P-R-P-}X_2\text{-P-P-R-}X_3\text{-I-Y-N-}X_4\text{-}X_6\text{-COR}_3 \qquad \text{(Formel 7)}$$

$X_5$ ist aus Prolin, Prolinderivaten oder einem neutralen Rest mit einer polaren Seitenkette (wie Asparagin, Glutamin) ausgewählt. Bevorzugte Reste $X_5$ sind aus den Gruppen ausgewählt, die Prolin, cis-4-Hydroxyprolin, trans-4-Hydroxyprolin, cis-3-Hydroxyprolin, trans-3-Hydroxyprolin, β-Cyclohexylalanin, 3,4-cis-Methanoprolin, 3,4-Dehydroprolin, Homoprolin, Pseudoprolin ebenso wie Asparagin, Glutamin, Citrullin, N-Methylserin, N-Methylglycin, Dihydroxyphenylalanin, N-Ethylasparagin, N-Ethylglycin, Homoserin, Penicillamin, Tetrahydropyranylglycin, allo-Threonin und 3,5-Dinitrotyrosin enthalten.

$X_6$ ist aus Prolin, Prolinderivaten, einem polaren Rest (wie Serin) oder einen hydrophoben Rest ausgewählt. Bevorzugte Reste $X_6$ sind aus den Gruppen ausgewählt, die Prolin, cis-4-Hydroxyprolin, trans-4-Hydroxyprolin, cis-3-Hydroxyprolin, trans-3-Hydroxyprolin, β-Cyclohexylalanin, 3,4-cis-Methanoprolin, 3,4-Dehydroprolin, Homoprolin oder Pseudoprolin, Serin, Threonin, δ-Hydroxylysin, Citrullin, Homoserin oder allo-Threonin ebenso Phenylalanin, N-Methylleucin, Leucin, Isoleucin, Valin, Methionin, *tert.*-Butylglycin, Cyclohexylalanin, Alanin, β-Alanin, 1-Aminocylcohexylcarbonsäure, N-Methylisoleucin, Norleucin, Norvalin, N-Methylvalin enthält oder es ist eine kurze Peptidsequenz mit vorzugsweise einem bis drei Resten, die bevorzugt ausgewählt sind aus Prolin, Isoleucin oder einem der zuvor erwähnten Reste.

Alternativ ist $X_6$ ein verzweigter Linker, der mehrere Peptideinheiten enthält. Dieser wird durch den Rest einer Aminosäure, die mehrere Aminogruppen enthält, wie z. B. Lysin, Hydroxylysin, Ornithin, 2,4-Diaminobuttersäure, 2,3-Diaminopropionsäure, 2,2'-Diaminopimelinsäure, Desmosin, Isodesmosin gebildet.

Die C-terminale Aminosäure ist zum Beispiel $X_4$ in den Formeln 1 bis 4, $X_5$ in Formel 6 oder $X_6$ in Formel 5 und 7.

Die erfindungsgemäßen Peptide und Peptiderivate zeigen gegenüber der bisher unvollständig bestimmten Sequenz des Prolin-reichen antimikrobiellen Peptids "Oncopeltus antibakterielles Peptid 4" eine verbesserte anti-bakterielle Aktivität, ein weiteres Wirkungsspektrum und eine erhöhte Proteaseresistenz.

Die erfindungsgemäßen Peptide und/oder multimeren Peptidkonstrukte, die modifiziert wurden, um die antirnikrobielle oder antimykotische Aktivität zu steigern und das Aktivitätsspektrum auf andere Bakterien oder Pilze zu erweitern und die Stabilität zu verbessern, zeichnen sich durch ihre hohe antibakterielle und/oder antimykotische Wirksamkeit und durch eine gute metabolische Stabilität in Säugetierserum aus.

Geeignete Modifikationen in den Positionen 11 ($X_2$), 15 ($X_3$) und 19 ($X_4$) verbessern die antibakterielle Aktivität der nativen Oncopeltus 4-Sequenz gegen verschiedene Bakterien, wie weiter unten diskutiert und in den Beispielen gezeigt wird.

Weiterhin können die Reste $Sub_1$-$X_1$, $X_3$ und $X_4$ die N- und C-terminalen Peptidsequenzen gegen proteolytische Abbau zusätzlich stabilisieren und so die Halbwertszeit in Serum erhöhen.

Die erfindungsgemäßen Sequenzen haben einen positiv geladenen Rest $X_2$ (Position 11).

Bevorzugte erfindungsgemäße Beispiele sind Sequenzen mit einem positiv geladenen Rest $X_3$ (Position 15).

Die erfindungsgemäßen Sequenzen haben einen positiv geladenen Rest $X_4$ (Position 19).

Des Weiteren ist die C-terminale Carboxylgruppe vorzugsweise modifiziert. Erstaunlicherweise führt dies zu einer erhöhten Halbwertszeit der Peptide in Serum.

Die Modifikationen der N- und C-Termini erlauben das Koppeln der Peptide an andere Gruppen, wie zum Beispiel andere Ärninosäuresequenzen (dabei werden eventuell multimere Peptide oder Proteine geschaffen) oder andere Biomoleküle, welche die Funktion eines Carriers oder Labels haben, beispielsweise von $Y_1$ über $Sub_1$. In einer speziellen Ausfüluungsform fungiert das Carriermolekül als Shuttle um die bakterielle Infektion in Säugerzellen zu bekämpfen oder das antibakterielle Peptid und Peptidderivat in Bakterien zu transportieren, in die das antibakterielle Peptid nicht allein eindringen kann (z.B. Gram-positive Bakterien). Beispiele für solche Zell-penetrierenden Peptide (CPP) sind beispielsweise Penetratinen, Tat-Peptide, amphipathische Modell-Peptiden (model amphipathic peptides) und Transportans. Zudem kann der Ort der Infektion durch die gekoppelte Struktur (Target-Molekül) erkannt werden und dadurch die antibiotische Substanz in die Nähe der (Bakterien-) Zelle gebracht werden, um diese bekämpfen. Solche Target-Moleküle sind z.B. Moleküle, die bekanntermaßen an Lipopolysaccharid (LPS)-Moleküle binden, welche die Außenseite der Gram-negativen Bakterien bilden. Bekannte Verbindungen für diese Anwendung sind beispielsweise Ankerpeptide, wie das AcmA-Motif aus Lactobacillus oder ein gegen Lipopolysaccharid gerichteter Antikörper. Die letztere Variante ist bevorzugt, da sie auch einen intrinsischen antibiotischen Effekt hat und deshalb zur Steigerung der Aktivität der erfindungsgemäßen Peptide genutzt werden kann.

Weitere bevorzugte Beispiele der Erfindung sind Sequenzen mit positiv geladenen Resten an $X_2$, $X_3$ und $X_4$ (Position 11, 15 und 19), wie z. B. die Sequenzen, die aus den Sequenzen gemäß SEQ ID NO. 62, 63, 65 bis 74, ausgewählt sind.

Besonders bevorzugte Beispiele sind Peptide, die eine positiv geladene Aminosäure in Positionen 11, 15 und 19 ($X_2$, $X_3$ und $X_4$) tragen (wie Ornithin, Arginin oder Lysin) und einen modifizierten C-Terminus aufweisen, insbesondere die Peptide gemäß SEQ ID NO. 63, 71, 72, 74.

Ein besonders bevorzugtes Peptid enthält Ornithin in Position 15 (Rest $X_3$), Arginin in Positionen 11, und 19 (Reste $X_2$ und $X_4$) und den C-Terminus als Propylamid (Rest $Sub_2$) gemäß SEQ ID NO. 71.

Ein anderes besonders bevorzugtes Peptid enthält Ornithin in Position 15 und 19 (Rest $X_3$ und $X_4$ und Arginin in Position 11 (Rest $X_2$) den C-Terminus als Amid (Rest $Sub_2$). Ein derart bevorzugtes Peptid hat die Sequenz gemäß SEQ ID NO. 72.

Ein weiteres besonders bevorzugtes Peptid enthält Arginin in Position 11 (Rest $X_2$), trans-4-Hydroxyprolin in Position 15 (Rest $X_3$) und Ornithin in Position 19 (Rest $X_4$) und den C-Terminus als Amid (Rest $Sub_2$). Ein derart bevorzugtes Peptid hat die Sequenz gemäß SEQ ID NO. 63.

Ein anderes besonders bevorzugtes Peptid enthält Ornithin in Position 15 und 19 (Rest $X_3$ und $X_4$, Arginin in Position 11 (Reste $X_2$), in Position 18 Glutamin statt Asparagin und den C-Tenninus als Amid (Rest $Sub_2$). Ein derart bevorzugtes Peptid hat die Sequenz gemäß SEQ ID NO. 74.

An Hand der Beispiele sieht man, dass die erfindungsgemäßen Modifikationen des C-Terminus zu einem Amid ($Sub = -NH_2$) erstaunlicherweise die antibiotische Wirkung gegen *E. coli* und *M. luteus* signifikant erhöht. Bevorzugte Sequenzen mit einem Amid am C-Terminus sind die SEQ ID NO. 18, 22, 50, 54 bis 57, 61 bis 63, 65 bis 70, 72 bis 79 und 82.

Die experimentellen Ergebnisse zeigen, dass offenbar die Aminosäuren an Position 6 und 7 ebenfalls sehr wichtig für die antibiotische Wirkung sind. Denn der Austausch einzelner Aminosäuren an diesen Position 6 und/oder 7 durch Alanin vernichtet die Effektivität im Vergleich zu der antibiotischen Aktivität des Oncocins.

Die am meisten bevorzugten Beispiele der Erfindung sind Peptide, welche die folgenden Vorteile erfüllen:

(i) eine gesteigerte Halbwertszeit in Säugetierserum und

(ii) eine gesteigerte antimikrobielle Aktivität gegen einen oder mehrere Bakterienstämme, besonders humane Pathogene, oder Pilze oder andere mikrobielle Infektionen und

(iii) die Peptide sind nicht toxisch gegenüber humanen Zellen, einschließlich Erythrozyten.

Die Wirkung von antimikrobiellen Peptiden ist sehr komplex, da sie die Zellmembran durchdringen und in das Zytoplasma eindringen müssen, um ein spezielles intrazelluläres bakterielles Zielmolekül zu inhibieren, ohne jedoch auf Säugetierzellen und Blutzellen toxisch zu wirken. Ein anderer wichtiger Punkt ist die Stabilität der Peptide oder Peptidderivate gegenüber dem Abbau durch Peptidasen oder Proteasen. Daher hat das ideale Peptid eine hohe antibakterielle Aktivität (kleine MHK-Werte), keine Zelltoxizität, keine hämolytische Aktivität und eine Halbwertszeit von mehreren Stunden in Blut. Im Vergleich zur nativen Oncopeltus 4-Sequenz zeigen die erfindungsgemäßen Peptidderivate eine mehr als zwanzigfach höhere antimikrobielle Aktivität. Der C-Terminus ist vorzugsweise modifiziert (Amid, Alkylamid, Ester) und der C-terminale Bereich nach Position 14 beispielsweise durch Substitution der Positionen 15 und/oder 19 ($X_3$ und $X_4$) mit nichtproteinogenen Aminosäuren verändert. Am N-Terminus ist eine positive Ladung bevorzugt, um eine gute Aktivität zu erreichen. Das Valin in Position 1 ($X_1$) der nativen Oncopeltus 4-Sequenz ist vorzugsweise frei oder durch einen acetylierten basischen Rest wie Arginin, Lysin oder Ornithin ersetzt. Besonders bevorzugt ist dabei Ornithin, was überraschenderweise zu einer erhöten Proteinstabilität führt. Aus demselben Grund werden Positionen 15 und 19 der Oncopeltus 4-Sequenz vorzugsweise substituiert, was erstaunlicherweise zu einer erhöten Peptidstabilität führt. Beispiele sind der Austausch der Positionen 15 und 19 ($X_3$ und $X_4$) gegen *trans*-4-Hydroxyprolin ($X_3$) und Onithin ($X_4$) oder beide gegen Ornithin, was die Halbwertszeit in 25% Serum unerwartet um mehr als den Faktor 10 erhöht, d.h. von weniger als 30 min auf über 6 h. Die überführung des C-Terminus von der freien Säure zu einem Propylamid führt ebenfalls zu einer Erhöhung der Serumstabilität Bevorzugt wird an die erfindungsgemäßen Peptide und Peptidderivate eine Zell-penetrierende Peptidsequenz gekoppelt. Bevorzugt erfolgt diese Kopplung über einen Linker, wie z. B. eine Acetylgruppe oder eine Alkylgruppe. Vorzugsweise erfolgt die Kopplung an den N-Terminus des erfindungsgemäßen Peptids und Peptidderivates. Bevorzugt wird dazu das Prolin-reiche Peptid oder Peptidderivat N-Terminal mit lodacetat derivatisiert und die Zell-penetrierende Peptidsequenz C-terminal um einen Cysteinrest verlängert. Die Thiolgruppe dieses Cysteins bildet dann mit der Acetylgruppe eine Thioetherbrücke.

Zell-penetrierende Peptide (CPP) sind relativ kurze polykationische oder hydrophobe Peptide, deren Anbindung die Passage durch die Zellmembran von pro- und eukaryontischen Zellen ermöglichen. CPPs können unterschiedliche Sequenzen und Längen aufweisen. In den meisten Fällen enthalten sie jedoch eine Sequenz von ca. 10 bis 40 Aminosäuren und sind reich an positiv geladenen Aminosäuren (z. B. Arg, Lys). Diese kurzen Sequenzen sind verntwortlich für die Passage durch die Zellmembran und werden "protein transduction domains (PTDs)" genannt.

Bevorzugte Zell-penetrierende Peptide sind ausgewählt aus Penetratin, Tat-Peptiden, model amphipathic peptides, Transportan (abgeleitet von Galanin), SynB (abgeleitet von Protegrin) und cis-γ-amino-L-Prolin-haltigen Peptiden.

Die erfindungsgemäß verwendeten Zell-penetrierenden Peptidsequenzen sind bevorzugt 8 bis 20 Aminosäurereste lang, wobei 30 % bis 90 % der Reste Seitenketten aufweiset, die unter physiologischen Bedingungen positiv geladen sind. Die verbleibenden Reste sind bevorzugt neutral. Bevorzugte Zell-penetrierende Peptidsequenzen sind ausgewählt aus:

RQIKIWFQNRRMKWKK-OH SEQ ID NO. 105 (ein Penetratin),
KLALKLALKALKAALKLA-NH$_2$ SEQ ID NO. 124 (amphipathisches Modell-Peptid)
RKKRRQRRR SEQ ID NO. 125 (ein Tat-Peptid).

Weitere bevorzugte Zell-penetrierende Peptidsequenzen werden in Literaturstelle (Langel, U.in Handbook of Cell-Penetrating Peptides 5-28 (CRC - Taylor & Francis Group, 2006), (Pujals S, Giralt E. Proline-rich, amphipathic cell-penetrating peptides Adv Drug Deliv Rev. 60(4-5): 473-84, 2008) und (Farrera-Sinfreu J, Giralt E, Royo M, Albericio F. Cell-penetrating proline-rich peptidomimetics. Methods Mol Biol. 386: 241-67, 2007) genannt; In bevorzugten Beispiele für derartige Peptidderivate wurden die AMP N-terminal vor Position 1 ($X_1$) um Penetratin-Cystein ($Y_1$) über lodacetyl (Sub$_1$) unter Ausbildung einer Thioetherbindung verlängert. Derart bevorzugte Beispiele sind vorzugsweise ausgewählt aus den Sequenzen gemäß SEQ ID NO. 101 und 102.

Durch die Ankoppelung einer Zell-penetrierenden Peptidsequenz, wie Penetratin, wird erstaunlicherweise die Aktivität gegenüber Gram-negativen und Gram-positiven Bakterien gesteigert und das Wirkungsspektrum auf andere Gram-positive und Gram-negative Bakterien erweitert und zusätzlich werden die antimikrobiellen Peptide in Säugerzellen eingeschleust werden ohne Zytotoxisch zu sein, so dass auch in diesen Zellen verborgene Bakterien, Pilze oder Viren erreicht werden können.

Penetratin entspricht der Teilsequenz R43 bis K58 der Antennapedia Homöodomäne (DNAbindende Region eines Transkriptionsfaktors), der Fruchtfliege *Drosophila melanogaster.* Die Sequenz der Penetratine (bevorzugt R Q I K I M F Q N R R M K N K K-OH; SEQ ID NO. 105) ist reich an kationischen Aminosäuren und ähnelt darin den Sequenzen vieler AMP.

In dieser Erfindung wird die Zell-penetrierende Peptidsequenz zur Einschleusung von AMP sowohl in Bakterien als auch Säugerzellen genutzt. Gekoppelt an Penetratin werden die AMP in eukaryotische Zellen transportiert, um auch dort Infektionen zu behandeln. Zusätzlich können toxische Effekte durch Interaktion der AMP mit intrazellulären Zielmolekülen untersucht werden.

Bestandteil der Erfindung ist die Kopplung des Penetratins an die erfindungsgemäßen antimikrobiellen Peptide über eine Thioetherbrücke.

Dabei wurde der C-Terminus des Penetratins um ein Cystein verlängert und an das N-terminal mit lodessigsäure markierte antimikrobielle Peptid gekoppelt.

Der Ausdruck "Peptid", wie hier verwendet, steht für eine Sequenz von Aminosäuren, die über eine Peptidbindung verknüpft sind, wobei die Aminosäuren bevorzugt aus den zwanzig natürlich vorkommenden Peptid-bildenden Aminosäuren ausgewählt sind und worin die Aminosäuren in der L-Konfiguration oder D-Konfiguration, oder im Fall von Isoleucin und Threonin, auch in der D-allo-Konfiguration vorliegen können (nur Inversion eines der beiden chiralen Zentren).

Der in der Erfindungsbeschreibung verwendete Ausdruck Peptidderivat (oder Peptidomimetika) beinhaltet nicht nur Peptide, die am N- oder C-Terminus, wie oben beschrieben, mit $Y_1$, $Sub_1$ und $Sub_2$ modifiziert sind. Er umfasst zudem Peptide, die durch Substitutionen und/oder Modifikationen von einem oder mehreren Aminosäureresten durch chemische Gruppen verändert wurden, wobei diese chemischen Gruppen andere als die natürlichen Protein-bildenden Aminosäurereste sind, wie z.B. nicht-proteinogene $\alpha$-Aminosäuren, $\beta$-Aminosäuren oder Peptide mit verändertem Rückgrat. Der Begriff "verändertes Rückgrat" bedeutet, dass mindestens eine Peptidbindung chemisch modifziert ist, d. h. ersetzt ist durch eine unter physiologischen Bedingungen nicht spaltbare Bindung, die nicht durch Endoproteasen geschnitten werden kann.

Bevorzugt ist die nicht spaltbare Bindung eine modifizierte Peptidbindung wie z. B. eine reduzierte Peptidbindung, eine alkylierte Amidbindung oder eine Thioamidbindung. Eine reduzierte Amidbindung ist eine Peptidbindung in der die Carboxylgruppe (C=O) zu einer Hydroxylgruppe (HCOH) oder einer Methylengruppe ($CH_2$) reduziert ist. Eine alkylierte Amidbindung ist eine entweder am Stickstoff (N-alpha) oder Kohlenstoffatom (C-alpha) alkylierte Peptidbindung. Der Alkylrest hat bevorzugt 1 bis 3 C-Atome. Ein Beispiel ist die N-Methylierung.

Außerdem umfasst der Begriff verändertes Rückgrat andere Gruppen, die geeignet sind, eine kovalente Bindung sowohl mit der COOH-Gruppe des vorangegangenen Aminosäurerestes als auch der $NH_2$-Gruppe des folgenden Aminosäurerestes zu bilden, und die daher nicht notwendigerweise die Peptidrückgrat-Struktur aufrecht erhalten, wie z. B. Zuckeraminosäure-Dipeptid-Isostere, Azapeptide, 6-homopolymere, gamma-Peptide, Y-Lactam-Analoga, Oligo(phenylenethylen)e, vinyloge Sulfonpeptide, poly=N-substituierte Glycine oder Oligocarbamate.

Modifikationen des Rückgrats sind an Positionen 14 bis 19, $R-X_3-I_{16}-Y_{17}-N_{18}-X_4$. Daher ist vorzugsweise mindestens eine der Bindungen zwischen $X_3$- $I_{16}$ (z.B. Arg-Ile), $N_{18}-X_4$ (z.B. Asn-Arg), $X_4-NH_2$ (z.B. Arg-$NH_2$), $X_6-X_7$ (z.B. Arg-Leu oder Arg-Ile) modifiziert. Diese Bindungen sind vorzugsweise aus der Gruppe der reduzierten Amidbindungen, alkylierten Amidbindungen oder Thioamidbindungen ausgewählt.

Die erfindungsgemäßen Peptide und Peptidderivate können linear sein, d.h. eine Sequenz, in der die erste und die letzte Aminosäure der Sequenz eine freie $NH_2$ und COOH-Gruppe besitzen oder durch $Sub_1$ und $Sub_2$ modifiziert sind. Alternativ sind die Peptide zyklisch, d. h. die erste und die letzte Aminosäure sind über eine Peptidbindung oder einen Linker verknüpft.

Weitere Bestandteile dieser Erfindung sind die Methoden zur Herstellung der oben erwähnten neuartigen antibiotisch wirksamen Verbindungen.

Die Peptide oder Peptidderivate dieser Erfindung können entweder synthetisch oder, wo anwendbar, rekombinant mit konventionellen Methoden hergestellt werden. Spezielle Ausführungsbeispiele der Erfindung werden ausführlich im experimentellen Teil unten offenbart. Vorzugsweise werden die Peptide oder Peptidderivate dieser Erfindung konventionell mit den bekannten Synthesetechniken hergestellt, wie beispielsweise von Merrifield beschrieben (Merrifield R B. Solid Phase Peptide Synthesis .1. Synthesis of A Tetrapeptide. Journal of the Americau Chemical Society 85: 2149-&, 1963).

Alternativ werden die in dieser Erfindung beschriebenen Peptide durch rekombinante Techniken hergestellt, indem man ein DNA-Fragment, welches eine Nukleinsäuresequenz enthält, die für eines der oben beschriebenen Peptide kodiert, kloniert, und z. B. in einem Mikroorganismus oder einer Wirtszelle exprimiert. Die kodierenden Nukleinsäuresequenzen können synthetisch hergestellt werden (Stemmer W P C, Crameri A, Ha K D, Brennan T M, & Heyneker H L. Single-Step Assembly of A Gene and Entire Plasmid from Large Numbers of Oligodeoxyribonucleotides. Gene 164: 49-53, 1995) oder durch seitenspezifische Mutagenese einer existierenden Nukleinsäuresequenz (z.B. Sequenz die das Wildtyp-Oncopeltus 4 kodiert) gewonnen werden. Die so hergestellte kodierende Sequenz kann von der RNA (oder DNA) mit entsprechend hergestellten Primern in einer Polymerasekettenreaktion (PCR) mit bekannten Techniken amplifiziert werden. Nach Reinigung, beispielsweise mittels Agarose-Gelelektrophorese, wird das PCR-Produkt in einen Vektor ligiert und letztlich die Wirtszelle mit dem entsprechenden rekombinanten Plasmid transformiert. Rekombinante Techniken sind für unterschiedliche Wirtszellen bekannt, beispielsweise *E. coli, Bacillus, Lactobacillus, Streptomyces,* Säugerzellen (z.B. CHO (Chinese hamster ovary) oder COS-1 Zellen), Hefezellen (z.B. *Sacchanomyces, Schizophyllum*), Insektenzellen oder virale Expressionssysteme (z.B. Baculovirus System). Die Auswahl weiterer geeigneter Wirtszellen und Methoden zur Transformierung, Kultivierung, Amplifizierung, Screening, Produktherstellung und Reinigung können von jedem, der den Stand der Technik beherrscht, aus der Literatur übernommen werden(Gething M J & Sambrook J. Cell-Surface Expression of Influenza Hemagglutinin from A Cloned Dna Copy of the Rna Gene. Nature 293: 620-5, 1981). Nach konventioneller rekombinanter Herstellung können die Peptide dieser Erfindung aus den Wirtszellen isoliert werden, entweder mit klassischen Zellaufschlusstechniken oder vom Zellmedium mit konventionellen Methoden, z.B. Flüssigchromatographie, insbesondere der Affinitätschromatographie. Das antimikrobielle Peptid kann als einzelnes Peptid oder als Oligomer exprimiert werden. Dabei können die Oligomere mehrere Peptidsequenzen enthalten, die über den N- oder C-Terminus verknüpft sind, oder gar einen N- oder C-terminalen Tag enthalten, der die einfachere Reinigung der rekombinanten Peptide oder Proteinkonstrukte erlaubt. Konventionelle molekularbiologische Techniken und ortsspezifische Mutagenese können eingesetzt werden, um die Sequenz weiter zu verändern und so die gewünschten nicht-nativen Peptidsequenzen zu erhalten. Alle diese rekombinaten Techniken sind dem Fachmann bekannt und wurden bereits für viele antimikrobielle Peptide einschließlich Apidaecin(Maeno M, Taguchi S, & Momose H. Production of Antibacterial Peptide Apidaecin Using the Secretory Expression System of Streptomyces. Bioscience Biotechstology and Biochemistry 57: 1206-7, 1993), Perinerin (Zhou Q F, Luo X G, Ye L, & Xi T. High-level production of a novel antimicrobial peptide perinerin in Escherichia coli by fusion expression. Current Micnobiology 54: 366-70, 2007) und Defensin (Si L G, Liu X C, Lu Y Y, Wang G Y, & Li W M. Soluble expression of active human beta-defensin-3 in Escherichia coli and its effects on the growth of host cells. Chinese Medical Journal 120: 708-13, 2007) angewandt.

Es ist auch möglich nicht natürlich vorkommende Aminosäuren gentechnisch in die Peptide einzubringen. Dies wurde ausführlich von Noren et al. und Ellman et al. (Noren C J, Anthonycahill S J, Griffith M C, & Schultz P G. A General-Method for Site-Specific Incorporation of Unnatural Amino-Acids Into Proteins. Science 244: 182-8, 1989; Ellman J, Mendel D, Anthonycahill S, Noren C J, & Schultz P G. Biosynthetic Method for Introducing Unnatural Amino-Acids Site-Specifically Into Proteins. Methods in Enzmology 202: 301-36, 1991) beschrieben.

Anschließend können die Peptide aus der Wirtszellkultur oder dem *in-vitro*-Translationssystem isoliert werden. Dies kann mit den üblichen Techniken zur Proteinreinigung und -isolierung erreicht werden, die Stand der Technik sind. Solche Techniken können beispielsweise die Immunadsorption oder Affinitätschromatographie beinhalten. Es ist außerdem möglich, die Peptide während der Synthese mit einem Tag zu versehen (z.B. Histidin-Tag), der eine schnelle Bindung und Reinigung gestattet. Der Tag kann nachträglich enzymatisch abgespalten werden, um die aktive Peptidsequenz zu erhalten.

Falls das Peptid selbst nicht kodiert oder exprimiert werden kann, aber sehr ähnlich zu einem kodierbaren oder exprimierbaren Peptid ist, kann die Methode zunächst auf das ähnliche Peptid angewandt werden, um dieses nachträglich in einem oder mehreren Schritten chemisch oder enzymatisch in das gewünschte Peptid oder Peptidomimetika zu überfahren. Einige umfassendere Zusammenstellungen dieser Methoden zur Herstellung der hier beschriebenen Peptide sind in der Literatur beschrieben (Anderson W F. Human gene therapy. Nature 392: 25-30, 1998; Pharmaceutical Biotechnology (eds. Crommelin D J A & Sindelar R D) pp. 8-20, 53-70, 123-152, 167-180 (Harwood Academic Publishers, 1997; Protein Synthesis: Methods and Protocols (ed. Martin R) 1-144 (Humana Press, 1998; Amino Acid and Peptide Synthesis (ed. Jones J) 1-89 (Oxford University Press, 1997; Solid-Phase Peptide Synthesis (ed. Fields G B) 1-780 (Academic Press, 1997).

Die erfindungsgemäßen Peptide und Peptidderivate können einzeln, in Kombination, als Multimere oder als verzweigte Multimere eingesetzt werden. Sinnvolle Kombinationen der erfindungsgemäßen Peptide umfassen Konkatamere, in denen die erfindungsgemäßen Peptide seriell miteinander oder über Spacer miteinander verknüpft sind, z. B. in der Form eines Peptiddimers oder eines Peptidtrimers usw. (Multimer), indem die einzelnen Peptide aneinander gereiht sind. Dieses Multimer kann aus Peptiden oder Peptidderivaten mit identischen Sequenzen oder verschiedenen Sequenzen gemäß irgendeiner der Formeln 1 bis 4 zusammengesetzt sein.

Einzelne Peptide oder Peptidderivate können an ein biokompatibles Protein gekoppelt werden, beispielsweise humanes Serumalbumin, humanisierte Antikörper, Liposomen, Mizellen, synthetische Polymere, Nanopartikel und Phagen. Alternativ können Multimere, in denen die erfindungsgemäßen Peptide oder Peptidderivate individuell kombiniert sind, in der Form von Dendrimeren oder Clustern hergestellt werden, wobei drei oder mehr Peptide an ein Zentrum gebunden sind.

In einer Ausführungsform können mehrere Peptide oder Peptidderivate als multimere Konstrukte oder Anordnung hergestellt werden. So können beispielsweise optional Aminosäuren (z.B. Gly-Ser-) oder andere Spacer basierend auf Aminosäuren oder anderen chemischen Verbindungen an den N- oder C-Terminus angehängt werden, um zwei oder mehr Peptide untereinander zu verknüpfen oder an einen Träger zu koppeln. Diese Anordnung kann die Form von einem oder mehreren der oben beschriebenen synthetischen Peptide gekoppelt an ein Trägerprotein annehmen. Alternativ enthält eine Anordnung mehrere Peptide, jedes als multiples antigenes Peptid exprimiert, optional an ein Trägerprotein gekoppelt. In einer weiteren Variante sind die ausgewählten Peptide sequentiell verknüpft und werden als rekombinantes Protein oder Polypeptid exprimiert. In einer Ausführungsform werden mehrere Peptide sequentiell, mit oder ohne Aminosäuren als Spacer dazwischen, verknüpft, um ein größeres rekombinantes Protein zu erhalten. Alternativ kann das rekombinante Protein an ein Trägerprotein fusioniert werden.

In einer anderen Ausführungsform enthalten die multimeren Konstrukte mindestens zwei der oben definierten Peptide (welche dieselben oder unterschiedliche Peptide sein können), wobei ein Peptid über eine beliebige Aminosäure an die anderen Peptide gekoppelt wird. Eine beliebige Anzahl weiterer Peptide können an beliebige weitere Aminosäuren dieser Peptide angehängt werden. In einer weiteren Ausführungsform einer multimeren Anordnung, welches mindestens zwei Peptide enthält, ist das zweite oder sind die weiteren Peptide an ein verzweigte Gerüst der anderen Peptide der Grundstruktur gekoppelt. Alternativ ist jedes weitere Peptid kovalent über die Gruppe $Sub_1$ oder $Sub_2$ an ein anderes Peptid der Anordnung verknüpft.

In einer anderen Ausführungsform eines multimeren Konstrukts oder einer Anordnung mit mindestens zwei Peptiden, sind mindestens eins oder mehrere Peptide an einen Träger gebunden. In einer anderen Ausführungsform sind ein oder mehrere der genannten Peptide ein synthetisches Peptid, das an ein Trägerprotein fusioniert ist. Weiterhin gibt es die Alternative mehrere der oben beschriebenen Peptide mit oder ohne flankierende Sequenzen sequentiell zu einem linearen Polypeptid zu kombinieren. Die Peptide oder das Polypeptid sind entweder an den gleichen Träger gekoppelt oder unterschiedliche Peptide können individuell als Peptide an eins oder unterschiedliche immunologisch inerte Trägerproteine gekoppelt werden.

Geeignete Träger verbessern die Stabilität, die Darreichung oder die Produktion, oder verändern bzw. verbessern das Aktivitätsspektrum der Peptide. Beispiele für Träger sind humanes Albumin, Polyethylenglykol oder andere Biopolymere bzw. andere natürlich oder nicht-natürlich vorkommende Polymere. In einer Ausführungsform, ist die Hauptkomponente des Trägers vorzugsweise ein Protein oder anderes Molekül, das die Peptidstabilität erhöt. Eine erfahrene Person kann einfach eine geeignete Kopplungseinheit von Träger und Peptid auswählen.

In einer weiteren Ausführungsform sind die Peptide in der Form eines multiplen Antigenpeptides (multiple antigenic peptide; MAP) angeordnet, die beispielsweise nach dem von Tam et al. (Tam J P, Mora A L, & Rao C. Lipidation as a novel approach to mucosal immunization. Modulation of the Immune Response to Vaccine Antigens 92: 109-16, 1998) beschriebenen "MAP"-Konzept konstruiert werden können. Dieses System nutzt eine zentrale Einheit aus Lysinresten, an die mehrere Kopien des gleichen erfindungsgemäßen Peptids synthetisiert werden (siehe z. B. Posnett D N, Mcgrath H, & Tam J P. A Novel Method for Producing Anti-Peptide Antibodies - Production of Site-Specific Antibodies to the T-Cell - Antigen Receptor Beta-Chain. Journal of Biological Chemistry 263: 1719-25, 1988). Jedes MAP enthält mehrere Kopien eines oder mehrerer der erfindungsgemäßen Peptide. Eine Ausführungsart eines MAP enthält mindestens drei, vorzugsweise aber vier oder mehr Peptide. Ein Fachmann kann einfach eine beliebige Anzahl multimerer Verbindungen gemäß der in obigen Formeln identifizierten Peptiden herstellen. Alle derartigen multimeren Arrangements und Konstrukte sind Bestandteil dieser Erfindung.

Weitere Kombinationen in der Form von Multimeren können an der Oberfläche von Partikeln hergestellt werden, wobei die Peptide oder Peptidomimetika an deren Oberfläche präsentiert werden. Der Partikel kann dann als Träger eines Peptids oder Peptidomimetikas fungieren und kann gleichzeitig als detektierbarer Marker wirken. Multimere können beispielsweise durch N-terminale Biotinylierung des N-terminalen Endes der Peptid- oder Peptidomimetika-Ketten und anschließende Komplexbildung mit Streptavidin erhalten werden. Da Streptavidin vier Biotinmoleküle oder -konjugate mit hoher Affinität binden kann, werden mit dieser Methode sehr stabile tetramere Peptidkomplexe erhalten. Multimere können aus identischen oder unterschiedlichen erfindungsgemäßen Peptiden oder Peptidomimetika hergestellt werden. Vorzugsweise enthalten die erfindungsgemäßen Multimere zwei oder mehr Peptid oder Peptidomimetika, in welcher jede Komponente einen Anteil zur bioziden Aktivität beiträgt (Zielerkennung, antimikrobielle Aktivität, Reinigung).

Ein anderer Gegenstand dieser Erfindung ist der Einsatz der erfindungsgemäßen Peptide oder Peptidderivate in der Medizin oder Pharmazie, z.B. zur Therapie mit einem Antibiotikum oder in einer Zusammensetzung mit antimikrobieller (insbesondere bakteriozider) Aktivität.

Die Erfindung umfasst auch ein erfindungsgemäßes Peptid, Peptidderivat und/oder Multimer zur Anwendung in der Behandlung von mikrobiellen, bakteriellen oder Pilz-Infektionen.

Ein weiterer Gegenstand dieser Erfindung sind pharmazeutische Zusammensetzungen, die ein oder mehrere erfindungsgemäße Peptide bzw. Peptidderivate oder multimere Konstrukte unabhängig von der Anwesenheit anderer pharmazeutischer aktiver Verbindungen enthält.

Bestandteil dieser Erfindung ist auch die Verwendung der erfindungsgemäßen Peptide als Pharmazeutikum und/oder zur Herstellung eines Wirkstoffs, der als Antibiotikum eingesetzt werden kann.

Die Peptide können auch einzeln in phannazeutischen Produkten eingesetzt werden. Alternativ können ein oder mehrere Peptide, wie oben beschrieben, an eine andere Verbindung fusioniert oder konjugiert werden, um die Pharmakokinetik oder Bioverfügbarkeit zu steigern, ohne dass eine Immunantwort ausgelöst wird. Eine beliebige Anzahl einzelner Peptide oder multimerer Konstrukte können miteinander gemischt werden, um eine einzelne Zusammensetzung herzustellen.

Eine erfindungsgemäße pharmazeutische Zusammensetzung enthält eine therapeutisch wirksame Menge eines oder mehrer Peptide oder Peptidderivate dieser Erfindung. Einmal zusammengestellt, kann die erfindungsgemäße pharmazeutische Zusammensetzung dem Subjekt direkt verabreicht werden, um mikrobielle (insbesondere bakterielle) Infektionen zu behandeln. Dazu wird dem zu behandelnden Subjekt eine therapeutisch wirksame Menge einer erfindungsgemäßen Zusammensetzung verabreicht.

Die erfindungsgemäßen Zusammensetzungen sind dazu bestimmt Infektionen eines mit Bakterien oder Pilzen infizierten Säugetiers einschließlich des Menschen zu behandeln.

Mindestens ein oder alternativ auch mehrere erfindungsgemäße Peptide oder multimere Konstrukte können zu einer antimikrobiell (insbesondere antibakteriell oder fungizid) wirksamen Zusammensetzung mit einem pharmakologisch vertretbaren Träger oder anderen Komponenten gemischt werden. Für den Gebrauch einer derartigen Zusammensetzung wird das ausgewählte Peptid vorzugsweise synthetisch oder auch rekombinant, wie oben beschrieben, hergestellt.

Die direkte Verabreichung dieser Zusammensetzung erfolgt entweder topisch (oberflächlich auf die Haut) oder in einer anderen Administrationsroute, wie z. B. oral, parenteral, subkutan, sublingual, intraläsional, intraperitoneal, intravenös, intramuskulär, pulmonal oder interstitiell ins Gewebe.

Die pharmazeutische Zusammensetzung kann weiter geeignete und pharmazeutisch akzeptable Träger, Streckmittel oder Lösungsmittel enthalten und die Form einer Kapsel, Tablette, Pastille, Dragee, Pille, Tropfen, Zäpfchen, Puder, Spray, Impfstoff, Salbe, Paste, Creme, Inhalat, Pflaster, Aerosol oder ähnlichem aufweisen. Als pharmazeutisch akzeptable Trägerstoffe können Lösungsmittel, Streckmittel oder andere flüssige Bindemittel wie Dispersions- oder Suspensionshilfsmittel, oberflächenaktive Agenzien, isotonische Wirkstoffe, Dickungsmittel oder Emulgatoren, Konservierungsmittel, Umhüllungsmittel (encapsulating agent), feste Bindestoffe oder Gleitmittel eingesetzt werden, je nachdem was für die jeweilige Dosierung am Besten geeignet ist und zugleich mit dem Peptid, Peptidomimetika (Pepidderivat), Peptid-Konjugat oder Peptidmimetika-Konjugat kompatibel ist.

Die pharmazeutische Zusammensetzung enthält daher vorzugsweise einen pharmazeutisch akzeptablen Träger. Der Begriff "pharmazeutisch akzeptabler Träger" umfasst dabei auch einen Träger zur Verabreichung der therapeutischen Zusammensetzung, wie beispielsweise Antikörper oder Polypeptide, Gene oder andere therapeutische Mittel. Der Begriff bezieht sich auf einen beliebigen pharmazeutischen Träger, der selbst nicht die Produktion von Antikörpern auslöst, die für das Individuum, dem die Rezeptur verabreicht wurde, gefährlich sein könnten, und der keine unangemessene Giftigkeit besitzen. Geeignete "pharmazeutisch akzeptable Träger" können große, langsam abbaubare Makromoleküle, wie beispielsweise Proteine, Polysaccharide, Polylactonsäuren, Polyglykolsäuren, polymere Aminosäuren, Aminosäure-Kopolymere und inaktivierte Virusbestandteile sein. Solche Träger sind dem Fachmann wohlbekannt.

Salze der Peptide oder funktionell äquivalenter Verbindungen können mit bekannten Methoden hergestellt werden, was typischer Weise bedeutet, dass die Peptide, Peptidomimetika, Peptid-Konjugate oder Peptidomimetika-Konjugate mit einer pharmazeutisch akzeptablen Säure zu einem sauren Salz oder mit einer pharmazeutisch akzeptablen Base zu einem basischen Salz gemischt werden. Ob eine Säure oder eine Base pharmazeutisch akzeptabel sind, kann leicht von einem Fachmann in Kenntnis der Anwendung und der Rezeptur festgelegt werden. So sind beispielsweise nicht alle Säuren und Basen, die für *ex vivo* Anwendungen akzeptabel sind, auch auf therapeutische Rezepturen übertragbar. Abhängig von der jeweiligen Anwendung können pharmazeutisch akzeptable Säuren sowohl organischer als auch anorganischer Natur sein, z.B. Ameisensäure, Essigsäure, Propionsäure, Milchsäure, Glykolsäure, Oxalsäure, Brenztraubensäure, Bernsteinsäure, Maleinsäure, Malonsäure, Zimtsäure, Schwefelsäure, Salzsäure, Bromwasserstoffsäure, Salpetersäure, Perchlorsäure, Phosphorsäure und Thiocyansäure, die mit den freien Aminogruppen von Peptiden und funktionell äquivalenten Verbindungen Ammoniumsalze ausbilden. Pharmazeutisch akzeptable Basen, die mit freien Carbonsäuregruppen der Peptide und funktionell äquivalenten Verbindungen Carboxylate ausbilden, beinhalten Ethylamin, Methylamin, Dimethylamin, Triethylamin, Isopropylamin, Diisopropylamin und andere Mono-, Di- und Trialkylamine sowie Arylamine. Ferner sind pharmazeutisch akzeptable Lösungsmittel eingeschlossen.

Pharmazeutisch akzeptable Salze können darin zur Anwendung kommen, wie z. B. Salze von Mineralsäuren, wie Hydrochloride, Hydrobromide, Phosphate, Sulfate und vergleichbare; aber auch Salze organischer Säuren, wie Acetate, Propionate, Malonate, Benzoate und vergleichbare. Eine ausführliche Diskussion zur pharmazeutisch akzeptablen Inhaltsstoffen findet man in Remington's Pharmaceutical Sciences (Mack Pub. Co., N.J., 1991).

Pharmazeutisch akzeptable Träger in den therapeutischen Zusammensetzungen können Flüssigkeiten enthalten, beispielsweise Wasser, Salzwasser, Glycerol und Ethanol. Zusätzlich können Hilfsstoffe zugegeben werden, wie Befeuchtungsmittel oder Emulgatoren, pH puffernde Substanzen und ähnliche Verbindungen können in solchen Mitteln vorhanden sein. Typischer Weise werden die therapeutischen Zusammensetzungen entweder in flüssiger Form oder als Suspension zur Injektion zubereitet, feste Formen zum Auflösen oder Suspendieren in Trägerflüssigkeiten vor der Injektion sind ebenfalls möglich. Liposomen sind auch in der Definition eines "pharmazeutisch akzeptablen Trägers" enthalten.

Zur therapeutischen Behandlung können Peptide, Peptidderivate, Peptid-Konjugate oder Peptidderivat-Konjugate, wie oben beschrieben, produziert und einem Subjekt, das diese benötigt, verabreicht werden. Das Peptid, Peptidderivat, Peptid-Konjugat oder Peptidderivat-Konjugat kann einem Subjekt/Patient in beliebiger, geeigneter Form verabreicht werden, vorzugsweise als pharmazeutischen Zusammensetzung, die der Darreichungsform angepasst ist und in einer für die angestrebte Behandlung angemessenen Dosierung vorliegt.

Die pharmazeutischen Zusammensetzungen dieser Erfindung können weitere aktive Verbindungen enthalten, beispielsweise konventionelle Antibiotika (z.B. Vancomycin, Streptomycin, Tetracyclin, Penicillin) oder andere antimikrobiell aktive Verbindungen, wie Fungizide, z.B. Intraconazol oder Myconazol. Auch andere Verbindungen, die mit der Infektion einhergehende Symptome wie Fieber (Salizylsäure) oder Hautausschlag lindern, können zugesetzt werden.

Neben der therapeutischen Verwendung zur Behandlung von Infektionen, oder auch bei der biologischen Kriegsführung, ist es weiter möglich, die erfindungsgemäßen Peptide oder Peptidderivate in Desinfektions- und/oder Reinigungsmitteln (z. B. einer bakterioziden Zusammensetzung) einzusetzen, die zur Desinfektion und/oder Reinigung von Oberflächen und/oder Gegenständen verwendet werden können. Ein anderes Anwendungsgebiet sind Verpackungen, wo Peptide an Verpackungsmaterial gebunden oder darin eingebunden werden können, oder als Konservierungsmittel für andere Materialien, die leicht durch Mikroorganismen abgebaut werden können. Die erfindungsgemäßen Peptide oder Peptidderivate sind insbesondere zur Verpackung von Lebensmitteln geeignet, da sie weder beim Kontakt noch bei der Aufnahme toxisch wirken.

Ein anderer Bestandteil dieser Erfindung ist eine Methode zur Behandlung von Säugetieren, die mit Mikroben (insbesondere Bakterien oder Pilze) infiziert sind, einschließlich der Verabreichung einer effektiven, therapeutisch wirksamen Menge der pharmazeutisch wirksamen erfindungsgemäßen Zusammensetzung.

Im Zusammenhang mit der vorliegenden Erfindung können bakterielle oder pilzliche Infektionen unter anderem ausgewählt sein aus der Gruppe bestehend aus Infektionen des Urogenitaltraktes, Infektionen des Blutes ("blood-stream-infections"), Sepsis, Atemwegsinfektionen, Bauchfellentzündungen, Wundinfektionen, Infektionen des Verdauungstraktes und Hirnhautentzündungen.

Der hier verwendete Begriff "therapeutisch wirksame Menge" bezeichnet die Menge eines Therapeutikums, d. h. eines erfindungsgemäßen Peptids, Peptidomimetikums, Peptid-Konjugats oder Peptidmimetika-Konjugats, welche die Vermehrung und Kolonienbildung der Bakterien zu reduzieren oder ganz zu verhindern oder einen messbaren therapeutischen bzw. prophylaktischen Erfolg zu erzielen vermag. Der Effekt kann beispielsweise für Biopsien in Kultur, durch Test der bakteriellen Aktivität oder mit einer anderen geeigneten Methode zur Beurteilung des Umfangs und des Grads einer bakteriellen Infektion bestimmt werden. Die genaue effektive Menge für ein Subjekt hängt von dessen Größe und Gesundheitszustand, der Art und dem Ausmaß der Erkrankung und den Therapeutika oder der Kombination mehrerer Therapeutika ab, die zur Behandlung ausgewählt wurden. Insbesondere die erfindungsgemäßen Zusammensetzungen können verwendet werden, um bakterielle Infektionen und/oder biologische oder physische Begleiterscheinungen (z.B. Fieber) zu reduzieren oder zu verhindern. Methoden zur Festlegung der Anfangsdosis durch einen Mediziner sind Stand der Technik. Die festgelegten Dosen müssen sicher und erfolgreich sein.

Die Menge eines erfindungsgemäßen Proteins, Peptids oder Nukleinsäure, die für eine antibakteriell effektive Dosis notwendig ist, kann unter Berücksichtigung des Pathogens, das die Infektion auslöst, der Schwere der Infektion, sowie vom Alter, Gewicht, Geschlecht, allgemeiner physischer Kondition usw. des Patienten festgelegt werden. Die notwendige Menge der aktiven Komponente, um effektiv antibakteriell und antimykotisch ohne nennenswerte Nebenwirkungen wirksam zu sein, hängt von der eingesetzten pharamazeutischen Rezeptur und der eventuellen Anwesenheit weiterer Bestandteile wie. Antibiotika, Antimykotika usw. ab. Für die erfindungsgemäßen Einsatzgebiete kann eine effektive Dosis zwischen 0,01 μg/kg und 50 mg/kg liegen, vorzugsweise zwischen 0,5 μg/kg und 10 mg/kg des Peptids, Peptidomimetikas, Pepid-Konjugats oder Peptidomimetik-Konjugats in dem behandelten Individuum.

Anfangsdosen der erfindungsgemäßen Peptide, Peptidomimetika, Multimere, Peptid-Konjugate oder Peptidomimetika-Konjugate können optional durch wiederholte Verabreichung verfolgt werden. Die Häufigkeit der Dosierungen hängt von den oben identifizierten Faktoren ab und beträgt vorzugsweise zwischen ein und sechs Dosen pro Tag über einen Behandlungszeitraum von etwa drei Tagen bis maximal einer Woche.

In einer weiteren, alternativen Zusammensetzung werden die erfindungsgemäßen Peptide, Peptidomimetika, Pepid-Konjugate oder Peptidomimetik-Konjugate oder Mischungen durch eine kontrollierte oder fortwährende Freisetzung aus einer Matrix, die in den Körper des Subjekts eingebracht wurde, verabreicht.

In einer Ausführungsform wird eine erfindungsgemäße Verbindung durch die Haut verabreicht. Diese Art der Gabe ist nicht invasiv und patientenfreundlich, gleichzeitig führt es wahrscheinlich zu einer gesteigerten Bioverfügbarkeit der Verbindung im Vergleich zu einer oralen Aufnahme, insbesondere falls die Verbindung nicht gegenüber dem Milieu im Verdauungssystem stabil ist oder falls es zu groß ist, um vom Darm effizient aufgenommen zu werden. Die Aufnahme durch die Haut ist beispielsweise in der Nase, der Wange, unter der Zunge, am Zahnfleisch oder in der Vagina möglich. Entsprechende Darreichungsformen können mit bekannten Techniken erreicht werden; sie können zu Nasentropfen, Nasenspray, Einlagen, Filmen, Pflaster, Gele, Salben oder Tabletten verarbeitet werden. Bevorzugt wird der Arzneiträger für die Aufnahme durch die Haut eine oder mehrere Komponenten enthalten, die an der Haut haften und dadurch die Kontaktzeit der Darreichungsform mit der adsorbierenden Oberfläche verlängern, um dadurch die Aufnahme durch Absorption zu steigern.

In einer weiteren Ausführungsform werden die Verbindungen pulmunal in einer bestimmten Menge verabreicht, z.B. durch einen Inhalator, Zerstäuber, Aerosolspray oder einen Inhalator für trockenes Pulver. Geeignete Formulierungen können durch bekannte Methoden und Techniken hergestellt werden. Eine transdermale oder rektale Zufuhr mag in einigen Fällen genau wie die Verabreichung in das Auge angebracht sein.

Es kann vorteilhaft sein, die erfindungsgemäßen Substanzen durch fortgeschrittene Formen der Arzneimittelgabe (advanced drug delivery or targeting methods) effektiver zu verabreichen. So kann, falls der Verdauungstrakt umgangen werden soll, die Darreichungsform eine beliebige Substanz oder Mischung enthalten, die die Bioverfügbarkeit steigert. Dies kann beispielsweise dadurch erreicht werden, dass der Abbau reduziert wird, z.B. durch einen Enzyminhibitor oder ein Antioxidants. Besser ist es, wenn die Bioverfügbarkeit der Verbindung durch eine Zunahme der Permeabilität der Absorptionsbarriere, meist die Schleimhaut, erzielt wird. Substanzen, die das Eindringen erleichtern, können auf verschiedene Arten wirken; einige erhöhen die Fluidität der Schleimhaut, während andere die Zwischenräume zwischen den Schleimhautzellen erweitern.

Wiederum andere reduzieren die Viskosität des Schleims auf der Schleimhaut. Zu den bevorzugten Aufnahmebeschleunigem gehören amphiphile Substanzen wie Cholinsäurederivate, Phospholipide, Ethanol, Fettsäuren, Ölsäure, Fettsäurederivate, EDTA, Carbomere, Polycarbophil und Chitosan.

Indikationen, für welche die erfindungsgemäßen Peptide, Peptidderivate, Konjugate oder Multimere eingesetzt werden können, sind bakterielle Infektionen mit sowohl Gram-positiven als auch Gram-negativen Bakterien, beispielsweise mit *Escherichia coli, Enterobacter cloacae, Erwinia amylovora, Klebsiella pneumoniae, Morganella morganii, Salmonella typhimurium, Salmonella typhi, Shigella dysenteriae, Yersinia enterocolitica, Acinetobacter calcoaceticus, Acinetobacter baumannii, Agrobacterium tumefaciens, Francisella tularensis, Legionella pneumophila, Pseudomonas syringae, Rhizobium meliloti, Haemophilus influenzae, Stenotrophomonas maltophilia, Pseudomonas aerugionsa, Proteus vulgaris* oder *Proteus mirabilis.*

Gegenstand der Erfindung ist auch die Verwendung eines erfindungsgemäßen Peptids, Peptidderivats oder Mutltimers in der biochemischen, biotechnologischen, medizinischen oder pharmazeutischen Forschung oder in Screeningverfahren, insbesondere zur Identifizierung von Substanzen, die eine potentielle antibakterielle oder antimykotische Wirkung aufweisen.

Ein weiterer Bestandteil der Erfindung ist daher auch ein Verfahren zur Identifizierung von Verbindungen mit anti-bakterieller oder anti-mykotischer Wirkung, welches folgendes beinhaltet:

(i) Durchführung eines kompetitiven Tests (Assays) mit:

(a) einem Mikroorganismus der gegenüber einem erfindungsgemäßen Peptid, Peptidderivat oder Multimer empfindlich ist,
(b) ein erfindungsgemäßes Peptid, Peptidderivat oder Multimer,
(c) mindestens eine Verbindung die getestet werden soll,

indem sie (a) mit (b) und (c) in Kontakt bringt; und

(ii) Auswählen einer Testverbindung, die kompetitiv das Peptid, Peptidderivat oder Multimer vom Mikroorganismus verdrängt.

Dieses Screeningverfahren identifiziert Testverbindungen, die mit dem erfindungsgemäßen Peptid oder dem multimeren Konstrukt kompetitiv um die Bindung an den unbekannten Rezeptor des Pathogens konkurrieren können. Dadurch können kleine Moleküle, die spezifisch an die gleiche Stelle wie das Peptid binden, effektiv in einem Hochdurchsatzscreening identifiziert werden. Dabei weisen die Testverbindungen voraussichtlich den gleichen Wirkmechanismus wie die Originalpeptidsequenz auf und sind daher auch gegen multiresistente Mikroben aktiv, die durch die erfindungsgemäßen Peptide oder Peptidderivate abgetötet werden.

Das Screeningverfahren wird mit bekannten Methoden durchgeführt, macht jedoch von mindestens einem erfindungsgemäßen Peptid, Peptidderivat oder Multimer Gebrauch. Bevorzugt wird das erfindungsgemäße Peptid, Peptidderivat oder Multimer dazu mit einem fluoreszierenden, radioaktiven oder anderweitig detektierbaren Marker versehen. Das Bindungsverhalten des markierten Peptids Peptidderivats oder Multimers an den Mikroorganismus wird in Anwesenheit und Abwesenheit der zu testenden Substanz(en) verglichen.

Vorzugsweise werden die Testverbindungen, die mit dem erfindungsgemäßen Peptid oder einem multimeren Konstrukt um die Bindung konkurrieren, danach identifiziert und auf ihre antibakterielle oder antimykotische Wirkung getestet.

In einer Ausführungsform wird die Fluoreszenz nach der Bildung eines Dimers (BIFC; bimolecular fluorescence complementation) in einem kompetitiven Assay gemessen. Die Methode ermöglicht die direkte Visualisierung intrazellulärer Proteinwechselwirkungen, was am Beispiel der SH3-Domäne aus der c-Abl Tyrosinkinase mit natürlichen und künstlichen Zielmolekülen in *E. coli* gezeigt wurde (Morell M, Espargaro A, Aviles F X, & Ventura S. Detection of transient protein-protein interactions by bimolecular fluorescence complementation: The Abl-SH3 case. Proteomics 7: 1023-36, 2007). Dieses Testsystem ist sensitiv genug, um sogar die Wechselwirkungen zwischen in *E. coli* niedrig exprimierten Proteine nachweisen zu können. Es basiert auf der Anlagerung von zwei Fragmenten des gelb fluoreszierenden Proteins (YFP), nachdem die SH3 Domäne an ihren Partner gebunden hat. Sobald diese beiden Proteine aneinander gebunden haben, bilden die beiden Fragmente von YFP einen Komplex, dessen Struktur dem nativen Protein sehr nahe kommt. Dies kann über die beobachtete Fluoreszenz des YFP-Kompexes beobachtet werden, da die einzelnen Fragmente nicht fluoreszieren. Ein ähnliches Konstrukt kann auch entworfen werden, um nach Verbindungen zu suchen, die mit den in dieser Erfindung beschriebenen Peptide und Peptidderivate um die Bindungsstelle konkurrieren. Ein Hochdurchsatzscreening kann einfach von einem Fachmann auf eine Mikrotiterplatte im 386er Format übertragen werden.

In einer anderen Ausführungsform werden die Peptide in einem geeigneten kompetitiven Assay verwendet, um die Fähigkeit der zu testenden Verbindungen, die Peptide kompetitiv von dem unbekannten Rezeptor der Pathogene zu verdrängen, zu ermitteln. Wo gewünscht, können (abhängig von dem gewählten Assay) Mikroorganismen (z.B.

Bakterium, Virus oder Pilz), die bekannter Maßen an das oder die ausgewählte(n) Peptid(e) binden, z.B. *E. coli* oder *K. pneumoniae* Stämme, direkt oder indirekt auf einer geeigneten Oberfläche immobilisiert werden, z.B. in einem ELISA-Format. Entsprechende Oberflächen zur Immobilsierung sind wohlbekannt. Beispielsweise kann ein inerter Partikel ("Bead") verwendet werden. Der Ligand kann aber auch an eine 96er Mikrotiterplatte gebunden sein. Danach werden ausgewählte Mengen der zu testenden Verbindungen und der erfindungsgemäßen Peptide mit den immobilisierten Mikroorganismen in Kontakt gebracht und die Verbindungen ausgewählt, die mit den Peptiden um die Bindung an die Mikroorganismen konkurrieren. Wenn diese Testverbindungen, die mit den Peptiden um die Rezeptorbindung an Bakterien oder Pilze konkurrieren, identifiziert sind, können sie mit weiter auf ihre antibakterielle oder antimykotische Wirkung untersucht werden. Dazu geeignete Methoden werden weiter unten in den Beispielen beschrieben.

In einem weiteren Gesichtspunkt stellt die Erfindung ein isoliertes Nukleinsäuremolekül bereit, dessen Sequenz für ein erfindungsgemäßes Peptid oder Multimer kodiert. Die für das erfindungsgemäße antibakterielle oder antimykotische Peptid oder multimere Konstrukt kodierende Nukleinsäure steht dabei in operativer Verbindung mit einer regulatorischen Sequenz, die dessen Expression in der Wirtszelle steuert. Ein weiterer Bestandteil der Erfindung ist eine Wirtszelle, die mit dem oben beschriebenen Nukleinsäuremolekül transfiziert oder transformiert ist.

Die Erfindung wird nachfolgend durch folgende Ausführungsbeispiele erläutert, ohne die Erfindung auf diese zu beschränken:

**BEISPIELE**

Beispiel 1: Peptidsynthese

Alle Chemikalien für die Peptidsynthese wurden, wenn nicht anders angegeben, von Fluka Chemie GmbH (Buchs, Schweiz) in der größtmöglichen Reinheit bezogen.

Alle Peptide und Peptidderivate wurden mittels konventioneller Festphasenpeptidsynthese unter Verwendung der Fmoc/$^t$Bu-Strategie (Fields G B & Noble R L. Solid-Phase Peptide-Synthesis Utilizing 9-Fluorenylmethoxycarbonyl Amino-Acids. International Journal of Peptide and Protein Research 35: 161-214, 1990) an einem multiplen Peptidsyntheseroboter Syro 2000 (MultiSynTech GmbH, Witten, Deutschland) synthetisiert. Alle Standard-Fmoc-Aminosäuren wurden von MultiSynTech GmbH (Witten, Deutschland) bzw. Orpegen Pharma GmbH (Heidelberg, Deutschland) bezogen. Als spezielle Arginin-Homologe wurden 2-Amino-3-guanidinopropionsäure (Agp; Iris Biotech GmbH, Marktredwitz, Deutschland), β-Homoarginin (βHar, Fluka Chemie GmbH, Buchs, Schweiz), Homoarginin (Har), N-Methylarginin (N-Me-Arg) und Nitroarginin (Arg(NO$_2$), Bachem AG, Bubendorf, Schweiz) verwendet. *Trans*-4-Hydroxyprolin (*t*-4-Hyp) und 2,3-Diaminopropionsäure (Dap) wurden von Novabiochem (Merck Biosciences GmbH, Darmstadt, Deutschland) bezogen.

Die Peptide wurden entweder als Säure an Wang-Harz (1,23 mmol/g) oder als Säureamid an Rink-Amid 4-Methylbenzylhydrylamin (MBHA) Harz (0,67 mmol/g), der Finna MultiSynTech GmbH (Witten, Deutschland) synthetisiert. Zur späteren Funktionalisierung mit primären Aminen wurden Peptide als Peptidthioester derivatisiert und dazu die erste Aminosäure an 4-Sulfamino-butyryl-aminomethyl Harz (SAB AM, 1,1 mmol/g; Novabiochem, Merck Biosciences GmbH, Darmstadt, Deutschland) gekoppelt.

An Wang Harz wurde die erste C-terminale Aminosäure (5 Äquivalente(eq)) mit 5 eq 2,6-Dichlorbenzoylchlorid (Merck KGaA, Darmstadt, Deutschland) und 8,25 eq Pyridin in Dichlormethan (DCM; Biosolve BV, Valkenswaard, Niederlande) gekoppelt (Sieber P. An Improved Method for Anchoring of 9-Fluorenylmethoxycarbonyl-Amino Acids to 4-Alkoxybenzyl Alcohol Resins. Tetrahedron Letters 28: 6147-50, 1987). Die Funktionalisierung des SAB AM Harzes mit der C-terminalen Aminosäure (5 eq) erfolgte bei -20°C durch Aktivierung mit 5 eq (Benzotriazol-1-yloxy)-tripyrrolidinophosphonium hexafluorophosphat (PyBOP, Novabiochem, Merck Biosciences GmbH, Darmstadt, Deutschland) und 10 eq N-Ethyldiisopropylamin (DIPEA) in DCM (Backes B J & Ellman J A. An Alkanesulfonamide Safety-Catch Linker for Solid-Phase Synthesis. The Journal of Organic Chemisty 64: 2322-30, 1999).

Die erste Aminosäure wurde an Rink-Amid Harz durch Aktivierung von jeweils 8 eq Aminosäure gelöst in 0,5 mol/L 1-Hydroxybenzotriazol (HOBt) mit 8 eq N,N'-Diisopropylcarbodiimid (DIC) in Dimethylformamid (DMF; Biosolve BV, Valkenswaard, Niederlande) gekoppelt, wie bei der automatischen Synthese an vorher beladenes Wang und SAB AM Harz beschrieben.

Als Seitenkettenschutzgruppen wurden Triphenylmethyl (trityl) für Cys, Asn, His und Gln, *tert.*-Butylether ($^t$Bu) für Tyr, Ser und Thr, *tert.*-Butylester (O$^t$Bu) für Asp und Glu, ω-N-2,2,4,6,7-Pentamethyldihydrobenzofuran-5-sulfonyl (Pbf) für Arg, ω-N-2,2,5,7,8-Pentamethylchroman-6-sulfonyl (Pmc) für βhar und Har, *tert.*-Butyloxy-carbonyl (Boc) für Lys, Orn, und Agp bzw. ω-N-4-Methoxy-2,3-6-trimethylphenyl-sulfonyl (Mtr) für N-Me-Arg eingesetzt. Die temporäre Fmoc-Schutzgruppe wurde mit 40% Piperidin (Biosolve BV, Valkenswaard, Niederlande) in DMF (v/v) für 5 min und erneut mit frischen 20% Piperidin in DMF (v/v) für 10 min abgespalten.

Die N-Termini der Peptide oder Peptidderivate wurden acetyliert, formyliert bzw. iodacetyliert indem 8 eq Essigsäure, Ameisensäure bzw. Iodessigsäure in 0,5 mol/L HOBT/DMF gelöst und mit 8 eq DIC in DMF aktiviert wurden.

Der N-Terminus der Peptide oder Peptidderivate wurde nach Gausepohl et al. (Gausepohl,H., Pieles,H., & Frank,R.W.in Peptides: chemistry, structure and biology (eds. Smith,J.A. & Rivier,J.E.) 523 (ESCOM, Leiden, 1992)) mit je 10 eq 2-($^1$H-Benzotriazol-1-yl)-1,1,3,3-tetramethylaminium hexafluorophosphat (HBTU, MultiSynTech GmbH, Witten, Deutschland) und DIPEA in DMF guanidiert. Die N-Termini wurden mit dem Fluoreszenzfarbstoff 5,6-Carboxyfluorescein (5 eq) mit 5 eq HBTU und 10 eq DIPEA in DMF modifiziert.

Die Vollständigkeit N-terminaler Modifikationen wurde mittels Kaisertest überprüft. Dazu wurde etwas Harz mit 0,28 mol/L Ninhydrin (Riedel de Haen, Seelze, Deutschland) in Ethanol (Carl Roth GmbH + Co. KG, Karlsruhe, Deutschland), 0,2 mmol/L Kaliumcyanid in Pyridin und 76% Phenol in Ethanol im Verhältnis (1:1:2) bei 95°C inkubiert. Falls eine Blaufärbung auftrat, was auf freie primäre Aminogruppen hindeutet, wurde die Kopplung nochmals wiederholt.

Nach Beendigung der Synthese der Peptide oder Peptidderivate wurden die Harze sorgfältig mit DMF und DCM gewaschen und unter Vakuum getrocknet. Die Harz-gebundenen Peptide wurden mit einer Mischung aus Wasser, m-Kresol, Thioanisol und Ethandithiol (5:5:5:2,5) in 87,5% Trifluoressigsäure (TFA) bei Raumtemperatur für 4 h abgespalten und zugleich die Seitenketten entschützt. Die Peptide und Peptidderivate wurden mit kaltem Diethylether präzipitiert und bei 3000*g g abzentrifugiert. Das Pellet wurde zweimal mit kaltem Ether gewaschen, getrocknet und in 0,1% wässriger TFA (UV-Spektroskopie) gelöst. Die Proben wurden bei -20°C gelagert.

Vor der Abspaltung der Peptide am SAB AM Harz, wurde der N-Terminus nach Abspaltung der Fmoc-Gruppe mit 20 eq Di-*tert*-butyldicarbonat (Boc$_2$O, Fluka Chemie GmbH, Buchs, Schweiz) und 10 eq DIPEA in DMF geschützt. Zum gewaschenen Harz wurden 100 eq Iodacetonitril und 20 eq DIPEA in DMF gegeben um den Sulfamyl-Linker durch Alkylierung zu aktivieren (Teruya K, Murphy A C, Burlin T, Appella E, & Mazur S J. Fmoc-based chemical synthesis and selective binding to supercoiled DNA of the p53 C-terminal segment and its phosphorylated and acetylated derivatives. Journal of Peptide Science 10: 479-93, 2004). Zur Abspaltung des Peptids wurde das Harz mit 50 eq Propylamin in DMF (10% v/v) versetzt. Nach dem Entfernen des DMF wurde das Rohpeptid in einer Lösung aus Wasser, m-Kresol, Thioanisol und Ethandithiol (5:5:5:2,5) in 87,5% TFA gelöst und alle. Seitenschutzgruppen und die N-terminale Boc-Schutzgruppe abgespalten. Die Peptide wurden mit kaltem Diethylether präzipitiert und bei 3000 x g abzentrifugiert. Der Niederschlag wurde zweimal mit kaltem Ether gewaschen, getrocknet und in 0,1% wässriger TFA (UV-Spektroskopie) gelöst. Die Proben wurden bei -20°C gelagert.

Die abgespaltenen Peptide und Peptidderivate wurden mittels RP-HPLC an einem Äkta HPLC System (Amersham Bioscience GmbH, Freiburg, Deutschland) mit einer Jupiter C$_{18}$ 5 μm 300 Å, 250 x 10 mm bzw. Jupiter C$_{18}$ 15 μm, 300 Å, 250 x 21 mm Säule (Phenomenex Inc., Torrance, USA) gereinigt.

Als Laufmittel wurde jeweils 0,1% wässrige TFA (Eluent A) und 60% wässriges Acetonitril (Biosolve BV, Valkenswaard, Niederlande) mit 0,1% TFA (Eluent B) verwendet. Ein typischer linearer Gradient begann bei 5% Eluent B und die Elution erfolgte bei einer Steigung von 1% B pro Minute mit einer Flussrate von 10 mL/min (250 x 21 mm Säule) bzw. 5 mL/min (250 x 10 mm Säule). Detektiert wurde bei 220, 230 und 240 nm. Die Analytik der gereinigten Peptide erfolgte mit dem gleichen HPLC System mit einer Jupiter C$_{18}$ 5 μm, 300 Å, 150 x 4,6 mm Säule (Phenomenex Inc., Torrance, USA). Eluiert wurde bei einer Flussrate von 1 mL/min mit einem linearen Gradienten von 5-95% B in 30 min und detektiert bei 220 nm. Zusätzlich wurde die Reinheit mittels Matrix-unterstützter Laser Desorption/Ionisierung mit Flugzeit-Massenspektrometrie (MALDI-TOF-MS; 4700 Proteomic Analyzer, Applied Biosystems GmbH, Darmstadt, Deutschland) bestimmt. Dafür wurde 0,5 μL Peptidlösung mit 0,5 μL α-Cyanohydroxyzimtsäure (Bruker Daltonik GmbH; Bremen, Deutschland) als Matrix (5,3 mg/mL in 50% Acetonitril in 0,1% wässriger TFA) co-kristallisiert.

Die Thioether-Verknüpfung der Penetratin-Derivate erfolgte durch Inkubation des gereinigten Penetratin-Cys-Monomers mit 4 eq gereinigtem iodacetyliertem AMP in entgaster phosphatgepufferter Salzlösung (PBS, pH 7,4) bei 4°C unter Stickstoff. Die Reaktion wurde mittels RP-HPLC verfolgt und das Penetratin-Konstrukt nach vollständiger Umsetzung des Penetratin-Cys-Monomers aufgereinigt. Hierbei wurde parallel das (Penetratin-Cys)$_2$-Dimer gewonnen.

Ausgehend von der unvollständig bestimmten Sequenz des Prolin-reichen antimikrobiellen Peptids "Oncopeltus antibakterielles Peptid 4" wurden zunächst erste Derivate von Peptid 4 (Tabelle 2) mit den C-terminalen Aminosäuren N$_{18}$N$_{19}$R$_{20}$ synthetisiert und die Carboxyfunktion nicht verändert. Unter den Modifikationen an Position 11 zeigten die kationischen Aminosäuren Lys und Arg überraschend gute Eigenschaften. Die Derivate zeigten antibakterielle Aktivität gegenüber *E. coli* und *M. lutetes* im unteren mikromolaren Bereich. Zur weiteren Derivatisierung des C-Terminus wurde die mit Arg11 derivatisierte Sequenz ausgewählt und Derivate mit unterschiedlicher Anordnung von Asn und Arg synthetisiert. Das am C-Terminus um Asn verkürzte Derivat (SEQ ID NO. 18) zeigte eine erstaunlich hohe Aktivität. Als C-terminales Säureamid erreichte es zudem eine überraschend hohe Serumstabilität. Das Peptid mit der Sequenz VDK-PPYLPRPRPPRRIYNR-NH$_2$ (SEQ ID NO. 18) wird im Folgenden als Oncocin bezeichnet und wurde vor allem bezüglich der Serumstabilität aber auch der antimikrobiellen Aktivität weiter verbessert.

**Tabelle 2:**

| SEQ ID NO. | Synthese-Nummer | Sequenz | | | *E. coli* BL21 AI | *M. luteus* 10240 |
|---|---|---|---|---|---|---|
| 123* | *O. fasciatus* | VDKPPYLPRP (X/P) PPRRIYN (NR) | | | | |
| 2* | A25 A4 | VDKPPYLPRP | <u>P</u> | PPRRIYN <u>NR-OH</u> | 128 | 64 |
| 4* | A25 A5 | VDKPPYLPRP | <u>T</u> | PPRRIYN <u>NR-OH</u> | 128 | 64 |
| 8 | A28 B2 | VDKPPYLPRP | <u>H</u> | PPRRIYN <u>NR-OH</u> | 64 | 32 |
| 5 | A29 B1 | VDKPPYLPRP | <u>K</u> | PPRRIYN <u>NR-OH</u> | 16 | 16 |
| 14 | A25 A6 | VDKPPYLPRP | <u>R</u> | PPRRIYN <u>NR-OH</u> | 8 | 16 |
| 16 | A29 A6 | VDKPPYLPRP | <u>R</u> | PPRRIYN <u>RN-OH</u> | 8 | 16 |
| 18 | A33 B3 | VDKPPYLPRP | <u>R</u> | PPRRIYN <u>R-NH$_2$</u> | 4 | 8 |
| *Vergleichsbeispiel. | | | | | | |

**Tabelle 3:** Übersicht der synthetisierten Peptidsequenzen

| SEQ ID NO. | Synthese-nummer | Sequenz |
|---|---|---|
| 1* | A21 B2 | VDKPPYLPRPPPPRRIYN-NH$_2$ |
| 2* | A25 A4 | VDKPPYLPRP**P**PPRRIYNNR-OH |
| 3* | A33 B1 | VDKPPYLPRP-**4tHyp**-PPRRIYNR-OH |
| 4* | A25 A5 | VDKPPYLPRP**T**PPRRIYNNR-OH |
| 5 | A29 B1 | VDKPPYLPRP**K**PPRRIYNNR-OH |
| 6 | A28 B1 | VDKPPYLPRP**K**PPRRIYN**RN**-OH |
| 7 | A35 A3 | VDKPPYLPRP**K**PPRRIYN**R**-NH$_2$ |
| 8 | A28 B2 | VDKPPYLPRP**H**PPRRIYNNR-OH |
| 9 | A28 B3 | VDKPPYLPRP**H**PPRRIYN**RN**-OH |
| 10* | A31 B2 | VDKPPYLPRP**Y**PPRRIYNR-OH |
| 11* | A31 B3 | VDKPPYLPRP**N**PPRRIYNR-OH |
| 12* | A31 B4 | VDKPPYLPRP**Q**PPRRIYNR-OH |
| 13* | A31 B5 | VDKPPYLPRP**F**PPRRTYNR-OH |
| 14 | A25 A6 | **VDKPPYLPRPRPPRRIYNNR-OH** |
| 15 | A29 A2 | VDKPPYLPRPRPPRRIYNNR-NH$_2$ |
| 16 | A29 A6 | VDKPPYLPRPRPPRRTYN**RN**-OH |
| 17 | A35 A2 | VDKPPYLPRPRPPRRIYN**RN**-NH$_2$ |
| 18 | A33 B3 | **VDKPPYLPRPRPPRRIYNR-NH$_2$** |
| 19 | A35 A4 | VDKPPYLPRPRPPRRIYN**R-OH** |
| 20 | A31 A4 | VDKPPYLPRPRPPR**P**IYNR-OH |
| 21 | A31 B1 | VDKPPYL-**4tHyp**-RPRPPRRIYNR-OH |
| 22 | A34 A2 | VDKPPYL-**4tHyp**-RPRPPRRIYNR-NH$_2$ |
| 23 | A33 A6 | VDK-4tHyp-PYLPRPRPPRRIYNR-OH |
| 24 | A34 A3 | VDKPPYLPRPRP-4tHyp-RRIYNR-NH$_2$ |

(fortgesetzt)

| SEQ ID NO. | Synthese-nummer | Sequenz |
|---|---|---|
| 25 | A53 E6 | VDKPPYLPRPRPPR-4tHyp-IYNR-NH$_2$ |
| 26 | A53 F3 | VDKPPYLPRPRPPRRIYNON-NH$_2$ |
| 27* | A35 A6 | VDKPPYLPRPRPPRRIYN-NH$_2$ |
| 28* | A51 A6 | VDKPPYLPRPRPPRRIYN-OH |
| 29 | A56 A1 | ADKPPYLPRPRPPRRIYNR-NH$_2$ |
| 30 | A56 A2 | VAKPPYLPRPRPPRRIYNR-NH$_2$ |
| 31* | A56 A3 | VDAPPYLPRPRPPRRIYNR-NH$_2$ |
| 32 | A56 A4 | VDKAPYLPRPRPPRRIYNR-NH$_2$ |
| 33 | A56 A5 | VDKPAYLPRPRPPRRTYNR-NH$_2$ |
| 34* | A56 A6 | VDKPPALPRPRPPRRIYNR-NH$_2$ |
| 35* | A56 B1 | VDKPPYAPRPRPPRRIYNR-NH$_2$ |
| 36 | A56 B2 | VDKPPYLARPRPPRRIYNR-NH$_2$ |
| 37* | A56 B3 | VDKPPYLPAPRPPRRIYNR-NH$_2$ |
| 38 | A56 B4 | VDKPPYLPRARPPRRIYNR-NH$_2$ |
| 39* | A56 B5 | VDKPPYLPRPAPRRIYNR-NH$_2$ |
| 40 | A56 B6 | VDKPPYLPRPRAPRRIYNR-NH$_2$ |
| 41 | A56 C1 | VDKPPYLPRPRPARRIYNR-NH$_2$ |
| 42* | A57 D5 | VDKPPYLPRPRPPARIYNR-NH$_2$ |
| 43* | A57 D6 | VDKPPYLPRPRPPRAIYNR-NH$_2$ |
| 44 | A56 C4 | VDKPPYLPRPRPPRRAYNR-NH$_2$ |
| 45 | A56 C5 | VDKPPYLPRPRPPRRIANR-NH$_2$ |
| 46 | A56 C6 | VDKPPYLPRPRPPRRIYAR-NH$_2$ |
| 47* | A56 D1 | VDKPPYLPRPRPPRRIYNA-NH$_2$ |
| 48* | A35 B1 | VDKPPYLPRPRPPRPIRV-OH |
| 49 | A70 A1 | VDKPPYLPRPRPPRRIY**PQPRPPHPRL**-NH$_2$ |
| 50 | A51 B1 | VDKPPYLPRPRPPRRIYN**O**-NH$_2$ |
| 51* | A51 B2 | VDKPPYLPRPRPPRRIYN**N**-NH$_2$ |
| 52 | A51 B3 | VDKPPYLPRPRPPRRIYM**Dap(Ac)**-NH$_2$ |
| 53 | A56 E1 | VDKPPYLPRPRPPRRIYN**H**-NH$_2$ |
| 54 | A59 A2 | VDKPPYLPRPRPPRRIYN**Agp**-NH$_2$ |
| 55 | A59 A3 | VDKPPYLPRPRPPRRIYN**Arg(NO$_2$)**-NH$_2$ |
| 56 | A59 A4 | VDKPPYLPRPRPPRRIYN**(N-Me-Arg)**-NH$_2$ |
| 57 | A59 A5 | VDKPPYLPRPRPPRRIYN**Har**-NH$_2$ |
| 58 | A66 D2 propyl. | VDKPPYLPRPRPPRRIYNR-NH**C$_3$H$_7$** |
| 59 | A45 A6 | VDKPPYLPRPRP**RP**RIYNR-NH$_2$ |
| 60* | A45 A5 | VOKPPYLPRPRPPR**P**IYNR-NH$_2$ |
| 61 | A45 A3 | VDKPPYLPRPRPPR**O**IYNR-NH$_2$ |

| SEQ ID NO. | Synthese-nummer | Sequenz |
|---|---|---|
| 62 | A51 B4 | VDKPPYLPRPRPPR-**betaHArg-**IYNR-NH$_2$ |
| 63 | A53 F1 | VDKPPYLPRPRPPR-**4tHyp-**IYN**O**-NH$_2$ |
| 64 | A66 C5 | VDKPPYLPRPRPPR**H**IYNH-NH$_2$ |
| 65 | A70 B4 | VDKPPYLPRPRPPR**Agp**IYN**Har**-NH$_2$ |
| 66 | A70 C1 | VDKPPYLPRPRPPR**Agp**IYN**Agp**-NH$_2$ |
| 67 | A70 B5 | VDKPPYLPRPRPPR**Har**IYN**Har**-NH$_2$ |
| 68 | A70 C2 | VDKPPYLPRPRPPR**Har**IYN**Agp**-NH$_2$ |
| 69 | A70 C3 | VDKPPYLPRPRPPR**O**IYIQ**Agp**-NH$_2$ |
| 70 | A70 B6 | VDKPPYLPRPRPPR**O**IYN**Har**-NH$_2$ |
| 71 | A66 D3 propyl. | VDKPPYLPRPRPPR**O**IYNR-NH**C$_3$H$_7$** |
| 72 | A53 F2 | VDKPPYLPRPRPPR**O**IYN**O**-NH$_2$ |
| 73 | A66 C6 | VDKPPYLPRPRPPR**OL**YNO-NH$_2$ |
| 74 | A66 D1 | VDKPPYLPRPRPPR**O**IY**QO**-NH$_2$ |
| 75 | A56 D2 | V**E**KPPYLPRPRPPRRIYNR-NH$_2$ |
| 76 | A56 D3 | VD**R**PPYLPRPRPPRRIYNR-NH$_2$ |
| 77 | A56 D4 | VDKPPY**I**PRPRPPRRIYNR-NH$_2$ |
| 78 | A56 D5 | VDKPPYLPRPRPPRR**L**YNR-NH$_2$ |
| 79 | A56 D6 | VDKPPYLPRPRPPRRIY**Q**R-NH$_2$ |
| 80 | A32 D3 ac. | **Ac**-VDKPPYLPRPRPPRRIYNR-OH |
| 81 | A32 D3 fo. | **For**-VDKPPYLPRPRPPRRIYNR-OH |
| 82 | A45 A2 | **O**DKPPYLPRPRPPRRIYNR-NH$_2$ |
| 83 | A45 A2 ac. | **Ac**-**O**DKPPYLPRPRPPRRIYNR-NH$_2$ |
| 84 | A45 A4 guan. | **Guan**-VDKPPYLPRPRPPRRIYNR-NH$_2$ |
| 85 | A62 B3 guan. | **Guan**-VDKPPYLPRPRPPR**O**IYN**O**-NH$_2$ |
| 86* | A35 A5 | DKPPYLPRPRPPRRIYNR-NH$_2$ |
| 87* | A54 E1 | GNNRPVYIPQPRPPHPRL-OH |
| 88* | A60 E4 CF | *CF*-GNNRPVYIPQPRPPHPRL-OH |
| 89* | A34 B3 | GKPRPYSPRPTSHPRPIRV-OH |
| 90* | A60 E2 CF | *CF*-GKPRPYSPRPTSHPRPIRV-OH |
| 91* | A35 A1 | VDKGSYLPRPTPPRPIYNRN-NH$_2$ |
| 92* | A60 E6 CF | *CF*-VDKGSYLPRPTPPRPIYNRN-NH$_2$ |
| 93 | A33 B3 | VDKPPYLPRPRPPRRIYNR-NH$_2$ |
| 94 | A60 F2 CF | *CF*-VDKPPYLPRPRPPRRIYNR-NH$_2$ |
| 95 | A60 E4 F5 | RQIKIWFQNRRMKWKKC-(CH$_2$CO-GNNRPVYIPQPRPPHPRL-OH)-OH |
| 96 | A60 E4 F6 CF | *CF*-RQIKIWFQNRRMKWKKC-(CH$_2$CO-GNNRPVYIPQPRPPHPRL-OH)-OH |
| 97 | A60 E2 F5 | RQIKINFQNRRMKNKKC-(CH$_2$CO-GKPRPYSPRPTSHPRPIRV-OH)-OH |
| 98 | A60 E2 F6 CF | *CF*-RQIKIWFQNRRMKWKKC-(CH$_2$CO-GKPRPYSPRPTSHPRPIRV-OH)-OH |

(fortgesetzt)

| SEQ ID NO. | Synthese-nummer | Sequenz |
|---|---|---|
| 99 | A60 E6 F5 | RQIKIWFQNRRMKWKKC-(CH$_2$CO-VDKGSYLPRPTPPRPIYNRN-NH$_2$)-OH |
| 100 | A60 E6 F6 CF | *CF*-RQIKIWFQNRRMKWKKC-(CH$_2$CO-VDKGSYLPRPTPPRPIYNRN-NH$_2$)-OH |
| 101 | A60 F2 F5 | RQTKTWFQNRRMKWKKC-(CH$_2$CO-VDKPPYLPRPRPPRRIYIQR-NH2)-OH |
| 102 | A60 F2 F6 CF | *CF*-RQIKIWFQNRRMKWKKC-(CH$_2$CO-VDKPPYLPRPRPPRRIYNR-NH2)-OH |
| 103* | A60 F5 | (RQIKIWFQNRRMKWKKC-OH)$_2$ |
| 104* | A60 F5 CF | (*CF*-RQIKIWFQNRRMKNKKC-OH)$_2$ |
| 105* | A60 G1 | RQIKIWFQNRRMKWKK-OH |
| 106* | | RQIKIWFQNRRMKWKKC(CH$_2$CO-ffSGDRSGYSSRGS-OH)-OH |
| 107 | A74 C6 | VDKPPYLPRPRP-4tHyp-ROIYNO-NH2 |
| 108 | A74 D1 | VDKPPYLPRPRP-4tHyp-R-4tHyp-IYNO-NH2 |
| 109 | A76 B3 | VDKPPYLPRPRPPR**O-Tle**-YN**O**-NH$_2$ |
| 110 | A76 B5 | VDKPPYLPRPRP-4tHyp-R-4tHyp-Tle-YNO-NH2 |
| 111 | T1 D9 | VDKPPYLPRPRPPRrlYNR-NH2 |
| 112 | T1 D11 | VDKPPYLPRPRPPRrlYNr-NH2 |
| 113* | A82 A4 | ONYIORPPRPRPLYPPKDV-NH2 |
| 114* | A82 A5 | ONYI-4tHyp-RPPRPRPLYPPKDV-NH2 |
| 115* | T1 C7 | vdkppylprprpprriynr-NH2 |
| 116* | T1 C9 | vdkppylprprpproiyno-NH2 |
| 117* | T1 C11 | rnyirrpprprplyppkdv-NH2 |
| 118* | T1 D1 | onyiorpprprplyppkdv-NH2 |

Es wurde der Einbuchstabencode für die Aminosäurereste verwendet, wobei O in der Aminosäurekette für Ornithin steht;

Propyl steht für ein Propylamid am C-Terminus (Sub$_2$ = OR$_3$ = NHC$_3$H$_7$); Ac = Acetyl-, for = Formyl-, guan = Guanidino-Gruppe und CF = 5,6-Carboxyfluorescein Beispiele für modifizierte N-Termini (modifizierte alpha-Aminogruppe der N-terminalen Aminosäure, Sub$_1$ = Acetyl-NH, Fonnyl-NH, Guanidino oder 5,6-Carboxyfluorescein),

βhar: β-Homoarginin, die beta-Aminosäure-Homologe zur Arginin,

Agp: 2-Amino-3-guanidinopropionsäure, Har: Homoarginin und Arg(NO$_2$): Nitroarginin sind Homologe des Arginin,

N-Me-Arg: N-Methyl-Ariginin - ein an der Peptidbindung methyliertes Arginin,

4tHyp: *trans*-4-Hydroxyprolin,

Tle: *tert*.-Butylglycin

Dap(Ac): 2,3-Diaminopropionsäure mit acetylierter Aminofunktion in der Seitenkette,

(CH$_2$CO): Acetyl-Linker an SHGruppe des Cystein,

f: α-Aminocapronsäure,

kleingeschriebene Buchstaben stehen für die entsprechenden D-Aminosäuren,

mit * sind Vergleichsbeispiele markiert.

**Beispiel 2: Stabilität**

Serumstabilität in 25% Mausserum

Die Serumstabilitätsstudien wurden als Doppelbestimmung nach Hoffmann et al. (Hoffmann R, Vasko M, & Otvos L. Serum stability of phosphopeptides. Analytica Chimica Acta 352: 319-25, 1997) in Mausserum und 25% wässrigem Mausserum (PAA Laboratories GmbH; Pasching, Österreich) durchgeführt. Dazu wurden die Peptide und Peptidomimetika in Wasser gelöst, Mausserum zugegeben und eine Peptidkonzentration von 75 μg/mL eingestellt. Unter ständigem Schütteln wurde die Mischung bei 37°C inkubiert. Nach 0, 30, 60, 120, 240 und 360 Minuten wurde jeweils ein Aliquot entnommen und mit 15%iger wässriger Trichloressigsäure (Carl Roth GmbH & Co. KG; Karlsruhe, Deutschland) gemischt. Nach weiteren 10 Minuten Inkubation auf Eis wurden die präzipitierten Serumproteine abzentrifugiert (5 min, 13400 rpm, MiniSpin, Eppendorf AG, Hamburg, Deutschland). Der Überstand wurde abgenommen, mit 1 mol/L wässriger Natriumhydroxidlösung (Fluka Chemie GmbH, Buchs, Schweiz) neutralisiert und bis zur Analyse bei -20°C gelagert.

Die Überstände wurden mittels RP-HPLC mit einem linearen Acetonitrilgradienten (Biosolve BV, Valkenswaard, Niederlande) in Gegenwart von 0,1% Trifluoressigsäure (TFA, UVgrade, Fluka Chemie GmbH, Buchs, Schweiz) als Ionenpaarreagenz analysiert. Die Fraktionen wurden mit α-Cyanohydroxyzimtsäure (Bruker Daltonik GmbH; Bremen, Deutschland) als Matrix (5,3 mg/mL in 50% Acetonitril in 0,1% wässriger TFA) co-kristallisiert und mit einem Tandem-Massenspektrometer (MALDI-TOF/TOF-MS, 4700 Proteomics Analyzer; Applied Biosystems GmbH, Weiterstadt, Deutschland) im Positiv-Ionenreflektor-Modus analysiert. Der Anteil der intakten Peptide und deren Abbauprodukte bzw. Metabolite konnten so zu den einzelnen Zeitpunkten identifiziert und quantifiziert werden. Als Kontrolle diente 25%iges wässriges Mausserum, das parallel für die gleichen Zeitintervalle analysiert wurde.

In Tabelle 4 sind die Halbwertszeiten von Oncocin (SEQ ID NO. 18) und ausgewählter Oncocin-Derivate in 25% Mausserum angegeben. Die aufgeführten Oncopeltus 4 Derivate mit SEQ ID NO. 14 und 19 haben mit weniger als 30 min Halbwertszeit die geringste Stabilität. Hier wird zuerst die das Arginin an Position 19 (Rest $X_4$) gespalten. Die Stabilität des Abbauproduktes VDKPPYLPRPRPPRRIYN-OH (SEQ ID NO. 28) erhöht sich danach auf 120 min, jedoch besitzt dieses Fragment nur noch sehr geringe antimikrobielle Aktivität (64 μg/mL *E. coli*). Mit der Amidierung des C-Terminus ($Sub_2$) im Oncocin erhöht sich neben der Aktivität gegen *E. coli* und *M. luteus* (4 bzw. 8 μg/mL) auch die Stabilität auf 60 min, einen erstaunlich hohen Wert für Peptidderivate. Durch die Amidierung mit Propylamid (SEQ ID NO. 58) verlängerte sich die Halbwertszeit des Oncocins sogar auf 120 min.

**Tabelle 4:** Serumstabilität: Halbwertszeiten von Oncocin und ausgewählten Oncocin-Derivaten in 25% Mausserum.

| SE Q ID NO. | Synthese Nr. | Sequenz | *E. coli* BL21AI | *M. luteus* 10240 | Halbwertszeit |
|---|---|---|---|---|---|
| 14 | A25 A6 | VDKPPYLPRPRPPRRIYNNR-OH | 8 | 16 | <30 min |
| 19 | A35 A4 | VDKPPYLPRPRPPRRIYNR-OH | 32 | 64 | <30 min |
| 17 | A35 A2 | VDKPPYLPRPRPPRRIYNRN-NH$_2$ | 16 | 128 | 45 min |
| 18 | A33 B3 | VDKPPYLPRPRPPRRIYNR-NH$_2$ | 4 | 8 | 60 min |
| 24 | A34 A3 | VDKPPYLPRPRP-4tHyp-RRIYNR-NH$_2$ | 8 | 8 | 60 min |
| 61 | A45 A3 | VDKPPYLPRPRPPROIYNR-NH$_2$ | 8 | 16 | 90 min |
| 28* | A51 A6 | VDKPPYLPRPRPPRRIYN-OH | 64 | 128 | 120 min |
| 50 | A51 B1 | VDKPPYLPRPRPPRRIYNO-NH$_2$ | 8 | 16 | 120 min |
| 51* | A51 B2 | VDKPPYLPRPRPPRRIYNN-NH$_2$ | 16 | 64 | 120 min |
| 58 | A66 D2 propyl. | VDKPPYLPRPRPPRRIYNR-NHC$_3$H$_7$ | 4 | 8 | >120 min |
| 60* | A45 A5 | VDKPPYLPRPRPPRPIYNR-NH$_2$ | 8 | 64 | 120 min |
| 62 | A51 B4 | VDKPPYLPRPRPPR-betaHArg-IYNR-NH$_2$ | 4 | 16 | 150 min |
| 63 | A53 F1 | VDKPPYLPRPRPPR-4tHyp-IYNO-NH$_2$ | 4 | 8 | >360 min |
| 71 | A66 D3 propyl. | VDKPPYLPRPRPPROIYNR-NHC$_3$H$_7$ | 4 | 8 | >360 min |
| 72 | A53 F2 | VDKPPYLPRPRPPROIYNO-NH$_2$ | 8 | 16 | >360 min |
| * Vergleichsbeispiele. | | | | | |

Die Substitution von Position 19 (Rest $X_4$) gegen die nicht proteinogene Aminosäure Omithin (SEQ ID NO. 61) erhöhte die Stabilität auf 120 min Halbwertszeit. Die Substitution Arg15 gegen Ornithin erhöhte die Stabilität diese Oncocin-Derivates (SEQ ID NO. 50) auf 90 min Halbwertszeit. Die Derivate mit Prolin (SEQ ID NO. 60) oder β-Homo-Arginin (SEQ ID NO. 62) an Position 15 (Rest $X_3$) waren sogar erst nach 120 bzw. 150 min zur Hälfte abgebaut. Die Substitution des Prolins an Position 13 in 4-trans-Hydroxyprolin (SEQ ID NO. 24) hatte keinen negativen Effekt auf die Stabilität des Oncocins.

Durch Kombination dieser Modifikationen an Position 15 und 19 (Reste $X_3$ und $X_4$) war es möglich sehr stabile Peptidderivate zu synthetisieren, für die Halbwertszeiten von über 360 min bestimmt wurden. Dabei war die Aktivität der bevorzugten Beispiele SEQ ID NO. 63 und 72 mit Orn19 und Hyp15 bzw. Orn15 mit MHK-Werten von 4 bzw. 8 mg/mL gegen *E. coli* vergleichbar mit Oncocin. Die Kombination Orn15 mit propylamidiertem C-terminus (SEQ ID NO. 71) stellt ein weiteres sehr bevorzugtes Beispiel der Erfindung dar.

Serumstabilität in 100% Mausserum

**Tabelle 5:** Serumstabilität: Halbwertszeiten von Oncocin und ausgewählten Oncocin-Derivaten in 100% Mausserum.

| SEQ ID NO. | Synthese Nr. | Sequenz | *E. coli* BL21AI | *M. luteus* 10240 | Halbwertszeit |
|---|---|---|---|---|---|
| 18 | A33 B3 | VDKPPYLPRPRPPRRIYNR-NH$_2$ | 4 | 8 | 30 min |
| 24 | A34 A3 | VDKPPYLPRPRP-**4tHyp**-RRIYNR-NH$_2$ | 8 | 8 | 35 min |
| 61 | A45 A3 | VDKPPYLPRPRPPR**O**IYNR-NH$_2$ | 8 | 16 | 65 min |
| 58 | A66 D2 propyl. | VDKPPYLPRPRPPRRIYNR-NH**C$_3$H$_7$** | 4 | 8 | 60 min |
| 67 | A70 B5 | VDKPPYLPRPRPPR-**Har**-IYN-**Har**-NH$_2$ | 4 | 4 | 55 min |
| 63 | A53 F1 | VDKPPYLPRPRPPR-**4tHyp**-IYNO-NH$_2$ | 4 | 8 | >480 min 60% |
| 71 | A66 D3 propyl. | VDKPPYLPRPRPPR**O**IYNR-NH**C$_3$H$_7$** | 4 | 8 | 105 min |
| 72 | A53 F2 | VDKPPYLPRPRPPR**O**IYN**O**-NH$_2$ | 8 | 16 | 175 min |
| 25 | A53 E6 | VDKPPYLPRPRPPR-**4tHyp**-IYNR-NH$_2$ | 16 | 8 | 240 min |
| 107 | A74 C6 | VOKPPYLPRPRP-**4tHyp**-ROIYNO-NH$_2$ | 4 | 4 | n.b. |
| 108 | A74 D1 | VDKPPYLPRPRP-**4tHyp**-R-**4tHyp**-IYNO-NH$_2$ | 2 | 32 | 150 min |
| 109 | A76 B3 | VDKPPYLPRPRPPR**O**-**Tle**-YNO-NH$_2$ | 4 | 4 | 130 min |
| 110 | A76 B5 | VDKPPYLPRPRP-**4tHyp**-R-**4tHyp**-Tle-YNO-NH$_2$ | 2 | 16 | >480 min 70% |

Durch den Austausch der Arginin-Reste an Position 15 und 19 mit Ornithin oder trans-4-Hydroxyprolin (SEQ ID NO. 63 und 72) konnte die Halbwertszeit in 25% wässrigem Mausserum auf über 6 Stunden erhöht werden (siehe oben). Nach Inkubation in 100% Mausserum konnten für diese Sequenzen beim Austausch $R_{15}O$ und $R_{19}O$ eine Halbwertszeit von 175 Minuten bestimmt werden. Durch den Austausch $R_{15}$Hyp und $R_{19}O$ (SEQ ID NO. 63) konnte nach 480 min noch 60% der eingesetzten Peptidmenge nachgewiesen werden. Von einem weiteren Derivat des Oncocins mit $P_{13}$Hyp, $R_{15}$Hyp, $R_{19}O$ und $I_{16}$Tle (SEQ ID NO. 110) waren nach 480 min sogar noch 60% bzw. 70% der ursprünglichen Peptidmenge enthalten. Die Aktivität beider Derivate war mit 4 bzw. 2 μg/mL gegenüber *E. coli* sehr gut.

Stabilität gegen bakterielle Proteasen

*Herstellung Bakterien-Lysat*

Für das Bakterien-Lysat wurden 500 mL Nährbouillon (Carl Roth GmbH + Co.KG, Karlsruhe) mit *E. coli* BL21AI inokuliert und über Nacht bei 37°C inkubiert. Zweimal 250 mL Bakteriensuspension wurden 25 min bei 5000 rpm und 4°C in einer Beckman Avanti™ J-20-XP Zentrifuge mit JLA-10.500 Rotor (Beckman Coulter, Fullerton, U.S.A.)zentrifugiert. Die Pellets wurden in je 30 mL PBS (pH 7,4) suspendiert und erneut in einer Beckman Allegra™ 2IR Zentrifuge (Beckman Coulter, Fullerton, U.S.A.) abzentrifugiert. Die Pellets wurden in je 10 mL PBS suspendiert und mit Ultraschall (Vibra-cell™ Mikrospitze, Fisher Bioblock Scientific, Illkirch, Frankreich) zweimal 5 min (750 W; Amplitude 40%; 2 s an/ 3 s aus) auf Eis aufgeschlossen. Die Bakterien-Lysate wurden vereinigt und 1 mL Aliquote bei 15400 rpm 20 min bei 4°C zentrifugiert (Beckman Allegra™). Die Überstände wurden abgenommen und bei -20°C gelagert. Der Proteingehalt des Bakterien-Lysats wurde mittels Proteinbestimmung nach Bradford bestimmt.

*Proteinbestimmung nach Bradford [M.M. Bradford; (1976): Analytische Biochemie, 72, 248-254]*

Als Stammlösung für die Standardreihe wurde eine 2 mg/mL BSA-Lösung in PBS hergestellt. Diese wurde mit PBS so verdünnt, dass 6 Standardlösungen von 10 bis 100 $\mu$g/mL BSA entstanden. Von der zu bestimmenden Probe wurde eine Verdünnungsreihe in PBS angefertigt. Jeweils 50 $\mu$L Probe- bzw. Standardlösung wurden in eine Polystyrol-Mikroplatte pipettiert. Für das Bradford-Reagenz wurden 0,01 % Coomassie Brilliant Blau G250 in 5 % Ethanol gelöst und dazu 8,5 % o-Phosphorsäure gegeben und mit bidestilliertem Wasser aufgefüllt. Das Gemisch wurde anschließend 1 h bei 60°C inkubiert und weitere 12 h bei RT stehen gelassen und danach abfiltriert. Je 200 $\mu$L des Bradford-Reagenz gegeben wurden pro Well zugegeben und 15 min dunkel inkubiert. Die Absorption wurde bei 595 nm gegen einen Blindwert (50 $\mu$L PBS + 200 $\mu$L Bradford-Reagenz) gemessen.

*Bestimmung der Stabilität in Bakterien-Lysat*

Eine Lösung von 0,15 $\mu$g/mL Peptid in Bakterien-Lysat mit 1,5 mg/mL oder 0,5 mg/mL Proteingehalt wurde bei 37°C inkubiert. Nach 0, 30, 60, 120, 240 bzw. 360 min wurden je 200 $\mu$L abgenommen und die Proteine mit 50 $\mu$L 15 % Trichloressigsäure gefällt. Danach wurde 10 min bei 4°C inkubiert und anschließend 5 min bei 13000 rpm in einer Minispin Tischzentrifuge zentrifugiert. Vom Überstand wurden 210 $\mu$L abgenommen und mit einer 1 mol/L Natriumhydroxidlösung neutralisiert. Die Analyse des intakten Peptids und der Abbauprodukte wurde mittels HPLC durchgeführt. Der Lösung wurden 60 $\mu$L 3% wässriges Acetonitril mit 0,1% Trifluoressigsäure (TFA) zugegebenund 250 $\mu$L des Gemisches injiziert. Nach der chromatographischen Trennung erfolgte die Identifizierung der Bestansteile mit MALDI-TOF-MS.

**Tabelle 6:** Stabilität gegen bakterielle Proteasen: Halbwertszeiten von Oncocin und ausgewählten Oncocin-Derivaten nach Inkubation in *E. coli*-Lysat mit 0,5 mg/mL Proteinkonzentration.

| SEQ ID NO. | Synthese Nr. | Sequenz | *E. coli* BL21 AI | *M. luteus* 10240 | Halbwertszeit |
|---|---|---|---|---|---|
| 18 | A33 B3 | VDKPPYVPRPRPPRRIYIQR-NH2 | 4 | 8 | 60 min |
| 25 | A53 E6 | VDKPPYLPRPRPPR-**4tHy**p-IYNR-NH2 | 16 | 8 | n.b. |
| 72 | A53 F2 | VDKPPYLPRPRPPROIYN**O**-NH2 | 8 | 16 | 125 min |
| 73 | A66 C6 | VDKPPYLPRPRPPR**OL**YN**O**-NH2 | 8 | 16 | 90 min |
| 107 | A74 C6 | VDKPPYLPRPRP-**4tHyp**-ROIYNO-NH2 | 4 | 4 | 215 min |
| 108 | A74 D1 | VDKPPYLPRPRP-**4tHyp**-R-**4tHyp**-IYNO-NH2 | 2 | 32 | >240 min 60% |
| 109 | A76 B3 | VDKPPYLPRPRPPR**O-Tle**-YNO-NH2 | 4 | 4 | >240 min 90 % |
| 110 | A76 B5 | VDKPPYLPRPRP-**4tHyp**-R-**4tHyp**-**Tle**-YNO-NH2 | 2 | 16 | >240 min 90% |

Um die Stabilität gegen bakterielle Proteasen zu untersuchen, wurde aus einer Übernachtkultur von *E. coli* BL21 AI ein Lysat hergestellt und die Peptide darin inkubiert. Die Proteinkonzentration wurde mittels Bradford-Proteinbestimmung auf 0,5 bzw. 1,5 mg/mL eingestellt.

Vom nativen Oncocin (SEQ ID NO. 18) wurde in Lysat mit einer Gesamtproteinkonzentration von 0,5 mg/mL eine Halbwertszeit von 60 Minuten bestimmt. Durch den Austausch R150 und R190 (SEQ ID NO. 72) verdoppelte sich die Halbwertszeit auf 115 min. In einem nächsten Schritt wurden das Prolin an Position 13 einer Spaltstelle der Prolin-Endopeptidase gegen *trans*-4-Hydroxyprolin ausgetauscht (SEQ ID NO. 107), wodurch die Stabilität weiter auf 215 min erhöht wurde. Durch die Kombination $P_{13}$Hyp, $R_{15}$Hyp und $R_{19}$O konnte eine Halbwertszeit von mehr als 240 min erreicht werden.

Das Isoleucin an Position 16 wurde in einem weiteren Derivat mit $R_{15}$O und $R_{19}$O gegen *tert.*-Butylglycin ausgetauscht (SEQ ID NO. 109) und erhöhte die Stabilität auf mehr als 240 min. Die Derivate mit der Kombination $P_{13}$Hyp, $R_{15}$Hyp, $R_{19}$O und $I_{16}$Tle (SEQ ID NO. 110) ereichten ebenfalls Halbwertszeiten von mehr als 240 min in Lysat mit 0,5 mg/mL Proteinkonzentration. Die Oncocin-Derivate hatte überraschend positive Effekte auf die antibakterielle Aktivität gegenüber *E. coli* und verminderte den MHK-Wert für SEQ ID NO. 108 und 110 auf 2 μg/mL.

**Beispiel 3: Antibakterielle Tests**

Hemmzonentest (Agar-Diffussions-Test)

Die gereinigten Peptide und Peptidderivate wurden in Wasser auf eine Endkonzentration von 500 μg/mL verdünnt. Die Testkeime wurden aus einer Kultur in logarithmischer Wachstumsphase in einer Konzentration von ca. 3x10⁵ Zellen/mL in 1% Tryptic Soy Broth und 1,2% Agarose (Fluka Chemie GmbH, Buchs, Schweiz) ausplattiert. In Abständen von 3 cm wurden je 10 μL wässrige Peptidlösung (500 μg/mL) bzw. 10 μL Wasser und Antibiotikalösung als Kontrollen aufgetropft. Nach 20 h Inkubationszeit bei 37 °C wurden die Hemmzonendurchmesser (HZD) bestimmt. Alle Tests wurden unter aeroben Bedingungen durchgeführt.

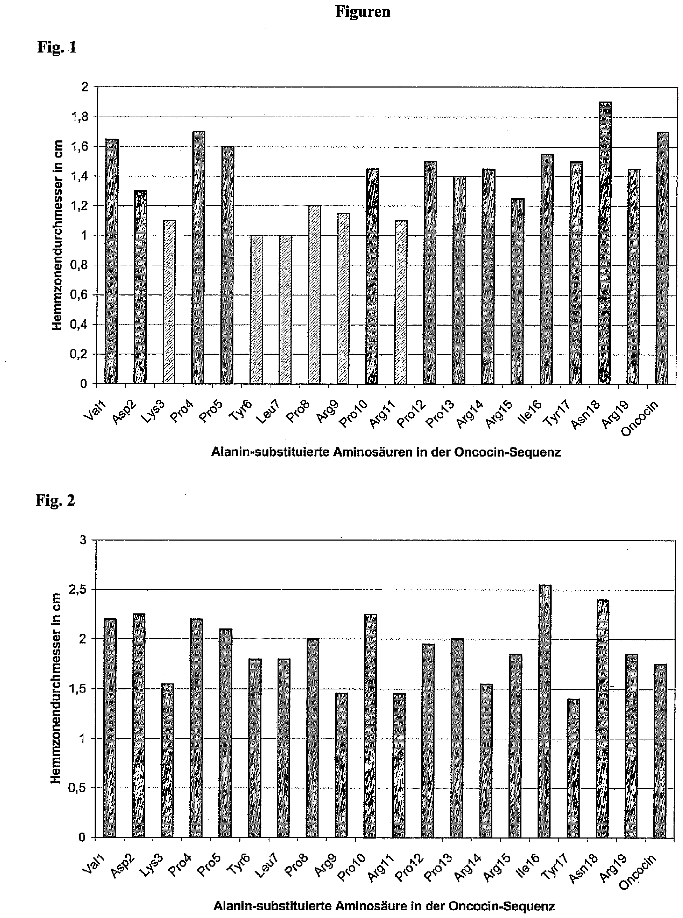

Mit dem Alanin-Scan der Oncocin-Sequenz konnten verschiedene Reste identifiziert werden, deren Austausch gegen Alanin zu einer deutlichen Abnahme der antimikrobiellen Aktivität gegen *E. coli* BL 21AI (Fig. 1) und *M. luteus* 10240 (Fig. 2) führte.

In Fig. 1 und Fig. 2 ist die Sequenz des Oncocins VDKPPYLPRPRPPRRIYNR (SEQ ID NO. 18) auf der X-Achse aufgetragen. Jede Aminosäure repräsentiert das entsprechende Peptid mit dem an dieser Postion ausgetauschten Alanin. So steht zum Beispiel der Balken Val1 für das Peptid ADKPPYLPRPRPPRRIYNR-NH₂ (SEQ ID NO. 29), dem der Wert des HZD auf der Y-Achse zugeordnet ist. Je größer der Durchmesser der Hemmzone, umso höher ist die Aktivität des Peptids.

In den Agar-Diffusions-Tests gegen *E. coli* BL21AI (Fig. 1) zeigte sich, dass sowohl der Alanin-Austausch an den Positionen Lys3, Tyr6-Arg9 und Arg11 die Aktivität im Vergleich zum Oncocin (HZD 1,7 cm) deutlich reduzierte. Diese Derivate (SEQ ID NO. 31, 34 bis 37 und 39) inhibieren das Wachstum von *E. coli* auf der Agar-Platte nur partiell mit teilweise bewachsenen Hemmzonen mit 1,0 bis 1,2 cm Durchmesser. Die kleinste, nicht bewachsene Hemmzone hatte das Peptid Ala15 (1,3 cm; SEQ ID NO. 43). Alle anderen Postionen können verändert werden ohne die Aktivität zu reduzieren, wobei aber eine Aktivitätssteigerung, Erweiterung des Spektrums, Stabilisierung und eine bessere *in vivo* Verteilung erreicht werden können. Positive Effekte wurden hier vor allem am N-terminalen Vall und am C-terminalen Arg19 erreicht, wenn proteasestabile Aminosäuren eingebaut und so die Serumstabilität erhöht wird ohne die antimikrobielle Aktivität zu verlieren.

Die Agar-Diffusions-Tests gegen das Gram-positive Bakterium *M. luteus* (Fig. 2) zeigten, dass durch den Alanin-Austausch sowohl positive als auch negative Effekte auf die antimikrobielle Aktivität erzielt werden. Für die Aktivität wichtige Positionen sind über die gesamte Sequenz verteilt, die Peptide inhibieren das Wachstum hier komplett mit minimal kleineren Hemmzonen gegenüber dem Oncocin (1,8 cm). Vor allem der Austausch von Lys bzw. Arg und damit der Verlust einer positiven Ladung im Peptid reduziert die antimikrobielle Aktivität. Die mit 0,7 cm größte Steigerung der Aktivität konnte mit dem Austausch von Ile16 und Asn18 erreicht werden (SEQ ID NO. 44,46). Änderungen an diesen Positionen finden sich in Beispielen mit jeweils Orn15 und Orn19 sowie einer Substitution in Position 16 gegen Leu (SEQ ID NO. 73) bzw. Position 18 gegen Gln (SEQ ID NO. 74) wieder. SEQ ID NO. 74 erreicht durch diese drei Substitutionen eine Aktivität die der des Oncocins entspricht, wobei das Gln18 den geringen negativen Effekt der Ornithin-Substitution kompensieren kann.

Wachstumsinhibitionsassay

Die minimalen Hemmkonzentrationen (MHK) der antimikrobiellen Peptide und Peptidderivate wurden in Mikrodilutionstests bestimmt. Dabei wurde eine fortlaufende Peptidverdünnung in sterilen 96-well Mikrotiterplatten mit flachem Boden (Polystyren, Greiner Bio-One GmbH, Frickenhausen, Deutschland) und einem Gesamtvolumen von 100 μL pro Vertiefung eingesetzt. Die wässrigen Peptid- oder Peptidomimetika-Lösungen wurden mit 1% TSB Wasser verdünnt, um eine Endkonzentration von 256 μg/mL zu erhalten. 50 μL der Peptid- oder Peptidomimetika-Lösung wurden in die erste Vertiefung jeder Reihe pipettiert und gemischt. Von dieser Lösung wurden 50 μL in die zweite Vertiefung transferiert, gemischt und erneut 50 μL in die nächste Vertiefung transferiert, usw. Dabei wird eine zweifache Verdünnungsserie erhalten, beginnend bei 256 μg/mL in der ersten Vertiefung bis zu 125 ng/mL in der zwölften Vertiefung. Die Bakterien, z. B. *E. coli* BL21 AI, wurden in Nährboullion (NB, Carl Roth GmbH + Co. KG, Karlsruhe, Deutschland) bei 37°C über Nacht kultiviert. In jede Vertiefung der Mikrotiterplatte wurden 50 μL einer 5 x 10⁶ Bakterien/mL Suspension in 1% TSB gegeben und so eine Endkonzentration der Peptide oder Peptidomimetika von 128 μg/mL (Vertiefung 1) bis zu 62,5 ng/mL (Vertiefung 12) in jeder Reihe eingestellt. Die Platten wurden für 20 h bei 37°C inkubiert und danach die Absorption bei 595 nm mit einem TECAN Mikrotiterplatten Spektrofotometer (Tecan Trading AG, Mannedorf, Schweiz) gemessen. Die MHK-Werte aller Peptide und Peptidomimetika wurden dreifach bestimmt. Steriles Wasser wurde als Negativkontrolle eingesetzt. Der MHK-Wert stellt die kleinste Peptidkonzentration dar, bei der nach einer Inkubationzeit von 20 h bei 37°C kein Bakterienwachstum beobachtet werden kann.

Die MHK-Werte der Peptide und Peptidderivate gegen *Escherichia coli* BL21 AI und *Micrococcus luteus* ATCC 10240 sind in Tabelle 7 angegeben.

**Tabelle 7:** Antimikrobielle Aktivität gegen *E. coli* BL21AI und *M. luteus* 10240. Minimale Hemmkonzentration (MHK) bestimmt in 1%TSB.

| SEQ ID NO. | Synthese-nummer | Sequenz | *E. coli* BL21 AI | *M. luteus* ATCC 10240 |
|---|---|---|---|---|
| 1* | A21 B2 | VDKPPYLPRPPPPPRRIYN-NH$_2$ | 128 | n.b. |
| 2* | A25 A4 | VDKPPYLPRP**P**PPRRIYNNR-OH | 128 | 64 |
| 3* | A33 B1 | VDKPPYLPRP-**4tHyp**-PPRRIYNR-OH | 128 | 32 |
| 4* | A25 A5 | VDKPPYLPRP**T**PPRRIYNNR-OH | 128 | 64 |
| 5 | A29 B1 | VDKPPYLPRP**K**PPRRIYNNR-OH | 16 | 16 |
| 6 | A28 B1 | VDKPPYLPRP**K**PPRRIYN**RN**-OH | 32 | 16 |
| 7 | A35 A3 | VDKPPYLPRP**K**PPRRIYN**R**-NH$_2$ | 8 | 16 |
| 8 | A28 B2 | VDKPPYLPRP**H**PPRRIYNNR-OH | 64 | 32 |
| 9 | A28 B3 | VDKPPYLPRP**H**PPRRIYN**RN**-OH | 64 | 16 |
| 10* | A31 B2 | VDKPPYLPRP**Y**PPRRIYNR-OH | 128 | 16 |
| 11* | A31 B3 | VDKPPYLPRP**N**PPRRIYNR-OH | 64 | 64 |
| 12* | A31 B4 | VDKPPYLPRP**Q**PPRRIYNR-OH | 64 | 64 |
| 13* | A31 B5 | VDKPPYLPRP**F**PPRRIYNR-OH | 64 | 32 |
| 14 | A25 A6 | **VDKPPYLPRPRPPRRIYNNR-OH** | 8 | 16 |
| 15 | A29 A2 | VDKPPYLPRPRPPRRIYNNR-NH$_2$ | 8 | 8 |
| 16 | A29 A6 | VDKPPYLPRPRPPRRIYN**RN**-OH | 8 | 16 |
| 17 | A35 A2 | VDKPPYLPRPRPPRRIYN**RN**-NH$_2$ | 16 | 128 |
| 18 | A28 A4/A33 B3 | **VDKPPYLPRPRPPRRIYNR-NH$_2$** | 4 | 8 |
| 19 | A35 A4 | VDKPPYLPRPRPPRRIYN**R-OH** | 32 | 64 |
| 20 | A31 A4 | VDKPPYLPRPRPPR**P**IYNR-OH | 8 | 16 |
| 21 | A31 B1 | VDKPPYL-**4tHyp**-RPRPPRRIYNR-OH | 16 | 16 |
| 22 | A34 A2 | VDKPPYL-**4tHyp**-RPRPPRRIYNR-NH$_2$ | 8 | 4 |
| 23 | A33 A6 | VDK-**4tHyp**-PYLPRPRPPRRIYNR-OH | 8 | 16 |
| 24 | A34 A3 | VDKPPYLPRPRP-**4tHyp**-RRIYNR-NH$_2$ | 8 | 8 |
| 25 | A53 E6 | VDKPPYLPRPRPPR-**4tHyp**-IYNR-NH$_2$ | 16 | 8 |

| SEQ ID NO. | Synthese-nummer | Sequenz | E. coli BL21 AI | M. luteus ATCC 10240 |
|---|---|---|---|---|
| 26 | A53 F3 | VDKPPYLPRPRPPRRIYN**ON**-NH$_2$ | 8 | 8 |
| 27* | A35 A6 | VDKPPYLPRPRPPRRIYN-NH$_2$ | 32 | 64 |
| 28* | A51 A6 | VDKPPYLPRPRPPRRIYN-OH | 64 | 128 |
| 29 | A56 A1 | ADKPPYLPRPRPPRRIYNR-NH$_2$ | 8 | 4 |
| 30 | A56 A2 | VAKPPYLPRPRPPRRIYNR-NH$_2$ | 16 | 4 |
| 31* | A56 A3 | VDAPPYLPRPRPPRRIYNR-NH$_2$ | 64 | 32 |
| 32 | A56 A4 | VDKAPYLPRPRPPRRIYNR-NH$_2$ | 8 | 8 |
| 33 | A56 A5 | VDKPAYLPRPRPPRRIYNR-NH$_2$ | 8 | 8 |
| 34* | A56 A6 | VDKPPALPRPRPPRRIYNR-NH$_2$ | 128 | 16 |
| 35* | A56 B1 | VDKPPYAPRPRPPRRIYNR-NH$_2$ | 128 | 16 |
| 36 | A56 B2 | VDKPPYLARPRPPRRIYNR-NH$_2$ | 32 | 8 |
| 37* | A56 B3 | VDKPPYLPAPRPPRRIYNR-NH$_2$ | 32 | 32 |
| 38 | A56 B4 | VDKPPYLPRARPPRRIYNR-NH$_2$ | 32 | 8 |
| 39* | A56 B5 | VDKPPYLPRPAPPRRIYNR-NH$_2$ | 64 | 16 |
| 40 | A56 B6 | VDKPPYLPRPRAPRRIYNR-NH$_2$ | 16 | 8 |
| 41 | A56 C1 | VDKPPYLPRPRPARRIYNR-NH$_2$ | 16 | 8 |
| 42* | A57 D5 | VDKPPYLPRPRPPARIYNR-NH$_2$ | 16 | 32 |
| 43* | A57 D6 | VDKPPYLPRPRPPRAIYNR-NH$_2$ | 32 | 16 |
| 44 | A56 C4 | VDKPPYLPRPRPPRRAYNR-NH$_2$ | 16 | 16 |
| 45 | A56 C5 | VDKPPYLPRPRPPRRIANR-NH$_2$ | 8 | 16 |
| 46 | A56 C6 | VDKPPYLPRPRPPRRIYAR-NH$_2$ | 8 | 8 |
| 47* | A56 D1 | VDKPPYLPRPRPPRRIYNA-NH$_2$ | 32 | 16 |
| 48* | A35 B1 | VDKPPYLPRPRPPR**PIRV**-OH | 128 | 8 |
| 49 | A70 A1 | VDKPPYLPRPRPPRRIY**PQPRPPHPRL**-NH$_2$ | 4 | 4 |
| 50 | A51 B1 | VDKPPYLPRPRPPRRIYN**O**-NH$_2$ | 8 | 16 |

| SEQ ID NO. | Synthese-nummer | Sequenz | E. coli BL21 AI | M. luteus ATCC 10240 |
|---|---|---|---|---|
| 51* | A51 B2 | VDKPPYLPRPRPPRRIYN**N**-NH$_2$ | 16 | 64 |
| 52* | A51 B3 | VDiCPPYLPRPRPPRRIYN**Dap(Ac)**-NH$_2$ | 32 | 64 |
| 53 | A56 E1 | VDKPPYLPRPRPPRRIYN**H**-NH$_2$ | 16 | 16 |
| 54 | A59 A2 | VDKPPYLPRPRPPRRIYN**Agp**-NH$_2$ | 8 | 8 |
| 55 | A59 A3 | VOKPPYLPRPRPPRRIYN**Arg(NO$_2$)**-NH$_2$ | 8 | 16 |
| 56 | A59 A4 | VDKPPYLPRPRPPRRIYN(**N-Me-Arg**)-NH$_2$ | 8 | 4 |
| 57 | A59 A5 | VDKPPYLPRPRPPRRIYN**Har**-NH$_2$ | 8 | 8 |
| 58 | A66 D2 propyl. | VDKPPYLPRPRPPRRIYNR-NH**C$_3$H$_7$** | 4 | 8 |
| 59 | A45 A6 | VDKPPYLPRPRPR**PR**IYNR-NH$_2$ | 8 | 16 |
| 60* | A45 A5 | VDKPPYLPRPRPPR**P**IYNR-NH$_2$ | 8 | 64 |
| 61 | A45 A3 | VDKPPYLPRPRPPR**O**IYNR-NH$_2$ | 8 | 16 |
| 62 | A51 B4 | VDKPPYLPRPRPPR**betaHArg**IYNR-NH$_2$ | 4 | 16 |
| 63 | A53 F1 | VDKPPYLPRPRPPR-**4tHyp**-IYN**O**-NH$_2$ | 4 | 8 |
| 64 | A66 C5 | VDKPPYLPRPRPPR**H**IYN**H**-NH$_2$ | 8 | 32 |
| 65 | A70 B4 | VDKPPYLPRPRPPR**Agp**IYN**Har**-NH$_2$ | 4 | 2 |
| 66 | A70 C1 | VDKPPYLPRPRPPR**Agp**IYN**Agp**-NH$_2$ | 8 | 4 |
| 67 | A70 B5 | VDKPPYLPRPRPPR**Har**IYN**Har**-NH$_2$ | 4 | 4 |
| 68 | A70 C2 | VDKPPYLPRPRPPR**Har**IYN**Agp**-NH$_2$ | 4 | 4 |
| 69 | A70 C3 | VDKPPYLPRPRPPR**O**IYN**Agp**-NH$_2$ | 4 | 4 |
| 70 | A70 B6 | VDKPPYLPRPRPPR**O**IYN**Har**-NH$_2$ | 4 | 4 |
| 71 | A66 D3 propyl. | VDKPPYLPRPRPPR**O**IYNR-NH**C$_3$H$_7$** | 4 | 8 |
| 72 | A53 F2 | VDKPPYLPRPRPPR**O**IYN**O**-NH$_2$ | 8 | 8 |
| 73 | A66 C6 | VDKPPYLPRPRPPR**O**LYN**O**-NH$_2$ | 8 | 16 |
| 74 | A66 D1 | VDKPPYLPRPRPPR**O**IY**QO**-NH$_2$ | 4 | 8 |
| 75 | A56 D2 | V**E**KPPYLPRPRPPRRIYNR-NH$_2$ | 16 | 8 |

EP 2 391 636 B1

| SEQ ID NO. | Synthese-nummer | Sequenz | E. coli BL21 AI | M. luteus ATCC 10240 |
|---|---|---|---|---|
| 76 | A56 D3 | VD**R**PPYLPRPRPPRRIYNR-NH$_2$ | 16 | 8 |
| 77 | A56 D4 | VDKPPY**I**PRPRPPRRIYNR-NH$_2$ | 32 | 8 |
| 78 | A56 D5 | VDKPPYLPRPRPPRR**L**YNR-NH$_2$ | 8 | 16 |
| 79 | A56 D6 | VDKPPYLPRPRPPRRIY**Q**R-NH$_2$ | 8 | 8 |
| 80 | A32 D3 ac. | **Ac**-VDKPPYLPRPRPPRRIYNR-OH | >256 | 16 |
| 81 | A32 D3 fo. | **For**-VDKPPYLPRPRPPRRIYNR-OH | 128 | 32 |
| 82 | A45 A2 | **O**DKPPYLPRPRPPRRIYNR-NH$_2$ | 8 | 8 |
| 83 | A45 A2 ac. | **Ac**-ODKPPYLPRPRPPRRIYNR-NH$_2$ | 16 | 16 |
| 84 | A45 A4 guan. | **Guan**-VOKPPYLPRPRPPRRIYNR-NH$_2$ | 32 | n.b. |
| 85 | A62 B3 guan. | **Guan**-VDKPPYLPRPRPPROIYNO-NH$_2$ | 64 | n.b. |
| 86* | A35 A5 | DKPPYLPRPRPPRRIYNR-NH$_2$ | 32 | 8 |
| 87* | A54 E1 | GNNRPVYIPQPRPPHPRL-OH | 2 | 64 |
| 89* | A34 B3 | GKPRPYSPRPTSHPRPIRV-OH | 4 | 0,5 |
| 91* | A35 A1 | VDKGSYLPRPTPPRPIYNRN-NH$_2$ | 16 | 128 |
| 95 | A60 E4 F5 | RQIKIWFQNRRMKWKKC (CH$_2$CO-GNNRPVYIPQPRPPHPRL-OH) -OH | 8 | 8 |
| 97 | A60 E2 F5 | RQIKIWFQNRRMKWKKC (CH$_2$CO-GKPRPYSPRPTSHPRPIRV-OH)-OH | 16 | 1 |
| 99 | A60 E6 F5 | RQIKIWFQNRRMKWKKC (CH$_2$CO-VDKGSYLPRPTPPRPIYNRN-NH$_2$)-OH | 32 | 4 |
| 101 | A60 F2 F5 | RQIKIWFQNRRMKWKKC(CH$_2$CO-VDKPPYLPRPRPPRRIYNR)-NH$_2$)-OH | 32 | 4 |
| 103* | A60 F5 | (RQIKIWFQNRRMKWKKC-OH)$_2$ | >128 | >128 |
| 107 | A74 C6 | VDKPPYLPRPRP-4tHyp-ROIYNO-NH2 | 4 | 4 |
| 108 | A74 D1 | VDKPPYLPRPRP-4tHyp-R-4tHyp-IYNO-NH2 | 2 | 32 |
| 109 | A76 B3 | VDKPPYLPRPRPPR**O-Tle**-YNO-NH2 | 4 | 4 |
| 110 | A76 B5 | VDKPPYLPRPRP-**4tHyp**-R-**4tHyp-Tle**-YNO-NH2 | 2 | 16 |
| 111 | T1 D9 | VDKPPYLPRPRPPRrIYNR-NH2 | 4 | 4 |
| 112 | T1 D11 | VDKPPYLPRPRPPRrIYNr-NH2 | 4 | n. b |

EP 2 391 636 B1

(fortgesetzt)

| SEQ ID NO. | Synthese-nummer | Sequenz | E. coli BL21 AI | M. luteus ATCC 10240 |
|---|---|---|---|---|
| 113 | A82 A4 | ONYIORPPRPRPLYPPKDV-NH2 | >256 | 64 |
| 114 | A82 A5 | ONYI-4tHyp-RPPRPRPLYPPKDV-NH2 | >256 | 128 |
| 115 | T1 C7 | vdkppylprprpprriynr-NH2 | 64 | 16 |
| 116 | T1 C9 | vdkppylprprproiyno-NH2 | 64 | 32 |
| 117 | T1 C11 | rnyirrpprprplyppkdv-NH2 | 64 | 32 |
| 118 | T1 D1 | onyiorpprprplyppkdv-NH2 | 256 | 32 |

Propyl steht für ein Propylamid am C-Terminus (Sub$_2$ = OR$_3$ = NHC$_3$H$_7$); Ac = Acetyl-, for = Formyl-, guan = Guanidino-Gruppe und CF = 5,6-Carboxyfluorescein sind Beispiele für modifizierte N-Termini (modifizierte alpha-Aminogruppe der N-terminalen Aminosäure, Sub$_1$ = Acetyl-NH, Formyl-NH, Guanidino oder 5,6-Carboxyfluorescein), βHar: β-Hamoarginin, die beta-Aminosäure-Homologe zur Arginin,

Agp: 2-Amino-3-guarudinopropionsäure, Har: Homoarginin und Arg(NO$_2$): Nitroarginin sind Homologe des Arginin,
N-Me-Arg: N-Methyl-Ariginin - ein an der Peptidbindung methyliertes Arginin,
4typ: *trans*-4-Hydroprolin,
Tle: *tert*.-Butylglycin
Dap(Ac): 2,3-Diaminopropionsäure mit acetylierter Aminofunktion in der Seitenkette, (CH$_2$CO): Acetyl-Linker an SH-Gruppe des Cystein,
f: α-Aminocapransäure,
kleingeschriebene Buchstaben stehen für die entsprechenden D-Aminosäuren,

Die SEQ ID NO. 2 entspricht der nativen Oncopeltus 4 Sequenz. SEQ ID NO. I ist ein N-terminal verkürztes Derivat der nativen Oncopeltus 4 Sequenz. Die Sequenzen mit den SEQ ID NO. 1, 2, 3, 4, 10 bis 13, 17, 28, 31, 34, 35, 39 und 48 sind Vergleichsbeispiele, diese und weitere sind in der Tabelle 7 mit * markiert. Die Sequenzen mit den SEQ ID NO. 8 bis 9, 80, 81 und 85 sind weniger bevorzugte Beispiele der Erfindung. Am meisten bevorzugte Beispiele sind die SEQ ID NO. 62, 63, 65 bis 71, 74, und 112.

Durch den Austausch des Pro11 (Rest X$_2$) in der Ausgangssequenz (SEQ ID NO. 2) gegen die kationischen Aminosäuren Lys und Arg (SEQ ID NO. 8 und 14) konnte überraschend eine höhere Aktivität gegen *E. coli* BL 21AI und *M. luteus* 10240 erreicht werden. Ein Austausch gegen His (SEQ ID NO. 5) und ein in Prolin-reichen AMP häufig an dieser Position vorkommendes Thr (SEQ ID NO. 4) hatte keinen positiven Effekt. Das Derivat mit Arg an Position 11 hatte mit 8 μg/mL (*E. coli*), die bis dahin niedrigste MHK und wurde daraufhin C-terminal verändert. Die Amidierung des C-Terminus veränderte die Aktivität nur gegen *M. luteus* negativ um eine Verdünnungstufe. Peptidderivate ohne Arg und damit fehlender positiver Ladung an letzter oder vorletzter Position (SEQ ID NO. 27, 28, 47, 51) verloren 8 bis 16fach an Aktivität, vor allem gegenüber *M. luteus* und gehören nicht zu den bevorzugten Beispielen.

SEQ ID NO. 16 mit Arg an Position 19 (Rest X$_4$) und Asn an 20 war in gleichem Maß aktiv wie SEQ ID NO. 14. Die Amidierung des C-Terminus (Sub$_2$) hat unerwarteterweise keinen negativen Einfluss auf die Aktivität (SEQ ID NO. 15). Die Amidierung des C-terminus (Sub$_2$) führt sogar zu einer gesteigerten Aktivität (vgl. SEQ ID NO. 18 mit SEQ ID NO. 19). Die C-terminale Amidierung hat zusätzlich einen signifikant positiven Effekt auf die Stabilität. Amidierte Peptidderivate haben so eine bis zu 30 min längere Halbwertszeit als das entsprechende Peptid mit freier Säurefunktion (Beispiel 2). Das Peptidderivat SEQ ID NO. 18 hatte mit 4 bzw. 8 μg/mL gegen *E. coli* bzw. *M. luteus* die niedrigsten MHK-Werte, bei einer Halbwertszeit von 60 min in 25% wässrigem Mausserum und wurde als Oncocin bezeichnet (Tabelle 4).

Im Serumstabilitätstest wurde Oncocin C-terminal an Position 15 (Rest X$_3$) und 19 (Rest X$_4$) gespalten und die Peptide VDKPPYLPRPRPPR-OH (entspricht SEQ ID NO. 126) und VDPPYLPRPRPPRRIYN-OH (entspricht SEQ ID NO. 28) als Hauptabbauprodukte identifiziert. Das um Arg19 verkürzte Derivat hatte mit einer MHK von 64 μg/mL nur noch sehr geringe antimikrobielle Aktivität gegenüber *E. coli.* Derivate mit Arginin oder andere kationische Aminosäuren an Position 19 (Rest X$_4$) zeigten erstaunlicherweise eine erhöhte Stabilität. Während ein Derivat mit His an Position 19 (Rest X$_4$; SEQ ID NO. 53)mit einer MHK von 16 μg/mL gegen *E. coli* eine 4mal geringere Aktivität hatte als Oncocin. Bevorzugte Beispiele sind Substitutionen mit Agp, Arg(NO$_2$) *N*-Me-Arg und Har (SEQ ID NO. 54 bis 57) an Position 19 (Rest X$_4$) substituierte Peptidderivate deren MHK-Werte den Werten des Oncocins entsprechen und somit unerwartet keinen negativen Einfluss auf die Aktivität der Peptide haben. Das bevorzugte kostengünstigste Beispiel war die Substitution des Arg19 gegen Ornithin in SEQ ID NO. 50. Dieses Derivat istdeutlich stabiler als Oncocin (60 min) bei annähernd gleicher Aktivität. Ein weiteres bevorzugtes Beispiel für die Stabilisierung des C-Terminus war die Amidierung der Carboxylfunktion (Sub$_2$) als Propylamid (SEQ ID NO. 58). Die antimikrobielle Aktivität bleibt erhalten und die Halbwertszeit beträgt ebenfalls mehr als 120 min.

In den an Position 19 (Rest X$_4$) stabilisierten Derivaten wurde in weiteren Beispielen die Position 15 (Rest X$_3$) durch Argininderivate oder andere kationische Aminosäuren substituiert. Bevorzugte Beispiele sind SEQ ID NO. 65 bis 70, mit unterschiedlichen Kombinationen von Agp, Arg(NO$_2$), *N*-Me-Arg, Har und Orn. Das kostengünstigste bevorzugte Beispiel ist SEQ ID NO. 72) mit Ornithin an Position 15 und 19 (Rest X$_3$ und X$_4$) und einer Halbwertszeit von mehr als 360 min, bei gegenüber Oncocin vergleichbarer Aktivität (MHK 8 μg/mL *E. coli*). SEQ ID NO. 74 wurde zusätzlich zu Orn15 und Orn16 mit eine Substitution von Glutamin an Position 18 gegen Asparagin vorgenommen, was in einer geringfügig höheren Aktivität (MHK 4μg/mL *E. coli*) resultierte. Die Kombination von Orn an Position 15 (Rest X$_3$) und der Amidierung des C-Terms (Sub$_2$) mit Propylamin in SEQ ID NO. 71 ist ein weiteres sehr bevorzugtes Beispiel mit erstaunlich hoher Aktivität (4 μg/mL *E. coli*) und sehr hoher Serumstabilität (>360 min). Eine nicht-kationische Substitution an Position 15 (Rest X$_3$) mit Hydroxyprolin wurde in der bevorzugten SEQ ID NO. 63 in Kombination mit Orn an Position 19 (Rest X$_4$) durchgeführt. Dieses Derivat besitzt ebenfalls eine hohe Aktivität (4 μg/mL *E. coli*, 8 μg/mL *M. luteus*) und hervorragende Stabilität (>360 min). Die Substitution des Prolins in Position 4, 8 oder 13 (SEQ ID NO. 21 bis 24) des Oncocin durch Hydroxyprolin hat weder einen Einfluss auf die MHK-Werte, noch reduziert es die Proteaseresistenz der zweiten labilen Spaltstelle an Position 15 (Rest $X_3$). Während dieser Austausch durch Hydroxyprolin weder einen Effekt auf die MHK-Werte noch einen Effekt auf die Serumstabilität hat, reduziert dieser Austausch unerwartet die Zelltoxizität und die Hämolyse.

Ein bevorzugtes Beispiel für Modifikation des N-Terminus im Oncocin ist SEQ ID NO. 82 mit Substitution von Position 1 (Rest $X_1$) in Orn, mit gleicher Aktivität gegenüber Oncocin. Acetylierung, Methanoylierung (Formylierung) oder Guanidierung der N-terminalen Aminofunktion ($Sub_1$; SEQ ID NO. 80, 81, 83, 84, 85) reduzierten die Aktivität auf z.B. 128 μg/mL bzw. 32. (*E. coli* bzw *M. luteus*; SEQ ID NO. 81).

Die Kopplung des Penetratins erfolgte über eine Thioetherbrücke an die Aminofunktion der N-Termini der antimikrobiellen Peptide. Diese Modifikation konnte das Aktivitätsspektrum der bis dahin gegen *M. luteus* wenig aktiven Peptide, auf dieses Bakterium erweitern. Am meisten wurde der MHK-Wert für Pyrrhocoricin (SEQ ID NO. 91) von 128 μg/mL auf 4 μg/mL für Penetratin-Pyrrhocoricin (SEQ ID NO. 98) reduziert, was einer 32fachen Steigerung der Aktivität entspricht. Auch das Penetratin-Apidaecin (8 μg/mL, SEQ ID NO. 94) war 8fach aktiver als das unmodifizierte Apidaecin 1b (64 μg/mL SEQ ID NO. 87). Für Oncocin (SEQ ID NO. 18) mit einem MHK-Wert von 8 μg/mL konnte noch eine 2fache Steigerung der Aktivität auf 4 μg/mL für Penetratin-Oncocin (SEQ ID NO. 100) beobachtet werden. Die hohe Aktivität des Drosocins (0,5 μg/mL, SEQ ID NO. 89) blieb beim Pentetratin-Drosocin-Konstrukt (1 μg/mL, SEQ ID NO. 96) erhalten.

Der Austausch von Prolin an Position 13 und 15 gegen *trans*-4-Hydroxyprolin und Arg19 gegen Ornithin (SEQ ID NO. 108) hat mit 2 μg/mL erstaunlicherweise eine Steigerung der Aktivität gegenüber *E. coli* im Vergleich zur nativen Oncocin Sequenz (4 μg/mL, SEQ ID NO. 18) zur Folge während die Stabilität gleichbleibend hoch ist. Wird das Isoleucin an Position 16 gegen *tert*.-Butylglycin ersetzt (SEQ ID NO. 110) bleibt der MHK-Wert von 2 μg/mL gegen *E. coli* erhalten. Interessant ist hier vor allem die erhöhte Stabilität.

Die Aktivität des Peptids mit SEQ ID NO.107 zeigt die gleiche antibiotische Wirkung gegen *M. luteus* wie Oncocin (SEQ ID NO. 18). Die Sequenzen SEQ ID NO. 113 bis 118 sind Vergleichsbeispiele. Die mit D-Aminosäuren synthetisierten Derivate (SEQ ID NO. 115 und 116) zeigen keine Aktivität gegenüber *E. coli* und nur geringe Aktivität gegenüber *M. luteus* (64 bzw. 128 μg/mL).

Sowohl die all D-Peptide mit D-Aminosäuren in nativer Reihenfolge (SEQ ID NO. 115 und 116) als auch die retro-invers synthetisierten Peptide (SEQ 1D NO. 117 und 118), zeigen nur geringe Aktivität gegenüber *E. coli* (64-256 μg/mL). Alle mit D-Aminosäuren synthetisierten Peptide (SEQ ID NO. 115 bis 118) zeigen jedoch mit MHK-Werten zwischen 16-32 μg/mL noch relativ gute Aktivität gegenüber *M. luteus*, die sich im Bereich der L-Peptide mit fünf positiven Nettoladungen befindet. Die von der Nettoladung abhängige Aktivität und die MHK-Werte der D-Peptide gegenüber *M. luteus* könnte auf einen Zielprotein-unspezifischen Wirkmechanismus in diesem Gram-positiven Bakterium hindeuten.

**Tabelle 8** : Antimikrobielle Aktivität gegen pathogene *Escherichia coli* DSM 10233, *Klebsiella pneumoniae* DSM 681 und *Pseudomonas aeruginosa* DSM 3227. Minimale Hemmkonzentration (MHK) bestimmt in 1%TSB.

| SEQ ID NO. | Sequenz | *E. coli* DSM 10233 | *K. pneumoniae* DSM 681 | *P. aeruginosa* DSM 3227 |
|---|---|---|---|---|
| 18 | VDKPPYLPRPRPPRRIYNR-NH$_2$ | 16 | 4 | >32 |
| 72 | VDKPPYLPRPRPPROIYNO-NH$_2$ | 32 | 4 | >32 |
| 63 | VDKPPYLPRPRPPR-4tHyp-IYNO-NH$_2$ | 1 | 2 | 16-32 |
| 107 | VDKPPYLPRPRP-4tHyp-ROIYNO-NH$_2$ | 16 | 4 | >32 |
| 108 | VDKPPYLPRPRP-4tHyp-R-4tHyp-IYNO-NH$_2$ | 1 | 2 | 16-32 |
| 111 | VDKPPYLPRPRPPRrIYNR-NH$_2$ | 4 | 4 | n.b. |
| 117 | rnyirrpprprplyppkdv-NH$_2$ | >32 | >32 | n.b. |

Die in Tabelle 8 angegebenen Peptide wurden auf ihre MHK gegen pathogene Bakterien wie *E. coli* DSM 10233, *K. pneumoniae* DSM 681 und *P. aeruginosa* DSM 3227 untersucht. Alle Derivate mit 4-trans-Hydroxyprolin an Position 15 (Seq. ID Nr. 63, 108, 113 und 114) haben mit MHK-Werten zwischen 1-4 $\mu$g/mL überraschend eine bis zu 16fach höhere Aktivität gegen *E. coli* DSM 10233 als Oncocin (SEQ ID NO. 18).

In Tabelle 10 sind die MHK-Werte einiger Peptide und Peptidderivate gegen multiresistente Bakterienstämme angegeben. Die Tests wurden dabei in dem zu 1% TSB äquivalenten Mueller-Hinton-Medium (1/4 konzentriert) durchgeführt. Einige der getesteten resistenten Bakterienstämme sind in Tabelle 9 und die getesteten Peptide in Tabelle 11 angeführt.

**Tabelle 9:** Getestete Multi-resistente Gram-negative Bakterien

| | |
|---|---|
| *E. coli* D31 (J. Wilson) | $\beta$-Lactamase-Überproduzent (ESBL+) - resistent gegen $\beta$-Lactamase-Inhibitoren |
| *E. coli* ATCC BAA-457 | Trimethoprim-Sulfomethoxazol-resistent |
| *E. coli* 045-849 SENTRY | Ciprofloxacin- und Trimethoprim-Sulfomethoxazol-resistent |
| *K. pneumoniae* ATCC 27799 | Gentamicin-, Cephalothin- und Naladixinsäure-resistent |
| *K. pneumonie* ATCC 700603 | produziert $\beta$-Lactamase SHV-18 (ESBL+) - resistent gegen $\beta$-Lactamase-Inhibitoren |
| *K. pneumoniae* 012-3132 | Fluoroquinolon-resistent |
| *S. typhimurium* ATCC 700408 | multi-resistent (z. B. Ampicilin, Chloramphenicol) |
| *S. typhimurium* S5 (J. Weiser) | multi-resistent (z. B. Cefotaxim, Tobramycin) |

**Tabelle 10:** Antimikrobielle Aktivität gegen multiresistente Bakterien. MHK in ¼ Muller-Hinton Medium.

| SEQ ID NO. | E. coli D31 J.Wilson | E. coli SEQ 102ATCC # BAA-457 | E. coli 045-849 SENTRY | K. pneumoniae ATCC 27799 | K. pneumoniae K6 ATCC 700603 | K. pneumoniae 012-3131 | S. typhimurium ATCC 700408 | S. typhimurium S5 (J.Weiser) | S. typhimurium ATCC 14028 | P. mirabilis ATCC 7002 | P. vulgaris ATCC 6896 | P. aeruginosa 39324 | P. aeruginosa 10 J.Wilson | S. saprophyticus 15305 |
|---|---|---|---|---|---|---|---|---|---|---|---|---|---|---|
| 87* | 1 | 1 | 0,5 | 4 | 16 | 16 | 0,13 | 0,5 | 0,5 | > | > | 64 | 32 | > |
| 89* | 4 | 2 | 2 | 8 | 8 | 4 | 0,5 | 1 | 8 | > | > | 32 | 16 | >64 |
| 18 | 2 | 4 | 4 | 16 | 8 | 4 | 0,25 | 4 | 2 | 128 | 128 | 4 | 8 | 16 |
| 24 | 1 | 4 | 2 | 8 | 8 | 2 | 0,13 | 2 | 2 | 128 | 128 | 4 | 2 | 16 |
| 15 | 16 | 32 | 4 | 64 | 32 | 16 | 0,5 | 8 | 4 | > | > | n.b | 16 | n.b |
| 22 | 2 | 4 | 4 | 16 | 8 | 4 | 0,5 | 8 | 2 | 128 | 128 | n.b | 16 | n.b |
| 14 | 32 | 64 | 4 | 128 | 64 | 32 | n.b | n.b | n.b | n.b | n.b | n.b | n.b | n.b |
| 20 | 4 | 32 | 8 | 64 | 128 | 64 | n.b | n.b | n.b | n.b | n.b | n.b | n.b | n.b |
| 23 | 8 | 32 | 8 | 64 | 32 | 16 | n.b | n.b | n.b | n.b | n.b | n.b | n.b | n.b |
| 21 | 32 | 64 | 16 | 128 | 64 | 32 | n.b | n.b | n.b | n.b | n.b | n.b | n.b | n.b |
| 16 | 32 | 32 | 4 | 128 | 64 | 32 | n.b | n.b | n.b | n.b | n.b | n.b | n.b | n.b |
| 5 | 64 | 128 | 16 | 128 | 128 | 64 | n.b | n.b | n.b | n.b | n.b | n.b | n.b | n.b |

n.b: nicht bestimmt

* Apidaecin 1b (SEQ ID NO. 87) und Drosocin (SEQ ID NO. 89) zum Vergleich.

**Tabelle 11: Getestete Sequenzen**

| SEQ ID NO. | Sequenz |
|---|---|
| 87 | GNNRPVYIPQPRPPHPRL-OH |

(fortgesetzt)

| SEQ ID NO. | Sequenz |
|---|---|
| 89 | GKPRPYSPRPTSHPRPIRV-OH |
| 18 | VDKPPYLPRPRPPRRIYNR-NH$_2$ |
| 24 | VDKPPYLPRPRP-4**tHyp-RRIYNR-NH$_2$** |
| 15 | VDKPPYLPRPRPPRRIYNNR-NH$_2$ |
| 22 | VDKPPYL-4**tHyp-RPRPPRRIYNR-NH$_2$** |
| 14 | VDKPPYLPRPRPPRRIYNNR-OH |
| 20 | VDKPPYLPRPRPPRPIYNR-OH |
| 23 | VDK-4**tHyp-PYLPRPRPPRRIYNR-OH** |
| 21 | VDKPPYL-4**tHyp-RPRPPRRIYNR-OH** |
| 16 | VDKPPYLPRPRPPRRIYNRN-OH |
| 5 | VDKPPYLPRPKPPRRIYNNR-OH |

Die bevorzugten Beispiele SEQ ID NO. 18, 22 und 24 zeigten überraschend eine mindestens gleich hohe oder höhere Aktivität gegenüber den drei verschiedenen Gram-negativen multi-resistente Bakterienspezies *E. coli, Klebsiella pnewnonia* und *Salmonella typhimurium.* Das Oncocin (SEQ ID NO. 18) hatte MHK-Werte von 2 ug/mL gegen β-Lactamase-überproduzierende *E. coli* D31, 4 μg/mL gegen Fluoroquinolon-resistente *K. pneumoniae* 012-3132 und 0,25 μg/mL gegen multi-resistente *S. typhimurium* ATCC 700408 Bakterien. Bis auf eine Ausnahme (SEQ ID NO. 15) zeigten alle getesteten amidierten Peptiden gegen die *E. coli* und *K. pneumoniae* Stämme gute MHK-Werte. Die Deletion des Asn 19 aus SEQ ID NO. 2, die zu Oncocin (SEQ ID NO. 18) führt, steigert erstaunlicherwiese die Atkivität der Peptide weiter. Die Amidierung des C-Terminus steigert nicht nur die Atkivität gegen *E. coli* und *K. pneumoniae,* sondern erhöht zusätzlich die Stabilität der Oncopeltus 4 bzw. Oncocin Derivate.

Mit *Pseudomonas aeruginosa, Proteus mirabilis* und *Proteus vulgaris* wurden weitere Gram-negative Bakterien getestet und die Aktivität der bevorzugten Beispiele SEQ ID NO. 18 und 24 bestimmt. Oncocin (SEQ ID NO. 18) war sowohl gegen *P. aeruginosa* 39324 (MHK 4 μg/mL) als auch gegen *P. mirabilis* und *P. vulgaris* (128 μg/mL) aktiv. Gegen Gram-positive *Staphylocaccus saprophyticus* 15305 Bakterien war Oncocin mit 16 μg/mL ebenfalls aktiv.

**Tabelle 11**: Antimikrobielle Aktivität gegen pathogene *Escherichia coli* DSM 10233, *Klebsiella pneumoniae* DSM 681 und *Pseudomonas aeruginosa* DSM 3227. Minimale Hemmkonzentration (MHK) bestimmt in 1%TSB.

| SEQ ID NO. | Sequenz | *E. coli* DSM 10233 | *K. pneumoniae* DSM 681 | *P. aeruginosa* DSM 3227 |
|---|---|---|---|---|
| 18 | VDKPPYLPRPRPPRRIYNR-NH$_2$ | 16 | 4 | >32 |
| 72 | VDKPPYLPRPRPPROIYNO-NH$_2$ | 32 | 4 | >32 |
| 63 | VDKPPYLPRPRPPR-4tHyp-IYNO-NH$_2$ | 1 | 2 | 16-32 |
| 107 | VDKPPYLPRPRP-4tHyp-ROIYNO-NH$_2$ | 16 | 4 | >32 |
| 108 | VDKPPYLPRPRP-4tHyp-R-4tHyp-IYNO-NH$_2$ | 1 | 2 | 16-32 |
| 111 | VDKPPYLPRPRPPRrIYNR-NH$_2$ | 4 | 4 | n.b. |
| 117 | rnyirrpprprplyppkdv-NH$_2$ | >32 | >32 | n.b. |

Derivate des Oncocins zeigen sehr gute Aktivität gegen pathogene Bakterien wie *E. coli* DSM 10233, *K. pneumoniae* DSM 681 und *P. aeruginosa* DSM 3227. Die Substitution mit 4-*trans*-Hydroxyprolin an Position 15 (SEQ ID NO. 63 und 108) führt zu erstaunlich hohen MHK-Werten gegen *E. coli* DSM 10233 zwischen 1-4 μg/mL, diese sind 16fach höher als für Oncocin (SEQ ID NO. 18). Gegenüber *K. pneumoniae* DSM 681 und *P. aeruginosa* DSM 3227 zeigen diese Derivate ebenfalls eine hohe Aktivität, was dsas breite Wirkungsspektrum der erfindungsgemäßen Peptide wiedergibt.

**Beispiel 4: Fluoreszenzmikroskopie**

HeLa- und SH-SY5Y-Zellen wurden in 96 Well Mikrotiterplatten (Greiner Bio-One GmbH, Frickenhausen, Deutschland) ausplattiert und über Nacht inkubiert. Am nächsten Tag wurden mit 5,6-Carboxyfluorescein-markierte Peptide oder Penetratinkonstrukte in frischem Medium gelöst (40 μmol/L) und die Zellen darin 2 h inkubiert. Die Zellen wurden zweimal mit PBS gewaschen und in PBS mittels Fluoreszenzmikroskopie untersucht.

Parameter für die Fluoreszenzmikroskopie

Mikroskop:      Leica DMI6000B (Leica Mikrosystems GmbH, Wetzlar, Deutschland)
Lichtquelle:     Leica EL6000 mit Metall-Halogen-Lampe
Objektiv:        N PLAN L 20x0,40 corr
Software:        Leica Application Suite 2.1.8.; Adobe Photoshop CS

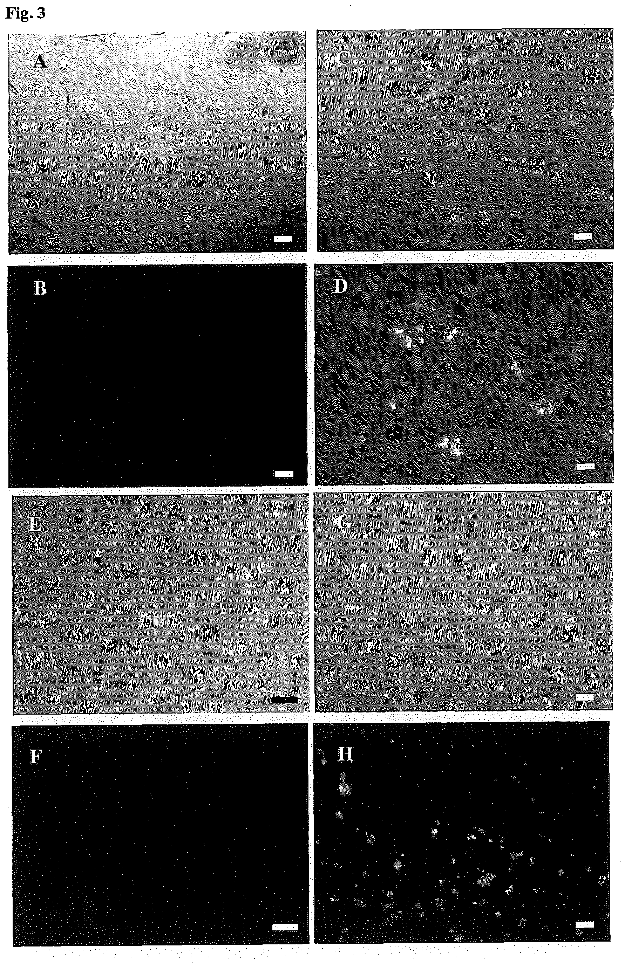

Die fluoresenzmikroskopischen Aufnahmen in Fig. 3 zeigen, dass nach Inkubation mit 40 μmol/L 5,6-Carboxyfluorescein-markiertem Oncocin (SEQ ID NO. 94) weder in HeLa- noch in SH-SY5Y-Zellen eine Fluoreszenz nachweisbar ist (Fig. 3B und F). Auch für 5,6-Carboxyfluorescein-markiertes Apidaecin 1b, Pyrrhocoricin und Drosocin (SEQ ID NO. 88, 90 und 92) konnte keine Fluoreszenz und somit keine Internalisierung dieser antimikrobiellen Prolin-reichen Peptide nachgewiesen werden. Nach Inkubation beider Zelllinien mit dem 5,6-Carboxyfluorescein-markiertem Penetratin-Oncocin (SEQ ID NO. 102) konnte dagegen in den mikroskopischen Aufnahmen eine starke Fluoreszenz im Zellinneren beobachtet werden (Fig. 3D und E), was die Internalisierung des gesamten Konstruktes belegt.
Die Penetratinkonstrukte mit Apidaecinlb, Pyrrhocoricin und Drosocin (SEQ ID NO. 96, 98 und 100) internalisieren ebenfalls in beide Zelllinien und zeigen deutliche Fluoreszenz.

**Beispiel 5: Konfokale Laser Scanning Mikroskopie**

Bakterien

Eine über Nacht kultivierte Bakteriensuspension wurde auf 150x10$^6$ Zellen/mL verdünnt und die die 5,6-Carboxyfluorescein-markierten Peptide und Penetratinkonstrukte wurden zugegeben (Endkonzentration von 30 μmol/L). Um die Fluoreszenz der Moleküle außerhalb der Bakterien zu quenchen wurden 60 eq 5,6-Carboxytetramethylrhodamin (TAMRA; Merck, Darmstadt, Deutschland) zugegeben. Die durch die Internalisierung der markierten Peptide in den Bakterien entstehende Fluoreszenz wurde sofort mit einem konfokalen Laser Scanning Mikroskop TCS SP5 der Firma Leica Microsystems GmbH (Wetzlar, Deutschland) untersucht.

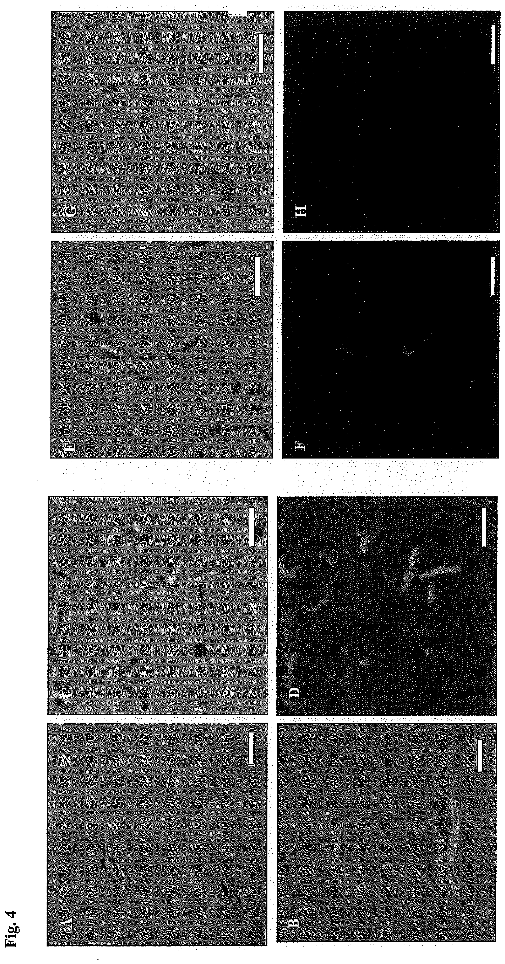

Nach 20 min Inkubation mit 5,6-Carboxyfluorescein-markiertem Oncocin, hatte sich das Peptid in der Bakterienmembran angereichert (Fig. 4B). Der Quenching-Effekt durch Fluoreszenz-Resonanz-Energie-Transfer (FRET) zwischen 5,6-Carboxyfluorescein und TAMRA, das ausserhalb der Zelle verbleibt, geht dadurch verloren und ein Fluoreszenzsignal kann detektiert werden. Nach weiteren 30 min hatte sich das Peptid im Inneren der Zelle angereichert (Fig. 4D). Die 5,6-Carboxyfluorescein-markierten Apidaecin 1b, Pyrrhocoricin und Drosocin Sequenzen (SEQ ID NO. 96, 98 und 100) internalisieren ebenfalls in *E. coli* und erzeugen eine deutliche Fluoreszenz. Im Gegensatz dazu erreichen die markierten Penetratinkonstrukte langsamer das Zellinnere und verursachen nach vergleichbaren Inkubationszeiten eine wesentlich schwächere Fluoreszenz (Fig. 4F). Diese Beobachtung korreliert mit den bestimmten minimalen Hemmkonzentrationen. So hatte das Penetratinkonstrukt des Oncocins mit 32 μg/mL (SEQ ID NO. 100) eine 8mal höheren MHK-Wert als das Oncocin (4 μg/mL; SEQ ID NO. 18) und damit wesentlich geringere Aktivität gegenüber *E. coli* (Tabelle 7). Der Eintritt des Penetratin-Homodimers konnte mittels Fluoreszenzmikroskopie auch nach 90 min nicht nachgewiesen werden (Fig. 4H). Das zeigt, dass im Penetratinkonstrukt die jeweilige Prolin-reiche Peptidsequenz das Penetratin als Kargo in die Bakterienzelle transportiert.

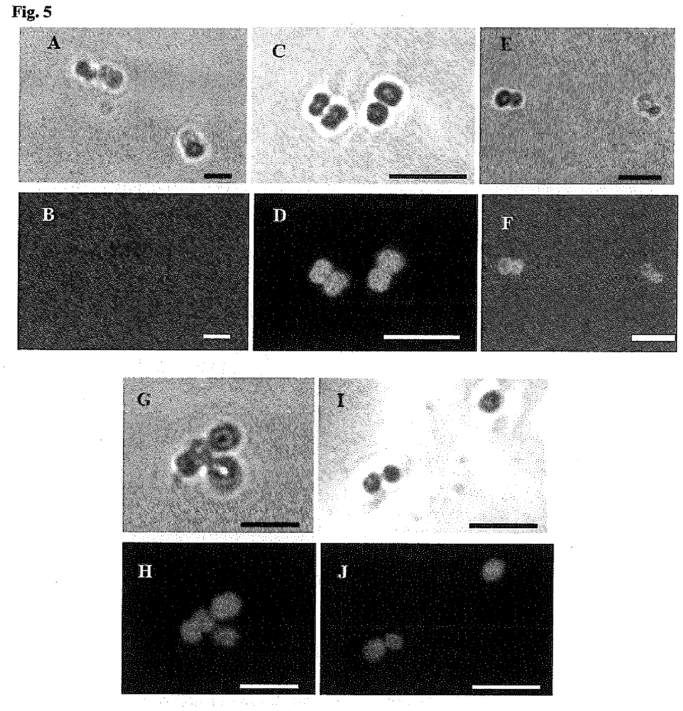

Im Gegensatz dazu, gelangt das 5,6-Carboxyfluorescein-markierte Penetratin-Homodimer in Gram-positive *M. luteus* Zellen und gibt dort nach 1 h Inkubation ein deutliches Fluoreszenzsignal (Fig. 5J). Das markierte Pyrrhocoricin kann nach der gleichen Inkubationszeit nicht in *M. luteus* nachgewiesen werden (Fig. 5B). Mit Penetratin als Transporter im markierten Penetratin-Pyrrhocoricin reichert sich das Derivat in *M. luteus* an und gibt ein starkes Fluoreszenzsignal (Fig. D). Der MHK-Wert sinkt von 128 μg/mL für Pyrrhocoricin auf 4 μg/mL für Penetratin-Pyrrhocoricin, was einer 32fachen Zunahme der Aktivität entspricht (Tabelle 7). Parallel dazu konnte eine 8fache Steigerung der Aktivität für Apidaecin 1b (64 μg/mL) im Penetratin-Apidaecin Derivat (8 μg/mL) bestimmt werden.

HeLa- und SH-SY5Y-zellen

Die Zellen wurden auf Glasboden Kulturschalen der Firma MatTek Corporation (Ashland, MA, USA) kultiviert und mit 5,6-Carboxyfluorescein-markierten Penetratinkonstrukten (10 μmol/L für SH-SY5Y oder 7 μmol/L für HeLa) für 2 h oder 24 h inkubiert. Das Medium wurde entfernt, zweimal mit PBS gewaschen und neues Medium zugefügt. Zur Färbung des Zellkerns wurde der Farbstoff Hoechst 33342 (Fluka Chemie GmbH, Buchs, Schweiz) zugefügt und weitere 15 min inkubiert. Die Fluoreszenz wurde sofort mit dem konfokalen Laser Scanning Mikroskop TCS SP5 analysiert. Alle Bilder wurden in einem sequentiellen Scan Modus aufgenommen und die Bilderstapel mit dem Leica Application Suite Advanced Fluorescence 1.7.1 Programm (Leica Microsytems) und Adobe Photoshop CS (Adobe Systems GmbH, München, Deutschland) analysiert.

Die Ergebnisse zeigen, dass die antimikrobiellen Peptide, die N-terminal um 5,6-Carboxyfluorescein-Penetratin verlängert wurden, innerhalb von 2 Stunden in die Zellen eindringen. Dagegen waren die nur mit 5,6-Carboxyfluorescein markierten antimikrobiellen Peptide ohne die Penetratin-Sequenz nicht in den Zellen nachweisbar, d.h. dass diese Peptide nicht durch die äußere Zellmembran in die getesteten Zelllinien eindringen können. Der Transport ins Zellinnere erfolgt nur über die Penetratin-Sequenz. Die Färbung der Zellkerne mit dem Farbstoff Hoechst 33342 und der Mitochondrien mit dem Farbstoff MitoTracker Red CMXRos konnte eine Lokalisierung der Peptide in diesen Kompartimenten ausgeschlossen werden. Nach längerer Inkubationszeit (24 h) konzentrierte sich die vom 5,6-Carboxyfluorescein emittierte Fluoreszenz in der Nähe des Zellkerns. Durch Färbung des Golgi konnte eine partielle Co-Lokalisierung nachgewiesen werden.

**Beispiel 6: Zytotoxizität**

MTT-Tests mit HeLa- und SH-SY5Y-Zellen

Die Zytotoxizität der Peptide, Peptidomimetika und Penetratinkonstrukte wurde mit dem "Cell Proliferation Kit I" der Firma Roche Diagnostics GmbH (Mannheim, Deutschland) bestimmt. Das Verfahren beruht auf der Reduktion des gelben Methylthiazolyldiphenyltetrazolium Bromids (MTT) durch zelluläre Oxidoreduktasen metabolisch aktiver Zellen (Vistica D T et al. Tetrazolium-Based Assays for Cellular Viability - A Critical-Examination of Selected Parameters Affecting Formazan Production. Cancer Research 51: 2515-20, 1991; Slater T F, Sawyer B, & Strauli U. Studies on Succinate-Tetrazolium Reductase Systems .3. Points of Coupling of 4 Different Tetrazolium Salts. Biochimica et Biophysica Acta 77: 383-&, 1963; Berridge M V & Tan A S. Characterization of the Cellular Reduction of 3-(4,5-Dimethylthiazol-2-Yl)-2,5-Diphenyltetrazolium Bromide (Mtt) - Subcellular-Localization, Substrate Dependence, and Involvement of Mitochondrial Electron-Transport in Mtt Reduction. Archives of Bioehemistry and Biophysics 303: 474-82, 1993). Die Bildung des wasserunlöslichen purpurfarbene Formazan Produkts ist proportional zur Zahl der lebensfähigen Zellen und kann nach Lyse der Zellen photometrisch detektiert werden.

Die Zellkultivierung erfolgte in Zellkulturflaschen (25 cm$^2$) mit Filterkappe bzw. in 96-well Mikrotiterplatten (Greiner Bio-One GmbH, Frickenhausen, Deutschland) bei 37°C unter 5% $CO_2$ und 95% Luftfeuchte. Alle Medien und Zusätze wurden von PAA Laboratories (Pasching, Österreich) bezogen. MEM/Glutamin Medium mit 5% fötalem Kälberserum (HeLa) bzw. DMEM/HAM's F-12 Medium mit 15% fötalem Kälberserum (SH-SY5Y) wurde jeweils 1% nicht essentielle Aminosäuren und 1% Penicillin/Streptomycin zugesetzt.

HeLa bzw. SH-SY5Y Zellen wurden in sterilen 96-well Mikrotiterplatten mit einer Konzentration von 2x10$^4$ Zellen/well ausplattiert und über Nacht inkubiert. Die Zellen wurden einmal mit sterilem PBS gewaschen und die Peptide in 100 $\mu$L frischem Medium gelöst zugegeben. Als Negativ- bzw. Positivkontrolle diente 12% PBS bzw. 12% DMSO in Medium. Nach Inkubation (24 h) wurden 10 $\mu$L MTT-Reagenz zugegeben um eine Endkonzentration von 0,5 mg zu erreichen und für weitere vier Stunden bei 37°C inkubiert. Mit einer 10%igen Natriumdodecylsulfat-Lösung in 0,01 mol/L Salzsäure wurden die Zellen und das kristalline Formazan gelöst und die Absorption nach 16 h bei 590 und 650 nm mit dem Paradigm™ Mikroplatten Reader (Beckman Coulter GmbH, Wals, Österreich) bestimmt.

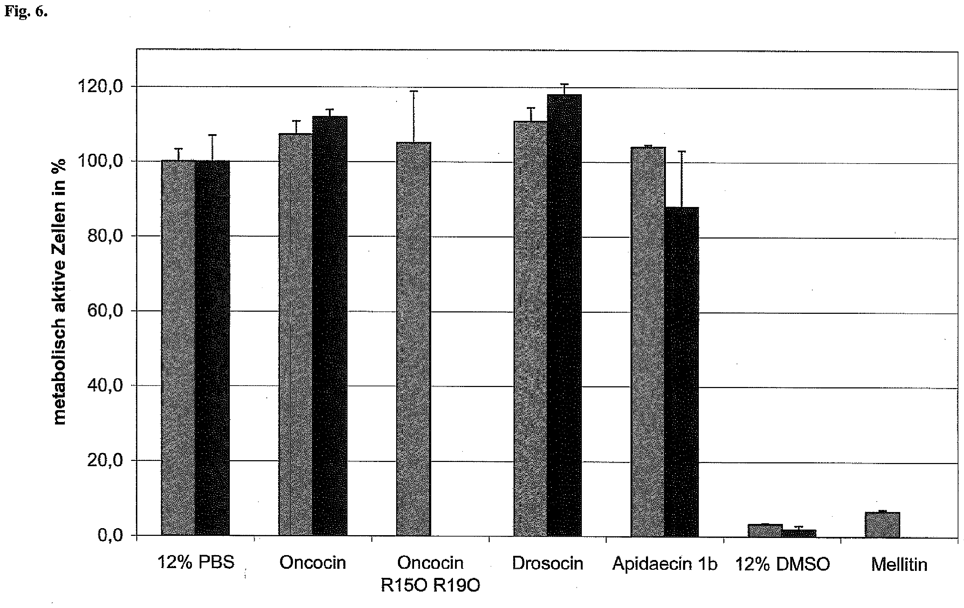

Die überraschenden Ergebnisse zeigen, dass keines der getesteten antimikrobiellen Prolin-reichen Peptide bei 600 $\mu$g/mL toxische Effekte auf SH-SY5Y- oder HeLa-Zellen (Fig. 6) hat. Die Experimente wurden dreimal unabhängig als Dreifachbestimmung durchgeführt und der Anteil metabolisch aktiver Zellen auf die Negativkontrolle 12% PBS in Medium normalisiert. Die Ergebnisse werden dadurch bestätigt, dass die fluoreszenzmarkierten Derivate dieser Peptide nach einer Inkubationszeit von einer Stunde im Inneren dieser Zellen nicht nachgewiesen werden konnten (siehe Beispiel 5). Eine Interaktion mit extrazellulären Zielmolekülen oder Rezeptoren in der äußeren Zellmembran konnte so für SH-SY5Y- und HeLa-Zellen ebenfalls ausgeschlossen werden.

Die Zytotoxizitätstests der Penetratin-Konstrukte wurden in drei unabhängigen Experimenten einer Verdünnungsreihe 50-400 $\mu$g/mL als Dreifachbestimmung durchgeführt. Das Penetratin-Monomer (SEQ ID NO. 105) und eine Penetratin-Tau Sequenz, die als Kontrolle diente, zeigte bis zur höchsten Konzentration von 400 $\mu$g/mL keine toxische Wirkung gegen HeLa Zellen (Fig. 6). Bei den Penetratin-Konstrukten mit antimikrobiellen Peptiden konnten zwischen 100 und 400 $\mu$g/mL vernachlässigbar geringe toxische Effekte beobachtet werden.. Das Penetratin-Homodimer (SEQ ID NO. 103) war bei 400 $\mu$g/mL fast 100% toxisch, während Penetratin-Drosocin und Penetratin-Oncoin (SEQ ID NO. 97 und 101) geringer Toxizitäten zeigten.. In den Untersuchungen mit SH-SY5Y-Zellen reduzierte das Penetratin-Monomer und das Penetratin-Tau-Konstrukt bei 400 $\mu$g/mL den Anteil wachsender Zellen auf 70%. Alle Penetratin-AMP Konstrukte zeigten überraschend keine toxisch Wirkung in den bevorzugten Kontentrationen.

Hämolysetest

Eine weitere Möglichkeit die Zytotoxizität der Peptide und Peptidderivate zu untersuchen ist der Hämolysetest. Die hämolytische Aktivität wird dabei an humanen Erythrozyten untersucht (Ryge T S & Hansen P R. Potent antibacterial lysine-peptoid hybrids identified from a positional scanning combinatorial library. Bioorganic & Medicinal Chemistry 14: 4444-51, 2006), die das Leipziger Universitätskrankenhaus (Deutschland) als humanes Erythrozyten-Konzentrat in Natriumchlorid-Adenin-Glukose-Mannitol-Puffer (Lagerung 4°C) zur Verfügung stellte. Die Erythrozyten wurden bei 1000 G abzentrifugiert und drei Mal mit dem zehnfachen Volumen an kalter Phosphat-gepufferter Kochsalzlösung (PBS, pH 7,4) gewaschen. Die Erythrozyten wurden auf eine Endkonzentration von 1% in PBS verdünnt. Einhundert Mikroliter Erythrozytensuspension wurde in jede V-förmige Vertiefung einer 96-well Polypropylen-Mikrotiterplatte (Greiner Bio-One GmbH) pipettiert. In jede Position wurden dann 100 $\mu$L der in PBS gelösten Peptide hinzugefügt, um eine Verdünnungsreihe von 600 $\mu$g/mL bis zu 4,7 $\mu$g/mL in sieben Verdünnungsstufen zu erhalten. Die Mikrotiterplatte wurde für 1 h bei 37°C inkubiert und anschließend bei 1000*g zentrifugiert. Vom Überstand wurden 100 $\mu$L abgenommen, in eine 96-well Polystyrol- Mikrotiterplatte mit Flachboden überführt (Greiner Bio-One GmbH) und die Absorption bei 405 nm in einem Sunrise Mikrotiterplatten Reader (Tecan Trading AG, Mannedorf, Schweiz) bestimmt, um die Freisetzung der Hämgruppe auszuwerten. PBS bzw. 0,1% Triton X-100® ((p-tert-Octylphenoxy) polyethoxyethanol; Fluka Chemie GmbH, Buchs, Schweiz) und Melittin (SIGMA-Aldrich-Laborchemikalien, Taufkirchen, Deutschland) wurden als Negativ- bzw. Positivkontrollen eingesetzt. Alle Hämolysetests wurden zweimal unabhängig als Doppelbestimmung durchgeführt und der Hämolysegrad mit folgender Gleichung bestimmt (Park Y et al. A Leu- Lys-rich antimicrobial peptide: activity and mechanism. Biochimica et Biophysica Acta-Proteins and Proteomics 1645: 172-82, 2003):

$$(E_{Peptid} - E_{PBS}) / (E_{Triton} - E_{PBS}) \times 100\% \qquad E = \text{Extinktion bei 405 nm}$$

Keines der analysierten antimikrobiellen Prolin-reichen Peptide zeigte hämolytische Aktivität bis zu einer Konzentration von 600 μg/mL (Tabelle 12). Das bedeutet selbst bei der 100fach höheren Konzentration als die bestimmten MHK-Werte konnte keine Lyse der humanen Erythrozyten beobachtet werden. Die Hämolyseraten aller Peptide betrugen relativ gesehen zum Triton X-100® nur etwa 1%, was innerhalb der Fehlergrenzen dieses Tests liegt. Das nicht-ionische Tensid Triton X-100® wurde als Positivkontrolle eingesetzt, da es rote Blutzellen in dem Versuchsaufbau innerhalb einer Stunde komplett zerstört. Melittin, das Gift der Honigbiene, hat als α-helikales Peptid stark lytische Wirkung auf biologische Membranen und zerstört sowohl prokaryotische als auch eukaryotische Zellmembranen bereits in Konzentrationen von 5 μg/mL. Dieser Zelltest zeigt, dass die Peptide und Peptidderivate in hohen Konzentrationen im Blut eingesetzt werden können, ohne Nebeneffekte auf humane Erythrozyten zu haben.

**Tabelle 12:** Hämolysegrad ausgewählter antimikrobieller Peptide.

| SEQ ID NO. | Name | Hämolysegrad [%]bei 600 μg/mL |
|---|---|---|
| 18 | Oncocin | 1,5 |
| 87 | Apidaecin 1b | 1,2 |
| 89 | Drosocin | 1,1 |
|  | Melittin | 96,3 |
|  | Triton X-100® | 100 |

**Abbildungslegenden:**

**Fig. 1** zeigt die antibakterielle Aktivität der Oncocin-Analoga (Ala-Scan) gegen E. coli BL 21 AI in Agar-Diffusions-Tests. Gestreifte Balken zeigen partielle Inhibierung an.

**Fig. 2** zeigt die antibakterielle Aktivität von Oncocin-Analoga (Alanin Scan) gegen Micrococcus luteus ATCC 10240 in Agar-Diffusions-Tests

**Fig. 3** zeigt Fluoreszenzmikroskopische Aufnahmen von HeLa- und SH-SY5Y-Zellen nach Inkubation mit 5,6-Carboxyfluorescein-markiertem Oncocin (CF-Oncocin SEQ ID NO. 94) und Penetratin-Oncocin (CF-Penetratin-Oncocin SEQ ID NO. 102). Obere Reihe: Phasenkontrast; Untere Reihe: Fluoreszenz (517 nm Emission). A, B: SH-SY5Y inkubiert mit CF-Oncocin; C, D: SH-SY5Y mit CF-Penetratin-Oncocin; E, F: HeLa inkubiert mit CF-Oncocin; G, H: HeLa inkubiert mit CF-Penetratin-Oncocin. Balken entsprechen 20 μm.

**Fig. 4** zeigt Konfokale Laser Scanning Mikroskopie Aufnahmen von *E. coli* BL21AI. Peptidkonzentration 30 μmol/L; TAMRA Konzentration 180 μmol/L. Obere Reihe: Phasenkontrast; untere Reihe: Fluoreszenz. A, B: 20 min Inkubation mit CF-Oncocin; C, D: 50 min Inkubation mit CF-Oncocin; EF: 50 min Inkubation mit CF-Penetratin-Oncocin; G, H: 90 min Inkubation mit CF-Penetratin-Homodimer. Balken entsprechen 5 μm.

**Fig. 5** zeigt Konfokale Laser Scanning Mikroskopie Aufnahmen von *M. luteus* 10240. Peptidkonzentration 30 μmol/L; TAMRA Konzentration 180 μmol/L. Obere Reihe: Phasenkontrast; untere Reihe: Fluoreszenz. A, B: CF-Pyrrhocoricin; C, D: CF-Penetratin-Pyrrhocoricin; E, F: CF-Drosocin; G, H: CF-Penetratin-Drosocin; I, J: CF-Penetratin-Homodimer. Balken entsprechen 5μm

**Fig. 6** zeigt die Ergebnisse des Zytotoxizitätstest für die antimikrobiellen Peptide gegenüber SH-SY5Y- (gestreifte Balken) und HeLa-Zellen (schwarze Balken) bestimmt mit dem "Cell Proliferation Kit I". Test nach 24 h Inkubation mit 600 μg/mL Oncocin, Oncocin R15O R19O, Drosocin und Apidaecin 1b (SEQ ID NO. 18, 72, 89 und 87) in Medium. Positivkontrollen 12% DMSO und 100 μg/mL Melittin. Normalisiert auf Negativkontrolle 12% PBS.

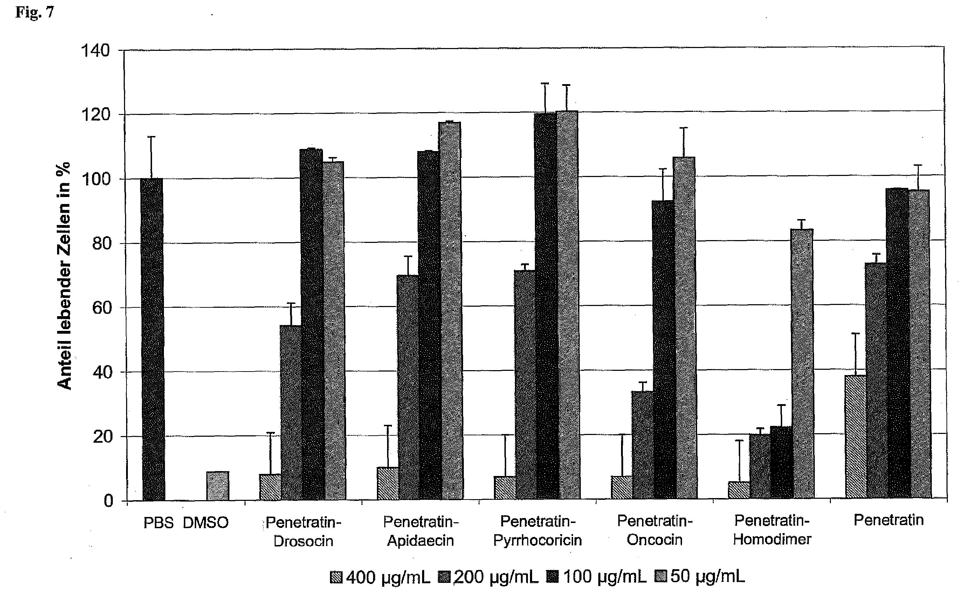

**Fig. 7** zeigt die Ergebnisse des Zytotoxizitätstest für die Penetratin-Konstrukte gegenüber HeLa-Zellen bestimmt mit dem "Cell Proliferation Kit I". Test nach 24 h Inkubation mit 50-400 µg/mL Penetratin-Drosocin (SEQ ID NO. 96), Penetratin-Apidaecin 1b (SEQ ID NO. 94), Penetratin-Pyrrhocoricin (SEQ ID NO. 98), Penetratin-Oncocin (SEQ ID NO. 100), Penetratin-Homodimer (SEQ ID NO. 102), Penetratin (SEQ ID NO. 105) in Medium. Negativkontrolle 12% PBS und Positivkontrolle 12% DMSO.

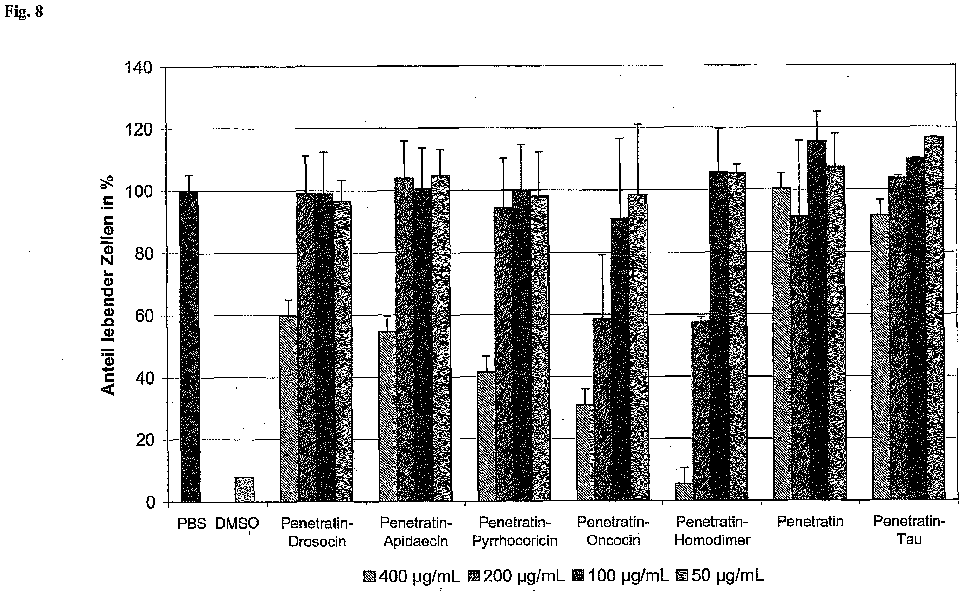

**Fig. 8** zeigt die Ergebnisse des Zytotoxizitätstest für die Penetratin-Konstrukte gegenüber SH-SY5Y-Zellen bestimmt mit dem "Cell Proliferation Kit I". Test nach 24 h Inkubation mit 50-400 µg/mL Penetratin-Drosocin (SEQ ID NO. 96), Penetratin-Apidaecin 1b (SEQ ID NO. 94), Penetratin-Pyrrhocoricin (SEQ ID NO. 98), Penetratin-Oncocin (SEQ ID NO. 100), Penetratin-Homodimer (SEQ ID NO. 102), Penetratin (SEQ ID NO. 105), Penetratin-Tau (SEQ ID NO. 106) in Medium. Als Negativkontrolle wurde 12% PBS und als Positivkontrolle 12% DMSO eingesetzt.

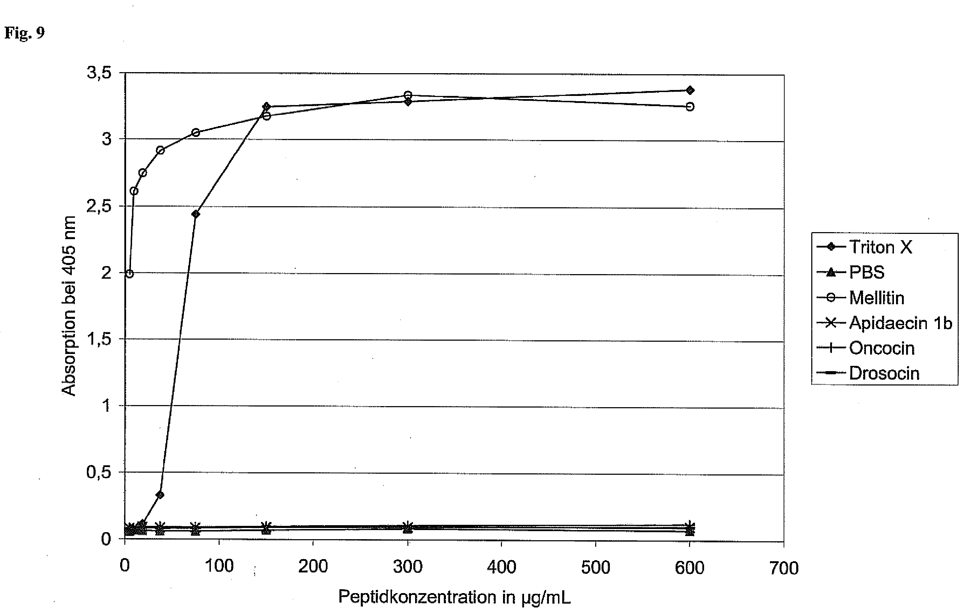

**Fig. 9** zeigt die Ergebnisse des Hämolyse-Test für die Peptide Oncocin, Drosocin und Apidaecin 1b (SEQ ID NO. 18, 89 und 87). Peptidverdünnungsreihe 4,7-600 µg/mL. Positivkontrollen: Melittin und TritonX-100®, Negativkontrolle PBS.

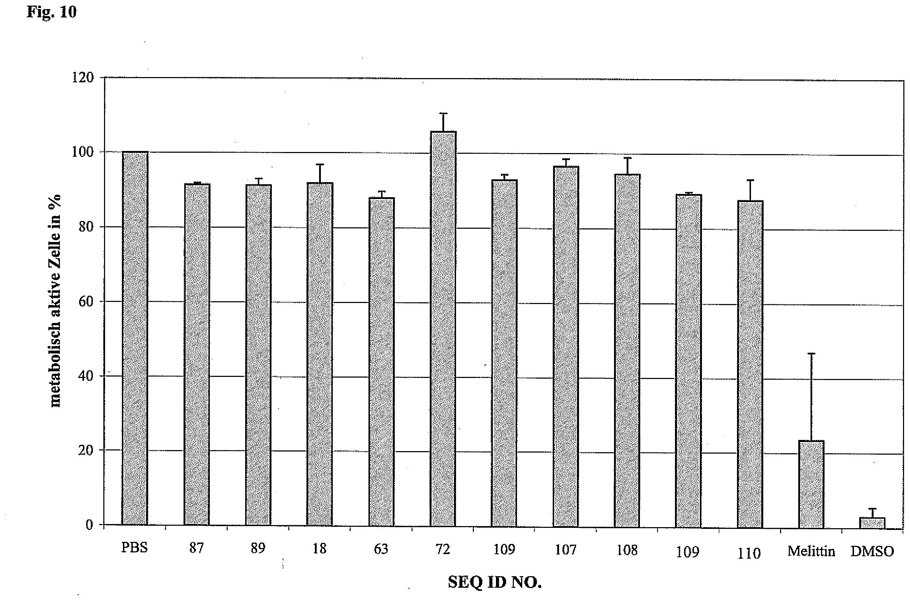

**Fig. 10:** zeigt die Ergebnisse des Zytotoxizitätstest für die antimikrobiellen Peptide gegenüber HeLa-Zellen bestimmt mit dem "Cell Proliferation Kit I". Test nach 24 h Inkubation mit 600 µg/mL Oncocin und Derivaten des Oncocins (SEQ ID NO. 18, 63, 72, 107 bis 110), sowie den Vergleichsbeispielen Apidaecin 1b und Drosocin (SEQ ID NO. 87 und 89) in Medium. Positivkontrollen 12% DMSO und 100 µg/mL Melittin. Normalisiert auf Negativkontrolle 12% PBS. Das Diagramm zeigt den Mittelwert von zwei unabhängigen Versuchen mit Triplikaten.

**Patentansprüche**

1. Peptid, umfassend eine der folgenden Sequenzen:

   SEQ ID NOs. 61-74, 111 und 112.

2. Peptid enthaltend ein anti-bakterielles Peptid und ein Zellpenetrierendes Peptid, wobei besagtes antibakterielles Peptid ein Peptid gemäß Anspruch 1 ist.

3. Peptid gemäß Anspruch 2, wobei besagtes Zell-penetrierendes Peptid ausgewählt ist aus der Gruppe umfassend Penetratin, Tat-Peptide, amphipathische Modell-Peptide, Transportan, SynB und cis-γ-amino-L-Prolin-haltige Peptide.

4. Peptid gemäß irgendeinem der Patentansprüche 1 bis 3, **dadurch gekennzeichnet, dass** mindestens eine der Peptidbindungen des Peptidrückgrats chemisch modifiziert ist.

5. Multimer, in dem mindestens zwei Peptide miteinander verbunden sind, **dadurch gekennzeichnet, dass** mindestens eines der Peptide ein Peptid gemäß irgendeinem der Patentansprüche 1 bis 4 ist.

6. Pharmazeutische Zusammensetzung, **dadurch gekennzeichnet, dass** sie mindestens ein Peptid oder Multimer gemäß irgendeinem der Patentansprüche 1 bis 5 enthält.

7. Verwendung eines Peptids oder Multimers gemäß einem der Patentansprüche 1 bis 5 als Desinfektions- und/oder Reinigungsmittel, als Konservierungsmittel und/oder in einem Verpackungsmaterial.

8. Peptid und/oder Multimer gemäß irgendeinem der Patentansprüche 1 bis 5 zur Anwendung in der Behandlung von mikrobiellen, bakteriellen oder Pilzinfektionen.

9. Ein Verfahren zur Identifizierung einer Substanz mit anti-bakterieller oder antimykotischer Wirkung, umfassend die folgenden Schritte:

(i) Durchführung eines kompetitiven Assays mit:

(a) einem Mikroorganismus der gegenüber einem Peptid oder Multimer gemäß irgendeinem der Patentansprüche 1 bis 5 empfindlich ist;
(b) einem Peptid oder Multimer gemäß irgendeinem der Patentansprüche 1 bis 5 und
(c) mindestens einer zu testenden Substanz durch in Kontakt bringen von (a) mit (b) und (c); sowie

(ii) Auswahl einer Testsubstanz, welche kompetitiv die Bindung des Peptids oder Multimers an den Mikroorganismen verdrängt.

**Claims**

1. A peptide, comprising one of the following sequences: SEQ ID NOs. 61-74, 111 and 112.

2. A peptide including an anti-bacterial peptide and a cell-penetrating peptide, wherein said anti-bacterial peptide is a peptide according to claim 1.

3. A peptide according to claim 2, wherein said cell-penetrating peptide is selected from the group comprising Penetratin, Tat-peptides, amphipathic model-peptides, Transportan, SynB and cis-γ-amino-L-Prolin-containing peptides.

4. A peptide according any of the claims 1 to 3, **characterized in that** at least one of the peptide-bonds of the peptide backbone is chemically modified.

5. Multimer, in which at least two peptides are attached to each other, **characterized in that** at least one of the peptides is a peptide according to any of the claims 1 to 4.

6. Pharmaceutical composition, **characterized in that** it includes at least one peptide or multimer according to any of the claims 1 to 5.

7. Use of a peptide or multimer according to claims 1 to 5 as disinfectant and/or detergent, as preserving agent and/or in a packaging material.

8. Peptide and/or multimer according to any of the claims 1 to 5, for use in the treatment of microbial, bacterial or fungal infections.

9. A method for identifying a substance with anti-bacterial or anti-mycotic effect, comprising the following steps:

(i) Conduction of a competitive assay with:

(a) a microorganism that is sensitive to a peptide or multimer according to any of the claims 1 to 5;
(b) a peptide or multimer according to any of the claims 1 to 5 and
(c) at least one substance to be tested by bringing into contact (a) with (b) and (c); as well as

(ii) Selection of a test substance that is competitively displacing the binding of the peptide or multimer to the microorganism.

**Revendications**

1. Peptide, comprenant une des séquences suivantes : SEQ ID NO. 61-74, 111 et 112.

2. Peptide contenant un peptide antibactérien et un peptide pénétrant dans les cellules, ledit peptide antibactérien étant un peptide selon la revendication 1.

3. Peptide selon la revendication 2, ledit peptide pénétrant dans les cellules étant choisi dans le groupe comprenant la pénétratine, le peptide Tat, les peptides modèles amphipathiques, le transportan, le SynB et les peptides contenant de la cis-γ-amino-L-proline.

4. Peptide selon l'une quelconque des revendications 1 à 3, **caractérisé en ce qu'**au moins une des liaisons peptidiques de la chaîne principale peptidique est modifiée chimiquement.

5. Multimère, dans lequel au moins deux peptides sont liés l'un à l'autre, **caractérisé en ce qu'**au moins un des peptides est un peptide selon l'une quelconque des revendications 1 à 4.

6. Composition pharmaceutique, **caractérisée en ce qu'**elle contient au moins un peptide ou multimère selon l'une quelconque des revendications 1 à 5.

7. Utilisation d'un peptide ou d'un multimère selon l'une quelconque des revendications 1 à 5 comme agent de désinfection et/ou de nettoyage, comme conservateur et/ou dans un matériau d'emballage.

8. Peptide et/ou multimère selon l'une quelconque des revendications 1 à 5 pour une utilisation dans le traitement d'infections microbiennes, bactériennes ou fongiques.

9. Procédé pour identifier une substance présentant un effet antibactérien ou antimycotique, comprenant les étapes suivantes :

   (i) réalisation d'un essai compétitif avec :

   (a) un micro-organisme qui est sensible à un peptide ou à un multimère selon l'une quelconque des revendications 1 à 5 ;
   (b) un peptide ou un multimère selon l'une quelconque des revendications 1 à 5 et
   (c) au moins une substance à tester par mise en contact de (a) avec (b) et (c) ; ainsi que

   (ii) sélection d'une substance test qui déplace de manière compétitive la liaison du peptide ou du multimère aux micro-organismes.

# Figuren

**Fig. 1**

Hemmzonendurchmesser in cm — Alanin-substituierte Aminosäuren in der Oncocin-Sequenz

**Fig. 2**

Hemmzonendurchmesser in cm — Alanin-substituierte Aminosäure in der Oncocin-Sequenz

Fig. 3

Fig. 4

Fig. 5

Fig. 6.

EP 2 391 636 B1

Fig. 7

Fig. 8

EP 2 391 636 B1

Fig. 9

Fig. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009007381

**In der Beschreibung aufgeführte Nicht-Patentliteratur**

- **TOMASZ A.** Multiple-Antibiotic-Resistant Pathogenic Bacteria - A Report on the Rockefeller-University Workshop. *New England Journal of Medicine,* 1994, vol. 330, 1247-51
- **WENZEL R P.** The Mortality of Hospital-Acquired Blood-Stream-Infections - Need for A New Vital Statistic. *International Journal of Epidemiology,* 1988, vol. 17, 225-7
- **MOELLERING R C.** Emerging resistance with gram-positive aerobic infections: Where do we go from here? Introduction: Problems with antimicrobial resistance in gram-positive cocci. *Clinical Infectious Diseases,* 1998, vol. 26, 1177-8
- **HAND W L.** Current challenges in antibiotic resistance. *Adolescent Medicine,* 2000, vol. 11, 427-38
- **HOOPER D C.** Emerging mechanisms of fluoroquinolone resistance. *Emerging Infectious Diseases,* 2001, vol. 7, 337-41
- **JONES R N.** Resistance patterns among nosocomial pathogens. *Trends over the past few years. Chest,* 2001, vol. 119, 397S-404S
- **PRACHAYASITTIKUL V ; LAWUNG R ; BULOW L.** Episome profiles and mobilizable beta-lactamase plasmid in Haemophilus ducreyi. *J Trop Med Public Health,* 2000, vol. 31, 80-4
- **TEUBER M.** Spread of antibiotic resistance with food-borne pathogens. *Cellular and Molecular Life Sciences,* 1999, vol. 56, 755-63
- **BOMAN H G.** Peptide Antibiotics and Their Role in Innate Immunity. *Annual Review of Immunology,* 1995, vol. 13, 61-92
- **BARRA D ; SIMMACO M ; BOMAN H G.** Gene-encoded peptide antibiotics and innate immunity. Do 'animalcules' have defence budgets?. *Febs Letters,* 1998, vol. 430, 130-4
- **LUDTKE S ; HE K ; HUANG H.** Membrane thinning caused by magainin 2. *Biochemistry,* 1995, vol. 34, 16764-9
- **WIMLEY W C ; SELSTED M E ; WHITE S H.** Interactions Between Human Defensins and Lipid Bilayers - Evidence for Formation of Multimeric Pores. *Protein Science,* 1994, vol. 3, 1362-73
- **SHAI Y.** Molecular Recognition Between Membrane-Spanning Polypeptides. *Trends in Biochemical Sciences,* 1995, vol. 20, 460-4
- **WADE D et al.** All-D Amino Acid-Containing Channel-Forming Antibiotic Peptides. *Proceedings of the National Academy of Sciences of the United States of America,* 1990, vol. 87, 4761-5
- **STEINER H ; ANDREU D ; MERRIFIELD R B.** Binding and Action of Cecropin and Cecropin Analogs - Antibacterial Peptides from Insects. *Biochimica et Biophysica Acta,* 1988, vol. 939, 260-6
- **OTVOS L et al.** Insect peptides with improved protease-resistance protect mice against bacterial infection. *Protein Science,* 2000, vol. 9, 742-9
- **KRAGOL G et al.** Identification of crucial residues for the antibacterial activity of the proline-rich peptide, pyrrhocoricin. *European Journal of Biochemistry,* 2002, vol. 269, 4226-37
- **KRAGOL G et al.** The antibacterial peptide pyrrhocoricin inhibits the ATPase actions of DnaK and prevents chaperone-assisted protein folding. *Biochemistry,* 2001, vol. 40, 3016-26
- **ROSENGREN K J et al.** Cyclization of pyrrhocoricin retains structural elements crucial for the antimicrobial activity of the native peptide. *Biopolymers,* 2004, vol. 76 (5), 446-58
- **SCHNEIDER M ; DORN A.** Differential infectivity of two Pseudomonas species and the immune response in the milkweed bug, oncopeltus fasciatus (Insecta : Hemiptera). *Journal of Invertebrate Pathology,* 2001, vol. 78, 135-40
- **CASTEELS P ; AMPE C ; JACOBS F ; VAECK M ; TEMPST P.** Apidaecins - Antibacterial Peptides from Honeybees. *Embo Journal,* 1989, vol. 8, 2387-91
- **BULET P et al.** A Novel Inducible Antibacterial Peptide of Drosophila Carries An O-Glycosylated Substitution. *Journal of Biological Chemistry,* 1993, vol. 268, 14893-7
- **MACKINTOSH J A et al.** Isolation from an ant Myrmecia gulosa of two inducible O-glycosylated proline-rich antibacterial peptides. *Journal of Biological Chemistry,* 1998, vol. 273, 6139-41

- **COCIANCICH S et al.** Novel Inducible Antibacterial Peptides from A Hemipteran Insect, the Sap-Sucking Bug Pyrrhocoris-Apterus. *Biochernical Jourrial,* 1994, vol. 300, 567-75
- **CHERNYSH S ; COCIANCICH S ; BRIAND J P ; HETRU C ; BULET P.** The inducible antibacterial peptides of the hemipteran insect Palomena prasina: Identification of a unique family of proline-rich peptides and of a novel insect defensin. *Journal of Insect Physiology,* 1996, vol. 42, 81-9
- **LANGEL, U.** Handbook of Cell-Penetrating Peptides. CRC - Taylor & Francis Group, 2006, vol. 5, 28
- **PUJALS S ; GIRALT E.** Proline-rich, amphipathic cell-penetrating peptides. *Adv Drug Deliv Rev.,* 2008, vol. 60 (4-5), 473-84
- **FARRERA-SINFREU J ; GIRALT E ; ROYO M ; AL-BERICIO F.** Cell-penetrating proline-rich peptidomimetics. *Methods Mol Biol,* 2007, vol. 386, 241-67
- **MERRIFIELD R B.** Solid Phase Peptide Synthesis .1. Synthesis of A Tetrapeptide. *Journal of the Americau Chemical Society,* 1963, vol. 85, 2149
- **STEMMER W P C ; CRAMERI A ; HA K D ; BRENNAN T M ; HEYNEKER H L.** Single-Step Assembly of A Gene and Entire Plasmid from Large Numbers of Oligodeoxyribonucleotides. *Gene,* 1995, vol. 164, 49-53
- **GETHING M J ; SAMBROOK J.** Cell-Surface Expression of Influenza Hemagglutinin from A Cloned Dna Copy of the Rna Gene. *Nature,* 1981, vol. 293, 620-5
- **MAENO M ; TAGUCHI S ; MOMOSE H.** Production of Antibacterial Peptide Apidaecin Using the Secretory Expression System of Streptomyces. *Bioscience Biotechstology and Biochemistry,* 1993, vol. 57, 1206-7
- **ZHOU Q F ; LUO X G ; YE L ; XI T.** High-level production of a novel antimicrobial peptide perinerin in Escherichia coli by fusion expression. *Current Micnobiology,* 2007, vol. 54, 366-70
- **SI L G ; LIU X C ; LU Y Y ; WANG G Y ; LI W M.** Soluble expression of active human beta-defensin-3 in Escherichia coli and its effects on the growth of host cells. *Chinese Medical Journal,* 2007, vol. 120, 708-13
- **NOREN C J ; ANTHONYCAHILL S J ; GRIFFITH M C ; SCHULTZ P G.** A General-Method for Site-Specific Incorporation of Unnatural Amino-Acids Into Proteins. *Science,* 1989, vol. 244, 182-8
- **ELLMAN J ; MENDEL D ; ANTHONYCAHILL S ; NOREN C J ; SCHULTZ P G.** Biosynthetic Method for Introducing Unnatural Amino-Acids Site-Specifically Into Proteins. *Methods in Enzmology,* 1991, vol. 202, 301-36
- **ANDERSON W F.** Human gene therapy. *Nature,* 1998, vol. 392, 25-30
- Pharmaceutical Biotechnology. Harwood Academic Publishers, 1997, 8-20, 53-70, 123-152, 167-180
- Protein Synthesis: Methods and Protocols. Humana Press, 1998, 1-144
- Amino Acid and Peptide Synthesis. Oxford University Press, 1997, 1-89
- Solid-Phase Peptide Synthesis. Academic Press, 1997, 1-780
- **TAM J P ; MORA A L ; RAO C.** Lipidation as a novel approach to mucosal immunization. *Modulation of the Immune Response to Vaccine Antigens,* 1998, vol. 92, 109-16
- **POSNETT D N ; MCGRATH H ; TAM J P.** A Novel Method for Producing Anti-Peptide Antibodies - Production of Site-Specific Antibodies to the T-Cell - Antigen Receptor Beta-Chain. *Journal of Biological Chemistry,* 1988, vol. 263, 1719-25
- Remington's Pharmaceutical Sciences. Mack Pub. Co, 1991
- **MORELL M ; ESPARGARO A ; AVILES F X ; VENTURA S.** Detection of transient protein-protein interactions by bimolecular fluorescence complementation: The Abl-SH3 case. *Proteomics,* 2007, vol. 7, 1023-36
- **FIELDS G B ; NOBLE R L.** Solid-Phase Peptide-Synthesis Utilizing 9-Fluorenylmethoxycarbonyl Amino-Acids. *International Journal of Peptide and Protein Research,* 1990, vol. 35, 161-214
- **SIEBER P.** An Improved Method for Anchoring of 9-Fluorenylmethoxycarbonyl-Amino Acids to 4-Alkoxybenzyl Alcohol Resins. *Tetrahedron Letters,* 1987, vol. 28, 6147-50
- **BACKES B J ; ELLMAN J A.** An Alkanesulfonamide Safety-Catch Linker for Solid-Phase Synthesis. *The Journal of Organic Chemisty,* 1999, vol. 64, 2322-30
- **GAUSEPOHL,H. ; PIELES,H. ; FRANK,R.W.** Peptides: chemistry, structure and biology. ESCOM, Leiden, 1992, 523
- **TERUYA K ; MURPHY A C ; BURLIN T ; APPELLA E ; MAZUR S J.** Fmoc-based chemical synthesis and selective binding to supercoiled DNA of the p53 C-terminal segment and its phosphorylated and acetylated derivatives. *Journal of Peptide Science,* 2004, vol. 10, 479-93
- **HOFFMANN R ; VASKO M ; OTVOS L.** Serum stability of phosphopeptides. *Chimica Acta,* 1997, vol. 352, 319-25
- **M.M. BRADFORD.** *Analytische Biochemie,* 1976, vol. 72, 248-254
- **VISTICA D T et al.** Tetrazolium-Based Assays for Cellular Viability - A Critical-Examination of Selected Parameters Affecting Formazan Production. *Cancer Research,* 1991, vol. 51, 2515-20

- **SLATER T F ; SAWYER B ; STRAULI U.** Studies on Succinate-Tetrazolium Reductase Systems .3. Points of Coupling of 4 Different Tetrazolium Salts. *Biochimica et Biophysica Acta,* 1963, vol. 77, 383
- **BERRIDGE M V ; TAN A S.** Characterization of the Cellular Reduction of 3-(4,5-Dimethylthiazol-2-Yl)-2,5-Diphenyltetrazolium Bromide (Mtt) - Subcellular-Localization, Substrate Dependence, and Involvement of Mitochondrial Electron-Transport in Mtt Reduction. *Archives of Bioehemistry and Bio-physics,* 1993, vol. 303, 474-82

- *Bioorganic & Medicinal Chemistry,* 2006, vol. 14, 4444-51
- **PARK Y et al.** A Leu- Lys-rich antimicrobial peptide: activity and mechanism. *Biochimica et Biophysica Acta-Proteins and Proteomics,* 2003, vol. 1645, 172-82